# EUROPEAN PATENT SPECIFICATION

(11) **EP 3 518 957 B1**
(45) Date of publication and mention of the grant of the patent: **15.05.2024**
(21) Application number: 17809192.2
(22) Date of filing: 02.10.2017
(51) Int. Cl.: A61K 38/16, A61K 36/48, A23L 33/185, C07K 16/16, A61P 29/00, A61P 35/00

(54) **THERAPEUTIC PROTEIN**
THERAPEUTISCHES PROTEIN
PROTÉINE THÉRAPEUTIQUE

(30) Priority: 30.09.2016 GB 201616715; 30.09.2016 PT 2016109645
(43) Date of publication of application: 07.08.2019
(73) Proprietor: Instituto Superior de Agronomia, 1349-017 Lisboa (PT)
(72) Inventor: LIMA, Ana Isabel Gusmão, 2845-004 Amora (PT); GUERREIRO, Joana Patrícia Mota, 1950-230 Lisboa (PT); FERREIRA, Ricardo Manuel De Seixas Boavida, 1500-505 Lisboa (PT)
(74) Representative: ip21 Ltd
(86) International application number: PCT/EP2017/075020
(87) International publication number: WO 2018/060528

(56) References cited:
- EP-A1- 2 333 086
- WO-A1-2006/003110
- WO-A1-2012/049215
- WO-A2-2009/144278
- LILLEY G G ET AL: "Amino acid sequence of conglutin delta, a sulfur-rich seed protein of Lupinus angustifolius L", FEBS LETTERS, ELSEVIER, AMSTERDAM, NL, vol. 195, no. 1-2, 20 January 1986 (1986-01-20), pages 235-241, XP025603846, ISSN: 0014-5793, DOI: 10.1016/0014-5793(86)80167-3 [retrieved on 1986-01-20]
- Anonymous: "DEFLAMIN - Trademark Details", Justia Trademarks website , 20 February 2018 (2018-02-20), XP002778320, Retrieved from the Internet: URL:https://trademarks.justia.com/790/68/d eflamin-79068318.html [retrieved on 2018-02-20]

## Description

### Field of the Invention

The invention relates to methods of extraction of deflamin, deflamin polypeptide compositions and associated nucleic acid vectors and products.

### Background of the Invention

During the last decades intensive research has been made to develop novel anti-cancer drugs, both prophylactic and therapeutic. However, despite the significant advances in diagnosis, screening and treatment, the overall long-term outcome in patients has not significantly changed in the last decades. Under this context, there has been an intense search on various biological sources to develop novel anti-cancer drugs to combat this disease, which can be used in prevention, in aiding chemotherapy or in preventing re-incidence. Further, currently there are no prescription drugs that specifically target chronic inflammation (there are, of course, over-the-counter medications that treat the minor and temporary inflammation and accompanying pain caused by injuries or procedures, such as surgery. However, these are not meant to treat chronic inflammation). Some drugs, such as hydroxychloroquine, once used to battle malaria, are useful in treating some lupus patients, but they don't cure the disease. Aspirin and statins have shown promise in reducing inflammation in some people, but researchers aren't sure how broadly useful such drugs are in that role. With the exception of far-from-perfect anti-inflammatory drugs, such as prednisone, a corticosteroid that brings with it a slew of side effects, scientists are still researching how best to contain inflammation. The following prior art documents are acknowledged EP2333086A1, WO2006003110A1, WO2009144278A2, and WO2012049215A1. EP2333086A1 provides the use of a polypeptide from Lupinus as a plant growth promoter, fertilizer or human or animal nutritional supplement. WO2006003110A1 describes a process of crushing and transforming Lupinus albus into flakes. WO2009144278A2 describes an enriched kind of conflutin protein for use as a medicament, a food integrator or dietary supplement. WO2012049215 discloses an antimicrobial polypeptide comprising Blad or an active variant thereof for use in a method of treatment of the human or animal body by therapy or prophylaxis, such as for use in a method of treating or preventing an infection in or on a subject by a microorganism.

### Summary of the Invention

The invention is defined in the claims.

The inventors have discovered 'deflamin', a novel method of extraction and related composition comprising novel polypeptides that has anticancer and anti-inflammatory properties..

Deflamin can be considered in one embodiment to be a mixture of fragments from storage proteins, present in many (but not all) seeds (β- and δ-conglutins, in the case of plants from the genus *Lupinus*)*,* typically purified by a specific procedure and exhibiting a number of unique biological/bioactive properties, namely anti-inflammatory and anti-cancer activities, as well as other biological activities derived from them.

Deflamin can be obtained from many seed species, such as from lupin seeds and from seeds of other species. As described herein a specific methodology was developed to extract and purify deflamin from seeds (lupins and others) that is suitable to undergo up-scaling, allowing its mass production at industrial facilities. The invention also includes recombinant production of deflamin.

The invention includes the preventive and curative use of deflamin in all diseases which develop as a direct or indirect (i.e. inflammation produced by a given treatment) result of inflammation and/or which involve the activity of matrix metalloproteinases (MMPs).

### Definition of Deflamin

Deflamin can be defined by its origin, bioactivities, how it is produced, and, in some cases, structurally. Deflamin is present in the seeds of many, but not all species. Deflamin that is made or used in the invention can have one or more of the physical or therapeutic properties mentioned herein. Such properties include one or more bioactivities as measured in any of the assays (including animal models) described herein and physical properties as measured by electrophoresis-based techniques, HPLC and mass spectrometry assays described herein. Deflamin may comprise naturally occurring sequence(s) or a related artificial (homologous or rearranged) sequence(s).

### Properties of Deflamin

Deflamin is preferably in the form of a mixture of soluble polypeptides/small proteins. It typically possesses one or more of the following characteristics: a) It is readily edible (non-toxic in humans); b) It occurs in seeds; c) It is soluble in water; d) It is comprised by one or any combination of a mixture of low molecular mass polypeptides/small proteins; e) Its bioactivities are resistant to boiling, to a wide range of pH values, to ethanol and and/or to digestive proteases (i.e. they resist the digestive process); f) It strongly inhibits matrix metalloproteinase (MMP)-9 and/or MMP-2, i.e. it is an MMP inhibitor (MMPI) at low concentrations; g) It reduces the migrating capacity of the human colon adenocarcinoma cell line HT29 without inducing significant cytotoxicity; h) It presents at least the following important bioactivities: (i) Potent anti- inflammatory; (ii) Potent antitumoural (anti-migration and anti-metastatic); (iii) No significant cytotoxicity.

When administered orally, deflamin does not trigger any significant immunogenic (i.e. IgG) or allergenic (i.e. IgE) responses. Furthermore, it is bioactive at low concentrations.

In one embodiment the polypeptides comprising deflamin have been identified as fragments of β-conglutin and δ-conglutin large chain (for example deflamin from *Lupinus* seeds). See SEQ ID NO: 192 and SEQ ID NO: 193.

As described in detail below, deflamin shows bioactivity in animal models when administered orally, intraperitoneally, intravenously or topically. In particular deflamin has the following properties: a) Anti-inflammatory activity, as measured in animal models of disease (i.e. mice) when administered by any one of the following routes: oral, intraperitoneal, intravenous and topical; b) Antitumoural activity, as studied by the powerful inhibition of matrix metalloproteinase (i.e. MMP-9 and MMP-2) activities, cancer cell antiproliferative activity and inhibition of cell tumour invasion.

### Relationship to Other Plant Proteins

Blad is a known bioactive plant polypeptide. Blad, as well as the Blad-containing oligomer (BCO), comprise fragments of β-conglutin. However, they are unrelated to deflamin, which typically comprises other fragments of β-conglutin and/or fragments of δ-conglutin large chain.

Functionally the two are very different, with deflamin being totally devoid of anti-microbial activity (as far as bacteria and fungi are concerned), whereas Blad-containing oligomer does not inhibit the gelatinases. Blad corresponds to a fixed fragment of β-conglutin, i.e. Blad comprises residues 109 to 281 of the precursor of β-conglutin (i.e. pro-β-conglutin). In addition to the δ-conglutin large chain, deflamin typically corresponds to other fragments of β-conglutin, for example which span across the entire polypeptide.

### Activity Against Matrix Metalloproteinases (MMPs) and Cancers

Deflamin is a novel type of MMP-9 and/or MMP-2 inhibitor discovered in *Lupinus albus* seeds and present also in other seeds, such as *Cicer arietinum* and *Glycine max.* It is generally established that death of patients in certain cancers, for example colorectal cancer patients, is usually caused by metastatic disease rather than from the primary tumor itself. Metastasis involves the release of the cancer cells from the primary tumour and attachment to another tissues or organs. Cancer cell invasion is a therefore a key element in metastasis and requires integrins for adhesion/de-adhesion and matrix metalloproteinases (MMPs) for focalized proteolysis.

Focalized proteolysis is required to open up the path in the extracellular matrix for the cancer cells to travel across. The wound healing assays provide an estimate of the ability that cells have for cell invasion. Usually, MMP-9 activities are highly related to cancer cell invasion, hence the reduction in MMP-9 activity inhibits cell invasion and the two activities are usually paired. This is why MMP-9 inhibition is so desired, because it directly blocks/limits cell invasion, therefore inhibiting death by metastasis.

On the other hand, cells adhere to a substrate through specific proteins called integrins, transmembrane receptors that are the bridges for cell-cell and cell-extracellular matrix (ECM) interactions. One important function of integrins on cells in tissue culture is their role in cell migration. Integrins are modulated by tumour progression and metastasis and are tightly connected to both MMP-9 and MMP-2 activities. Targeting and disabling integrins in cancer cell membranes can also be desirable because when cells are released from the tumor, they lack the ability to attach to another tissue. This will inhibit metastasis as well and can even help to disaggregate the primary tumor.

Another target for many studies is the specific cytotoxicity against cancer cells. Many bioactive compounds, such as phenolic compounds, strongly reduce cell growth and impair metabolism, reaching toxic levels at moderately high doses. The measurement of cell growth and cell metabolism in the presence of a bioactive compound is a direct measure of its toxicity to the cell. The MTT assay uses a specific coloring agent which needs to be absorbed to the living cells, and then metabolized by them into the corresponding formazan.

In brief, the MTT assay is a colorimetric assay that measures the reduction of soluble, yellow MTT [3-(4,5-dimethylthiazol-2-yl)-2,5-diphenyltetrazolium bromide] by mitochondrial succinate dehydrogenase. The MTT enters the cells and passes into the mitochondria where it is reduced to an insoluble, coloured (dark purple) formazan product [(E,Z)-5-(4,5-dimethylthiazol-2-yl)-1,3-diphenylformazan]. The cells are then solubilised with an organic solvent (e.g. isopropanol) and the released, solubilised formazan reagent is quantified spectrophotometrically. Since reduction of MTT can only occur in metabolically active cells, the level of activity is a measure of the viability of the cells. Therefore, if the cell is dead, or metabolically impaired, it will not produce the coloring agent. Hence, higher levels of color are indicative of a higher number of living, metabolically active cells. If a compound reduces cell growth, or kills the cells, there will be a lower level in color.

Targeting and killing cancer cells is, in theory, a good approach. However, it can only work if there is a high specificity towards the cancer cells and not towards healthy, normal (i.e. non-cancer) cells. Most compounds that destroy cancer cells will also destroy normal healthy cells at a given dose, and although many studies focus only on the ability of a metabolite (e.g. phenolic compounds) to reduce cancer cell growth, they don't often take into account their effects on control healthy cells. One of the reasons why this is rather common, relates to the fact that unlike normal, healthy cells, it is relatively straightforward to culture cancer cells under laboratory conditions. Consequently, later-on, at the level of pre-clinical or even clinical assays, the use of these compounds is frequently hampered by dose-limiting toxicity, insufficient clinical benefits and extremely adverse side-effects. Therefore, a bioactive agent which reduces cell invasion but does not affect the cells normal metabolism (as is the case with deflamin) will produce less (or even negligible) side-effects, and will be safer to use in preventive, long-term administrations, because it will not exert cytotoxicity towards regular cells.

Certain embodiments of the invention envisage curative and/or preventive procedures and/or approaches. For example, deflamin may be administered to healthy individuals to prevent ailments. Certain embodiments of the invention envisage specific routes of administration including one or more of the following: oral, anal, injected and topical.

### Properties of Other MMP Inhibitors

Other plant MMP inhibitors derived from plants have one or more of the following disadvantages a) Toxicity; b) Chemical inactivation (e.g. denaturation) or degradation/destruction (e.g. proteolysis) during the digestive process; c) Absorption into the blood stream, with or without triggering immunogenic (i.e. IgG) or allergenic (i.e. IgE) responses; d) Destruction during boiling (e.g. during cooking); e) No specificity towards all or individual gelatinases; f) High dose requirements; g) Lack of a suitable, effective and low-cost isolation procedure.

This explains, for the most part, why there is not yet a single, plant derived biological compound which found successful application in the realms of human health and nutrition at the level of MMPI inhibition.

### Conglutins

Typically, deflamin polypeptides have sequences which are identical to fragments of conglutin sequences or have strong homologies to fragments of conglutin sequences. In one embodiment such conglutin sequences are from specific conglutins mentioned herein or from naturally occurring homologues of those specific conglutins.

In lupins, conglutins have been classified into four families: α, β, γ and δ conglutins. β Conglutin, the main seed globulin in lupins, is the vicilin or 7S member of the seed storage proteins, whereas α-conglutin is the legumin or 11S member of the seed storage proteins. In narrow-leafed lupin (*Lupinus angustifolius*), a total of three α-conglutin, seven β-congiutin, two γ-conglutin and four δ conglutin encoding genes were previously identified. These genes have been referred to as conglutin alpha 1, 2 and 3, conglutin beta 1, 2, 3, 4, 5, 6 and 7, conglutin gamma 1 and 2, and conglutin delta 1, 2, 3 and 4, respectively.

δ Conglutin belongs to the 2S sulphur-rich albumin family. *Lupinus* seeds 2S albumin, also termed δ conglutin, is a monomeric protein which comprises two small polypeptide chains linked by two interchain disulfide bonds: a smaller polypeptide chain, which consists of 37 amino acid residues resulting in a molecular mass of 4.4 kDa, and a larger polypeptide chain containing 75 amino acid residues with a molecular mass of 8.8 kDa. The sole amino acid sequence of *L. albus* δ conglutin has been inferred from the gene sequence. The larger polypeptide chain contains two intrachain disulfide bridges and one free sulfhydryl group. This protein presents specific unique features among the proteins from *L. albus*: besides its high cysteine content, it exhibits a low absorbance at 280 nm.

As far as the physiological role of δ-conglutin is concerned, a storage function has been proposed for this class of proteins. Structural similarity with the plant cereal inhibitor family, which includes bi-functional trypsin/alpha-amylase inhibitors, may suggest a defence function for this protein in addition to its storage role. Its presence in *L. albus* seeds was assessed to be around 10 to 12%, but more recent data suggest a lower content of around 3 to 4%. The *Lupinus* seed 2S albumin is typically present in both the albumin and the globulin fractions.

### Inflammation

Deflamin can be used to prevent or treat inflammation. Inflammation is the body's immediate response to damage to its tissues and cells by pathogens, noxious stimuli such as chemicals, or physical injury. Acute inflammation is a short-term response that usually results in healing: leukocytes infiltrate the damaged region, removing the stimulus and repairing the tissue. Chronic inflammation, by contrast, is a prolonged, deregulated and maladaptive response that involves active inflammation, tissue destruction and attempts at tissue repair. Deflamin can be used to prevent or treat acute or chronic inflammation.

Deflamin can be used to prevent or treat skin or mucosal inflammatory processes, such as dermatitis, melanoma, periodontitis and gum inflammation. It can be used to treat generalized and chronic digestive inflammation.

It is now widely accepted that chronic inflammation has a role in a host of common and often deadly diseases, including a) Inflammatory bowel disease (IBD), heart disease, stroke, cancer, chronic respiratory diseases, neurological diseases, obesity, and diabetes; b) Atherosclerosis, arthritis, diabetes, acquired immune deficiency syndrome (AIDS) mediated by the human immunovirus, asthma, neoplasia, degenerative and cardiovascular diseases; c) Allergy and autoimmune diseases; d) Obesity and metabolic disease; e) Alzheimer and other neurodegenerative diseases; f) Depression.

Deflamin can be used to prevent or treat any of these conditions.

### Cancer

Cancer is a term for diseases in which abnormal cells divide without control and can invade nearby tissues of the same organism. Cancer cells can also spread to other parts of the body through the blood and lymph systems. There are several main types of cancer a) Carcinoma is a cancer that begins in the skin or in tissues that line or cover internal organs; b) Sarcoma is a cancer which begins in bone, cartilage, fat, muscle, blood vessels, or other connective or supportive tissue; c) Leukemia is a cancer that starts in blood-forming tissue, such as the bone marrow, and causes large numbers of abnormal blood cells to be produced and enter the blood; d) Lymphoma and multiple myeloma are cancers which begin in the cells of the immune system; e) Central nervous system cancers are cancers that start in the tissues of the brain and spinal cord; f) Melanoma is a disease in which malignant (cancer) cells form in melanocytes (cells that color the skin).

Cancer-related conditions: ductal carcinoma *in situ,* male breast cancer, breast cancer, pancreatic cancer, pancreatic exocrine cancer, prostate cancer, colon cancer, rectal cancer, colorectal cancer, cervical cancer, melanoma of the skin, carcinoma, basal cell carcinoma, skin cancer, squamous cell carcinoma, testicular cancer, thyroid cancer, ovarian cancer, ovarian germ cell tumor, lung cancer, bladder cancer, esophageal cancer, stomach cancer, uterine cancer, endometrial cancer, hepatocellular carcinoma, liver cancer, oropharyngeal cancer, hypopharyngeal cancer, laryngeal cancer, nasopharyngeal cancer, pharyngeal cancer, oral cavity cancer, brain tumors, lymphoma, Hodgkin lymphoma, acute myeloid leukemia, kidney cancer, renal cell cancer, non-Hodgkin lymphoma, non-small cell lung cancer, urethral cancer, small cell lung cancer, osteosarcoma, sarcoma, carcinoid tumor, carcinoid syndrome, chronic lymphocytic leukemia, Wilms tumor, retinoblastoma, pituitary tumors, hairy cell leukemia, penile cancer, leukemia, vaginal cancer, Ewing sarcoma, Kaposi sarcoma, malignant fibrous histiocytoma, paget disease of the nipple, gallbladder cancer, acute lymphoblastic leukemia, lymphoma of the eye, adrenocortical carcinoma, adenocarcinoma, parathyroid cancer, pancreatic neuroendocrine tumors, gastrinoma, Merkel cell carcinoma, salivary gland cancer, vulvar cancer, gastrointestinal stromal tumor, anal cancer.

The invention provides deflamin for preventing or treating any of the types of cancer or specific cancers mentioned above or herein. Deflamin is effective during the initial stages of tumourigenesis, inhibiting metastases formation, as part of therapy in chemotherapy and avoiding recurrence of cancer post-surgery.

### Methods of Producing Deflamin

Deflamin for use in the therapeutic aspects of the invention can be made by any suitable method, such as any method described herein. It is preferably made by recombinant expression or by extraction from plant material. Deflamin can be obtained from any plant material that expresses deflamin or deflamin precursors, typically seeds, such as mature seeds for example of any suitable plant genus or species mentioned herein.

### From Plant Material Such as Seeds

Typically, deflamin is obtained by a method that follows a sequential precipitation scheme. The method may be based on deflamin's resistance to high temperatures, low pH and high ethanol concentrations. If flour is used as the starting point for the method it can be obtained by milling a suitable seed.

### Method 1

In one embodiment the method comprises extraction of deflamin from suitable seeds, comprising:
- at least one step at high temperature, preferably at least 80 degrees Celsius or boiling; and
- at least one step at low pH, preferably pH 4 or lower;
- at least one step of contacting the extract with high ethanol concentrations, preferably at least 70% (v/v) ethanol or at least 90% (v/v) ethanol.

### Method 2

In another embodiment the method comprises the following steps:
(a) boiling the intact seeds in water, followed by extraction in water or buffer, and fat removal, or reducing the intact seeds to flour, extraction in water or buffer followed by fat removal and boiling, or fat removal from the flour followed by extraction in water or buffer and boiling;
(b) the soluble fraction is exposed to a sufficiently low pH value (e.g. pH 4.0 or lower) to allow the precipitation of most of the remaining proteins/polypeptides;
(c) the precipitated fraction is resuspended in about 40% (v/v) ethanol, with the solution also optionally containing 0.4 M NaCl. The supernatant contains deflamin;

Optionally the following steps may also be performed:
(d) the soluble fraction is made to 90% (v/v) ethanol to precipitate deflamin and stored at - 20 ºC, or deflamin precipitation may be achieved by other means such as, for example, freeze-drying;
(e) precipitated deflamin may be cleaned from eventual contaminants by repeating steps (c) and (d);
(f) precipitated deflamin may then be dissolved, for example, in water, and desalted by any suitable technique to remove low molecular mass contaminants and/or stored frozen (liquid or dry) until required.

### Method 3

In one embodiment the method comprises the following steps:
(a) providing a flour from suitable seed; (b) defatting said flour; (c) boiling for a period the remaining sample from said defatting step; (d) centrifuging said sample for a period; (e) thereafter discarding a resulting pellet and further processing a resultant supernatant to precipitate polypeptides whilst lowering its pH; (f) further centrifuging to obtain a further pellet; and discarding said supernatant; and (g) further processing said further pellet with ethanol and centrifuging to obtain a further pellet which is then discarded; the remaining supernatant comprising deflamin.

Any of steps (a) to (g) can be replaced with the more specific equivalent steps listed for Methods 4 and 5.

### Method 4

In one embodiment the method comprises the following steps:
(a) flour from a seed is defatted. It can be defatted with an organic solvent and then suspended in water (typically 1:10 to 1:50 w/v; pH typically adjusted to pH 8.0-8.5), under stirring, typically 1 to 6 hours, for example at room temperature, or the fat is simply removed from the top of the slurry after suspension of the flour in water (typically 1:10 to 1:50 w/v; pH typically adjusted to pH 8.0-8.5), under stirring typically for 1 to 6 hours at room temperature. As an alternative to water, typically at a lab scale, the flour may be suspended in Tris-HCl buffer typically 30 to 70 mM, typically at pH 7.5.
(b) after stirring for at least 2 to 6 hours, for example about 4 hours or overnight, typically at 4 ºC, the soluble proteins are obtained by centrifugation, typically at 10,000 to 20,000 *g,* such as 13,500 g for 10 to 90 min, for example for 30 min, typically at 4 degrees Celsius. The pellet is discarded.
(c) the protein solution is boiled, typically at 100 degrees Celsius for 5 to 20 min, such as 10 min, and centrifuged at 10,0000 to 20,000 *g,* for example at 13,500 g, typically for 20 min at 4 degrees Celsius. The pellet is discarded. The supernatant is collected and provides the Heat treated extract (HT).
(d) polypeptides/proteins in the supernatant are precipitated by addition of diluted HCl down to about pH 4.0. Upon centrifugation at 10,000 to 20,000 *g,* for example at 13,500 *g,* typically for 20 min at 4 degrees Celsius, the supernatant is discarded.
(e) the pellet is re-suspended in 30 to 50%, e.g. 40% (v/v) ethanol containing 0.2 to 0.6 M NaCl, e.g. 0.4 M NaCl, stirred, typically for 1 h at room temperature and centrifuged at 10,000 to 20,000 *g,* e.g.13,500 g for 30 min at 4 degrees Celsius. This treatment brings deflamin into solution, unlike the vast majority of the seed storage proteins. The pellet is discarded.
(f) the supernatant is made to 80 to 95%, e.g. 90% (v/v) ethanol and stored at below -10 degrees Celsius, e.g. -20 degrees Celsius, for at least 4 hours, e.g. overnight, precipitating deflamin, followed by centrifugation at 10,000 to 20,000 *g,* e.g. 13,500 g, typically for 30 min at 4 degrees Celsius. The supernatant is discarded.

Optionally the 40 to 90% (v/v) ethanol differential precipitation should be repeated. Thus, the following additional steps may be performed:
(g) deflamin present in the pellet is once again dissolved in 40% (v/v) ethanol containing 0.4 M NaCl, stirred for 1 h at room temperature and centrifuged at 13,500 *g* for 30 min at 4 ºC. This second wash cleans deflamin from final contaminants. The pellet is again discarded.
(h) the supernatant is once more made to 90% (v/v) ethanol and stored at-20 degrees Celsius overnight, precipitating deflamin, followed by centrifugation at 13,500 *g,* for 30 min at 4 degrees Celsius. The supernatant is discarded.
(i) the final pellet contains pure deflamin, which is subsequently dissolved in the smallest possible volume of Milli-Q water. The deflamin solution is finally desalted into water in Sephadex G-25 columns (for example NAP-10 columns, GE Healthcare Life Sciences) to remove low molecular mass contaminants.
(j) the extract obtained may optionally be stored at -20 degrees Celsius.

### Method 5

In one embodiment deflamin is obtained using the following method:
(a) flour from a suitable seed is
   - defatted with n-hexane and then suspended in water (1:20 w/v; pH adjusted to pH 8.0-8.5), under stirring for 2 to 3 h at room temperature, or
   - the fat is simply removed from the top of the slurry after suspension of the flour in water (1:20 w/v; pH adjusted to pH 8.0-8.5), under stirring for 2 to 3 h at room temperature.
   - as an alternative to water, typically at a lab scale, the flour may be suspended in 50 mM Tris-HCl buffer, pH 7.5. Stirring is for 4 h (or overnight) at 4 degrees Celsius.
(b) the soluble proteins are obtained by centrifugation at 13,500 *g* for 30 min at 4 degrees Celsius. The pellet is discarded.
(c) the protein solution is boiled at 100 degrees Celsius for 10 min and centrifuged at 13,500 *g* for 20 min at 4 degrees Celsius. The pellet is discarded. The supernatant is collected and provides the Heat treated extract (HT).
(d) polypeptides/proteins in the supernatant are precipitated by addition of diluted HCl down to pH 4.0. Upon centrifugation at 13,500 *g* for 20 min at 4 degrees Celsius, the supernatant is discarded.
(e) the pellet is re-suspended in 40% (v/v) ethanol containing 0.4 M NaCl, stirred for 1 h at room temperature and centrifuged at 13,500 *g* for 30 min at 4 degrees Celsius. This treatment brings deflamin into solution, unlike the vast majority of the seed storage proteins. The pellet is discarded.
(f) the supernatant is made to 90% (v/v) ethanol and stored at-20 degrees Celsius overnight, precipitating deflamin, followed by centrifugation at 13,500 *g* for 30 min at 4 degrees Celsius. The supernatant is discarded.

Optionally, the 40 to 90% (v/v) ethanol differential precipitation should be repeated. Thus the following additional steps may be performed:
(g) deflamin present in the pellet is once again dissolved in 40% (v/v) ethanol containing 0.4 M NaCl, stirred for 1 h at room temperature and centrifuged at 13,500 *g* for 30 min at 4 degrees Celsius. This second wash cleans deflamin from final contaminants. The pellet is again discarded.
(h) the supernatant is once more made to 90% (v/v) ethanol and stored at-20 degrees Celsius overnight, precipitating deflamin, followed by centrifugation at 13,500 *g* for 30 min at 4 degrees Celsius. The supernatant is discarded.
(i) the final pellet contains pure deflamin, which is subsequently dissolved in the smallest possible volume of Milli-Q water. At a lab scale, the deflamin solution is finally desalted into water in Sephadex G-25 columns (for example NAP-10 columns, GE Healthcare Life Sciences) to remove low molecular mass contaminants.
(j) The extract obtained is optionally stored at-20 degrees Celsius.

The flour used in any of the above methods is optionally obtained by milling a seed, such as milling about 100 g ± 0.1 g of dry seed (typically without embryo and tegument) to obtain flour.

### Method 6

Methods of producing recombinant proteins are well known in the art. Such methods as applied here will involve inserting the polynucleotide encoding a deflamin polypeptide into a suitable expression vector-enabling the juxtaposition of said polynucleotide with one or more promoters (e.g. an inducible promoter, such as T7lac) and with other polynucleotides or genes of interest-introducing the expression vector into a suitable cell or organism (e.g. *Escherichia coli*), expressing the polypeptide in the transformed cell or organism and removing the expressed recombinant polypeptide from that cell or organism. To assist such purification the expression vector may be constructed such that the polynucleotide additionally encodes, for example, a terminal tag that can assist purification: e.g., a tag of histidine residues for affinity purification. Once the recombinant polypeptide is purified, the purification tag may be removed from the polypeptide, e.g., by limited proteolytic cleavage.

### Method 7

Simpler, economical and expeditious methodologies are possible, leading to a rather pure deflamin, but not as much as that achieved with the procedures described above. These methodologies will necessarily involve extraction, boiling, exposure to low pH values and treatment with ethanol.

### Physical Structure of Deflamin

Deflamin is a composition that comprises a mixture of polypeptides obtainable from seed storage proteins by the method of any one of claims 1 to 3. It typically comprises a mixture of at least 2 to 200 different polypeptides, such as 20 to 150, 30 to 100 or 50 to 80 different polypeptides which have one or more of the following characteristics:
(a) they have a length of 5 to 250 amino acid residues, such as 5 to 200, 50 to 200, 75 to 150, or preferably 100 to 180 or 120 to 170 amino acid residues, and/or
(b) they comprise or consist of a sequence which is a portion and/or homologue of sequence from a conglutin, such as any conglutin mentioned herein or a portion represented by any of SEQ ID NO's 8 to 190, and/or
(c) they each comprise a sequence that is
   (i) a portion of a conglutin protein, wherein said portion is at least 5, 10, 20, 30 or 50 amino acid residues long, and/or
   (ii) a homologue of the portion defined in (i), which preferably has at least 70% identity to said portion,
   wherein said conglutin protein is optionally a conglutin beta 1, 2, 3, 4, 5, 6 or 7 or a conglutin delta 2 protein;
   and/or
(d) at least 20, 30 or 50 of the polypeptides comprise a sequence which is a rearrangement of sequence derived from a conglutin.

Deflamin polypeptides may exhibit microheterogeneity. Thus, in the case of lupins, deflamin polypeptides correspond to sequences of conglutins which overlap for the most part but which show varying length.

Deflamin polypeptides with a rearranged sequence typically comprise portions of sequence from different conglutins or from different parts of the same conglutin molecule; or homologues of such portions. Such portions (including homologues of portions) can be at least 5,10,20,30 or 50 amino acid residues long. Portions which are from differentpart of the same conglutin can separated by at least 10, 20, 50 or 200 amino acids in the original conglutin in which they occur. A deflamin polypeptide with a rearranged sequence may comprise at least 2, 3, 4, 5 or 6 different portions of conglutin sequence which are from different conglutin molecules and/or from different parts of the same conglutin molecule. Such portions may be the same as, be portions of and/or be homologues of any specific sequence mentioned herein, including any of SEQ ID NO's 8 to 190.

In one embodiment the deflamin composition comprises at least 10%, 20%, 30%, 50%, 80% or all of the sequence of SEQ ID NO's 8 to 55 and/or 56 to 75 and/or 76 to 190 as part of all the polypeptides which are present; or homologues of any of these specific sequences or any other sequences specified herein.

Deflamin typically comprises a mixture of at least two to 200 different polypeptides, such as 20 to 150, 30 to 100 or 50 to 80 different polypeptides which each comprise a sequence that is
(a) the same as any one of SEQ ID NO's 8 to 190 or is a portion of any of SEQ ID NO's 8 to 190 that is at least 5, 10, 20, 30 or 50 amino acid residues long, and/or
(b) a homologue of the sequence defined in (a), which preferably has at least 70% homology to (a).

In one embodiment, the deflamin composition does not comprise any polypeptides other than the ones defined in this section or such other polypeptides represent less than 30%, such as less than 10% of the total mass of the polypeptides in the composition.

Where groups of polypeptides defined as SEQ ID NO's are mentioned herein the polypeptides of category I (SEQ ID NO's 8 to 55) are most preferred, followed by category II (SEQ ID NO's 56 to 75), followed by category III (SEQ ID NO's 76 to 190).

In one embodiment the deflamin composition comprises in the form of sequences within all its polypeptides portions of sequences from a conglutin (such as any conglutin mentioned herein) which 'span' the conglutin. Typically, there are at least 3, 4, 5 or 6 portions where at least 1, 2 or 3 of the portions occur in the first half and second half of the conglutin polypeptide (where the N terminal end of the conglutin represents the start of the molecule). Preferably in this situation at least one portion is from each of the first, second and third parts of the conglutin polypeptide if the conglutin polypeptide is imagined as being divided into sections of three equal lengths.

In certain embodiments deflamin comprises polypeptides derived from both β- and δ-conglutins, for example at least 1, 2, 3, 5, or 10 peptides from both β- and δ-conglutins (preferably *L. albus* β- and δ-conglutins). Deflamin may comprise 2 groups of such peptides corresponding to molecular masses of 13 kDA and 17 kDa. Their lengths may be of at least or from a range defined by any two or more of the following 100, 110, 120, 130, 140, 150, or 160 amino acid residues. In certain embodiments, deflamin is composed of a mixture of polypeptides which originate from two peaks of polypeptides (13 and 17 kDA).

### Deflamin: Variants, Homologues and Portions

In any embodiment of deflamin described herein one or more of the deflamin polypeptides can be replaced by 'variants', and thus typically naturally occurring sequences may be replaced with homologous sequences or one or more portions of the natural sequence. Preferably such variants are homologues and/or portions of the sequence shown by SEQ ID NO's 8 to 190. Levels of percentage identity for such homologues are described below. Portions of the sequence will consist of at least 50, 80 or 90% of the original sequence, and may be at least 5,10, 20, or 30 amino acid-residues in length. The variant will preferably retain the activity of the original polypeptide/sequence, for example as measured using any assay or test described herein.

Homologous sequences typically have at least 40% identity, preferably at least 60%, preferably at least 70%, preferably at least 80%, preferably at least 85%, preferably at least 90%, preferably at least 95%, preferably at least 97%, and most preferably at least 99% identity, for example over the full sequence or over a region of at least 20, preferably at least 30, preferably at least 40, preferably at least 50, preferably at least 60, preferably at least 80, preferably at least 100, preferably at least 120, preferably at least 140, and most preferably at least 160 or more contiguous amino acid residues. Methods of measuring protein homology are well known in the art and it will be understood by those of skill in the art that in the present context, homology is calculated on the basis of amino acid identity (sometimes referred to as "hard homology").

The homologous sequence typically differs from the original sequence by substitution, insertion or deletion, for example by 1, 2, 3, 4, 5 to 8, 9 to 15 or more substitutions, deletions or insertions. The substitutions are preferably 'conservative', that is to say that an amino acid may be substituted with a similar amino acid, whereby similar amino acids share one of the following groups (in what their lateral chain R is concerned): aromatic residues (F/H/W/Y), non-polar aliphatic residues (G/A/P/I/L/V), polar-uncharged aliphatic residues (C/S/T/M/N/Q) and polar-charged aliphatic-residues (D/E/K/R). Preferred sub-groups comprise: G/A/P; I/LN; C/S/T/M; N/Q; D/E; and K/R.

Homology (identity) can be measured using known and available methods. For example, the UWGCG Package provides the BESTFIT program which can be used to calculate homology (for example used on its default settings) (Devereux et al. (1984) Nucleic Acids Res. 12, 387-395). The PILEUP and BLAST algorithms can be used to calculate homology or line up sequences (typically on their default settings), for example as described in Altschul (1993) J. Mol. Evol. 36, 290-300, and Altschul et al. (1990) J. Mol. Biol. 215, 403-410 .

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al.,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or when the end of either sequence is reached. The BLAST algorithm parameters W, T and X determine the sensitivity and speed of the alignment The BLAST program uses as defaults a word length (W) of 11, the BLOSUM62 scoring matrix (see Henikoff and Henikoff (1992) Proc. Natl. Acad. Sci. USA 89, 10915-10919) alignments (B) of 50, expectation (E) of 10, M = 5, N = 4, and a comparison of both strands. The BLAST algorithm performs a statistical analysis of the similarity between two sequences; see e.g., Karlin and Altschul (1993) Proc. Natl. Acad. Sci. USA 90, 5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

### Forms of Deflamin

A composition comprising, consisting or consisting essentially of deflamin is typically in an isolated or purified form (e.g. removed from a plant or cellular source). This typically comprises less than 50% or less than 20% or 10% or 5% non-deflamin dry mass.

A deflamin composition may also be a formulation comprising another compound(s) added to the composition by the skilled person. In preferred embodiments, such a formulation is a pharmaceutical formulation comprising deflamin and a pharmaceutically acceptable carrier or diluent The skilled person will be able to identify, through routine methods, a suitable concentration with which to use deflamin in any particular setting, for example when administered in therapy. Preferably, for example, it is used at a concentration of at least 1 µg/mL, at least 5 µg/mL, at least 10 µg/mL, at least 20 µg/mL, at least 50 µg/mL, or at least 100 µg/mL, and up to 500 µg/mL, up to 600 µg/mL, up to 1 mg/mL, up to 2.5 mg/mL, up to 5 mg/mL or up to 10 mg/mL. Preferably the concentration is between 10 µg/mL and 5 mg/mL, more preferably between 50 µg/mL and 2.5 mg/mL, more preferably between 100 µg/mL and 1 mg/mL, and even more preferably between 100 µg/mL and 600 µg/mL (such as about 250 µg/mL).

In one embodiment, the deflamin composition comprises less than 20%, less than 10% or less than 1% by weight or is completely free of lunasin or Blad protein, for example as defined by the specific sequences given herein. In another embodiment none of the deflamin polypeptides comprise any sequence from lunasin or Blad, i.e. they do not comprise any portions of sequence from lunasin and/or Blad.

### Therapeutic Uses

When used in therapy to prevent or treat a condition deflamin is preferably used in a therapeutically effective amount. Preferably, the therapeutically effective amount is non-toxic to the human or animal subject.

The invention provides a deflamin polypeptide composition for use in a method of treatment of the human or animal body by therapy, wherein said therapy is preferably preventing or treating inflammation or cancer. To this end the invention also provides a method of treating a human or animal comprising administering to a subject in need thereof a composition comprising a therapeutically effective amount of an antimicrobial polypeptide comprising deflamin or containing deflamin in addition to antimicrobial polypeptide(s). The invention also provides use of deflamin in the manufacture of a medicament for treating or preventing inflammation or cancer.

The individual polypeptides making up deflamin can be delivered separately, and accordingly the invention provides a product comprising a multiplicity of different polypeptides which together form a deflamin composition for simultaneous, separate or sequential use in a method of treatment of the human or animal body by therapy, wherein said therapy is preferably preventing or treating inflammation or cancer.

Deflamin may be administered by any suitable route, for example by an intradermal, subcutaneous, intramuscular, intravenous, intraosseous, and intraperitoneal, topical, oral or transmucosal (such as nasal, sublingual, vaginal or rectal) route.

Deflamin is preferably administered together with carriers, diluents and auxiliary substances. Pharmaceutically acceptable carriers include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, although not required, that the preparation will contain a pharmaceutically acceptable carrier that serves as a stabilizer. Examples of suitable carriers that also act as stabilizers for polypeptides include, without limitation, pharmaceutical grades of dextrose, sucrose, lactose, trehalose, mannitol, sorbitol, inositol, dextran, and the like. Other suitable carriers include, again without limitation, starch, cellulose, sodium or calcium phosphates, citric acid, tartaric acid, glycine, high molecular mass polyethylene glycols (PEGs), and combination thereof.

Once formulated, the composition can be delivered to a subject *in vivo* using a variety of known routes and techniques. For example, the liquid preparations can be provided as an injectable solution, suspension or emulsion and administered via parenteral, subcutaneous, intradermal, intramuscular, intravenous, intraosseous or intraperitoneal injection using a conventional needle and syringe, or using a liquid jet injection system. Liquid preparations can also be administered topically to the eyes, to skin, hair or mucosal tissue (e.g. nasal, sublingual, vaginal or rectal), or provided as a finely divided spray suitable for respiratory or pulmonary administration. Other modes of administration include oral administration, suppositories, and active or passive transdermal delivery techniques.

The subject in need of therapy may be any human or animal individual. The subject is typically a chordate, mammal, agricultural animal or rodent. In preferred embodiments the deflamin may be used in therapy of subjects at particular risk of inflammation or cancer.

Deflamin can be administered by use of nucleic acid expression vectors which express deflamin *in vivo.* The invention provides one or more nucleic acid vectors which together or individually express a deflamin composition for use in a method of treatment of the human or animal body by therapy, wherein said therapy is preferably preventing or treating inflammation or cancer. The nucleic acid vector may be a viral vector or any other type of vector which allows delivery of the nucleic acid.

The invention also provides a product comprising a multiplicity of nucleic acid vectors which together express a deflamin composition for simultaneous, separate or sequential use in a method of treatment of the human or animal body by therapy, wherein said therapy is preferably preventing or treating inflammation or cancer.

### Antibodies

The invention provides one or more antibodies or their fragments thereof which bind any one of the polypeptides corresponding to sequences SEQ ID NO's 8 to 190 in a specific manner.

### Brief Description of the Drawings

The invention will now be described with reference to the accompanying drawings, in which:
Figure 1 shows the internal fragment of the β-conglutin precursor encoding sequence that corresponds to Blad;
Figure 2 shows the secondary polypeptide structure of Blad;
Figure 3 shows the tertiary polypeptide structure of Blad;
Figure 4 shows a diagrammatic representation of the methodology used to extract and purify deflamin from *Lupinus albus* seeds;
Figure 5 shows a comparison between the albumin and globulin polypeptide profiles for each of the eight legume seeds initially analysed;
Figure 6 is a graph showing MMP-9 inhibitory activity of the eight legume seeds initially analysed;
Figure 7 is a graph that compares a cell proliferation assay between the albumin and globulin fractions from each of the eight legume seeds initially analysed;
Figure 8 shows the images of a cell migration wound assay for three legume seeds: *Lupinus albus, Cicer arietinum* and *Glycine max;*
Figure 9 is a graph showing the results of the cell migration wound assay comparing the albumin and globulin fractions from each of the eight legume seeds initially analysed (performed as shown in Figure 8);
Figure 10 is a graph showing gelatinolytic activity comparing the albumin and globulin fractions from each of the eight legume seeds initially analysed;
Figure 11 shows zymographic profiles of the MMP-9 and MMP-2 activities comparing the albumin and globulin fractions from three legume seeds: *Lupinus albus, Cicer arietinum* and *Glycine max;*
Figure 12 is a graph showing phytin concentration comparing the uncooked and cooked fractions from six legume seeds analysed;
Figure 13 is a graph showing saponin concentration comparing the uncooked and cooked fractions from six legume seeds analysed;
Figure 14 is a graph showing phenolic compound concentration comparing the uncooked and cooked fractions from six legume seeds analysed;
Figure 15 is graph showing soluble protein concentration comparing the uncooked and cooked fractions from six legume seeds analysed;
Figure 16 shows polypeptide profiles obtained by R-SDS-PAGE comparing the uncooked and cooked fractions from six legume seeds analysed;
Figure 17 shows images of cell migration assessed by a wound healing assay comparing several cooked and uncooked fractions from three legume seeds: *Lupinus albus, Cicer arietinum* and *Glycine max;* Figure 18 is a graph showing the relative migration rates of the wound healing assay comparing several cooked and uncooked fractions from three legume seeds: *Lupinus albus, Cicer arietinum* and *Glycine max* (Figure 17);
Figure 19 is a graph showing cell proliferation comparing several cooked and uncooked fractions from three legume seeds: *Lupinus albus, Cicer arietinum* and *Glycine max;*
Figure 20 is a graph showing gelatinolytic activity comparing several cooked and uncooked fractions from three legume seeds: *Lupinus albus, Cicer arietinum* and *Glycine max;*
Figure 21 is a graph showing the inhibitory effect on MMP proteolytic activity comparing several cooked and uncooked fractions from three legume seeds: *Lupinus albus, Cicer arietinum* and *Glycine max;*
Figure 22 is a graph showing protein peaks of a deflamin partially purified extract from *L*. *albus* seeds as fractionated by FPLC gel filtration;
Figure 23 shows the separation of the polypeptide peaks in Figure 22, separated by Tricine SDS-PAGE;
Figure 24 is a graph showing MMP-9 inhibitory activity of each of the protein fractions from Figure 22;
Figure 25 is a graph showing HPLC-reverse phase chromatography profiles of the MMP-inhibitory fraction isolated from *L. albus,*
Figure 26 shows electrophoretic profiles under reducing conditions of the MMP-inhibitory fractions isolated from *L. albus* (Figure 25);
Figure 27 is a graph showing the effects of the different peak fractions, shown in Figure 25, on MMP-9 activity.
Figure 28 shows the *L. albus* polypeptide composition of peak 2 collected from the HPLC run depicted in Figure 25.
Figure 29 compares the percentage wound closure for the *L. albus* sample of Figure 28 with several *L. albus* protein fractions;
Figure 30 shows images of the wound closure assays corresponding to Figure 29;
Figure 31 is a graph showing the gelatinolytic activity profile corresponding to Figure 29;
Figure 32 is a graph showing quantified MMP-9 and MMP-2 activities corresponding to Figure 29;
Figure 33 shows zymographic profiles of MMP-9, of MMP-2 and of their zymogens enzyme activities in HT29 extracellular media after a 48 h exposure of the cells to 'deflamin';
Figure 34 shows a representative image of the polypeptide distribution between *Lupinus albus* seeds simply extracted with buffer (extraction buffer; BE) or after heat treatment (HT), and visualized by SDS-PAGE (left) or the reverse gelatin zymography (right);
Figure 35 shows representative images of the polypeptide profiles obtained after each step of the deflamin purification protocol as specified on the top of the gels;
Figure 36 is a graph showing total gelationlytic activity of MMP-9 proteolytic activity in the present of extracts collected at various stages along the deflamin purification protocol;
Figure 37 is a graph showing HT29 cell migration in the present of extracts collected at various stages along the deflamin purification protocol;
Figure 38 shows examples of the cell migration obtained in the present of extracts collected at various stages along the deflamin purification protocol (Figure 37);
Figure 39 is a graph showing the effect of different concentrations of deflamin on gelatineolytic activity;
Figure 40 is a graph showing the effect of different concentrations of deflamin on cell migration (Figure 41);
Figure 41 shows examples of cell migration obtained for different concentrations of deflamin;
Figure 42 shows the effects of different concentrations of deflamin on cell proliferation;
Figure 43 shows a polypeptide profile of deflamin under reducing and non-reducing conditions. Molecular masses of standards are indicated in kDa;
Figure 44 shows a representative image of deflamin fractionation into its constituent polypeptides monitored at 214 nm (HPLC reverse-phase chromatography);
Figure 45 shows a representative image of deflamin fractionation into its constituent polypeptides monitored at 280 nm (HPLC reverse-phase chromatography);
Figure 46 shows polypeptide profiles of each peak collected from the fractionation in Figures 44 and 45, as visualized by SDS-PAGE;
Figure 47 is a graph showing MMP-9 proteolytic activity in the presence of fractions 1 to 4 obtained by the fractionation of deflamin in Figures 44 and 45;
Figure 48 is a graph showing the effect of selected deflamin peaks (fractions 1 to 4 obtained by the fractionation of deflamin in Figures 44 and 45) on cell migration;
Figure 49 shows the electrophoretic profile of the deflamin fractions that are soluble and of those that are precipitated with Ca and Mg after fractionation of *L. albus* deflamin in two fractions by Ca2+ and Mg2+;
Figure 50 shows the inhibition of cell invasion in HT29 cells by deflamin and its two subfractions, precipitated or not with Ca and Mg, that is, it shows that the separation of deflamine in two fractions with Ca2+ and Mg2+ influences its anti-tumoral activity;
Figure 51 shows the influence of *L. albus* deflamin on the transcription of specific genes in HT29 cells related to inflammation and tumor invasion;
Figure 52 shows the bioactivity (at the level of HT29 cell invasion inhibition) of *L. albus* deflamin in food products, i.e. when used in the manufacture of cooked salted biscuits;
Figure 53 shows the analysis of the *L. albus* deflamin by HPLC and electrophoresis;
Figure 54 shows the mass spectrometric analysis of the two *L. albus* deflamin fragments by MALDI-TOF;
Figure 55 shows preliminary results on the anti-colitis effects of deflamin on colitis-induced mice;
Figure 56 is a graph showing the effect of several routes of deflamin administration (and the corresponding controls) on colon length from colitis-induced mice;
Figure 57 is a graph showing the effect of several routes of deflamin administration (and the corresponding controls) on the extent of intestine injury in colitis-induced mice;
Figure 58 shows macroscopic observations of the colons isolated from the different treatments groups (deflamin and controls) of colitis-induced mice;
Figure 59 shows macroscopic observations of the colons isolated from the different treatments groups (deflamin and controls) of colitis-induced mice;
Figure 60 shows the effect of deflamin administration on the histological features of colon inflammation from colitis-induced mice;
Figure 61 shows the effect of deflamin administration on the colon tissue expression of COX-2 and iNOS in colitis-induced mice;
Figure 62 is a graph showing the effect of deflamin administration on the colon tissue gelatinase activities of MMP-2 and MMP-9 from colitis-induced mice;
Figure 63 shows zymographic profiles showing the effect of deflamin administration on the colon tissue gelatinase activities of MMP-2 and MMP-9 from colitis-induced mice.
Figure 64 is a graph showing the effect of deflamin administration on the rat paw oedema development;
Figure 65 is a graph showing the effect of topical deflamin administration on paw oedema in rats;
Figure 66 shows a reverse zymography of blood and faeces from colitis-induced mice treated with deflamin;
Figure 67 shows representative images of wound closure assays showing the cell anti-migration effect of deflamin (purified, cooked seeds and un- cooked seeds);
Figure 68 shows representative images of wound healing assays assessing cell migration in the presence of different extract concentrations of *Lupinus albus, Cicer arietinum* and *Glycine max* seeds;
Figure 69 shows an SDS-PAGE of deflamin as isolated by the diagram depicted in Figure 4 from *Lupinus albus, Glycine max* and *Cicer arietinum* seeds;
Figure 70 is a graph showing a comparison of the anti-gelatinase (MMP-9 and MMP-2) activity of deflamin from *Lupinus albus, Glycine max* and *Cicer arietinum* seeds;
Figure 71 is a graph showing a comparison of the anti-invasion activity of different concentrations of deflamin from *Lupinus albus, Glycine max* and *Cicer arietinum* seeds; and
Figure 72 is a graph showing a comparison of cell growth in the presence of deflamin from *Lupinus albus, Glycine max* and *Cicer arietinum* seeds.
Figure 73 shows a reverse zymography performed on a polyacrylamide gel containing gelatin and HT-29 medium with MMP-9 and MMP-2 to detect the presence of deflamine in seeds of other *Lupinus* species, other genera of legumes and other non-leguminous species, including cereals and others;
Figure 74 shows a reverse zymography performed on a polyacrylamide gel containing gelatin and HT-29 medium with MMP-9 and MMP-2 to detect the presence of deflamine in seeds of other species of the genus *Lupinus*;
Figure 75 shows a representative polypeptide profile of *Lupinus mutabilis* deflamine by SDS-PAGE performed under reducing and non-reducing conditions;
Figure 76 shows the representative polypeptide profile of *Vigna mungo* deflamin by SDS-PAGE performed under reducing and non-reducing conditions;
Figure 77 shows a reverse zymography performed on a polyacrylamide gel containing gelatin and HT-29 medium with MMP-9 and MMP-2 to detect the presence of deflamine in seeds of species of the genus *Triticum*;
Figure 78 shows the SDS-PAGE representative polypeptide profile of the isolated deflamin from various species of the genus *Triticum.*

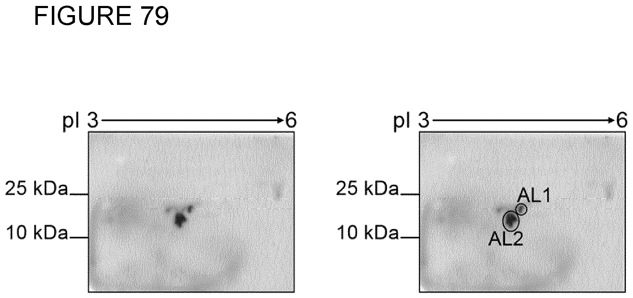

Figure 79 shows 2D-gel electrophoresis IPG pH 3-6, 7 cm and SDS-PAGE 17.5% (w/v) acrylamide/ bis-acrylamide. IEF was performed at 4,000 V, current limit of 50 µA/ strip, 10,000 V-h. SDS-PAGE separation 65 min at 200 V.

### Examples

### Technical Information

With increasing incidence of inflammatory diseases, an inevitable boost in medical and pharmaceutical costs is occurring. It seems increasingly likely that the future of human health will rely on two basic areas: traditional clinical therapies for treatment, and appropriate diets (including both nutraceuticals and functional foods) for both treatment and prevention. It has been predicted that the continued ingestion of functional foods and/or nutraceuticals that reduce inflammation will constitute the most effective human tool against the majority of the ailments that inflict today's modern societies. MMP inhibitors (MMPIs) are considered anti-angiogenic agents for primary tumours and metastasis deterrents, and have also been demonstrated to effectively inhibit pre-cancer states such as colitis and other inflammatory bowel diseases. Over the last decade a substantial amount of research has turned towards discovery of novel plant foods and compounds presenting MMPI activity, but targeting individual MMPs in a specific manner has proven itself difficult.

Embodiments of the invention describe a new type of MMPIs that are proteinaceous in nature, survive the digestion process and may be administered orally, intraperitoneally, intravenously or applied topically, and which may be used as a functional food in the prevention/treatment of inflammation, tumourigenesis and cell proliferation, as well as of any disease derived from them. These MMPIs have been shown to be potent inhibitors of the matrix metalloproteinases MMP-9 and MMP-2, thus exhibiting powerful anti-inflammatory, antitumour and antiproliferative activities. Embodiments of the invention show deflamin in the composition of functional foods in the prevention or treatment of a very wide array of diseases.

### Brief Note on Deflamin Discovery

Embodiments of deflamin comprises new types of MMP-9 and/or MMP-2 inhibitors extracted from *Lupinus albus* seeds and present also in other seeds, both legumes (such as *Cicer arietinum* and *Glycine max*) and non-legumes. These findings led to the promising discovery of deflamin, a group of water soluble polypeptides/small proteins isolated from the edible seeds of a commonly eaten legume species, *Lupinus albus,* which exhibits a highly potent inhibitory activity towards MMP-9 and/or MMP-2 in cultured colon cancer cells and reduces colitis in animal models when administered orally, intraperitoneally, intravenously or topically, without exerting any apparent significant cytotoxicity. Deflamin was also found to be obtained from other seeds as well, either legumes (e.g. *Cicer arietinum* and *Glycine max*) and non-legumes. In the case of *Lupinus albus,* deflamin comprises in certain embodiments polypeptide fragments from both β-conglutin and δ-conglutin large chain. In addition to high stability to extreme values of pH and temperature, preliminary evidence indicates that they also resist digestion, making these polypeptides/small proteins excellent candidates to become valuable anti-inflammatory agents. These novel polypeptides/small proteins may be produced in certain embodiments as an anticancer drug. Embodiments of the invention also include efficient methods to isolate deflamin, appropriate for scaling-up to an industrial scale. A search for homologues in the seeds of other species was undertaken and will be further pursuit in the future.

### MMPs and Cancer

During the last decades intensive research has been made to develop novel anti-cancer drugs, both prophylactic and therapeutic. However, despite the significant advances in diagnosis, screening and treatment, the overall long-term outcome in patients has not significantly changed in the last decades (Herszényi *et al.,* 2012). Under this context, there has been an intense search on various biological sources to develop novel anti-cancer drugs to combat this disease, which can be used in prevention, in aiding chemotherapy or in preventing re-incidence, especially in the case of plant-food products or bioactive plant compounds, which can be used as nutraceuticals for prevention and to aid chemotherapy (Su *et al.,* 2006). Plants have proved to be an important natural source of compounds for medical therapy (including for cancer) for several years. It has been estimated that over the last 20 years, 25 to 30% of the new drugs entering the US market were discovered in plants. Worldwide, the over-the-counter value of these drugs is estimated at more than $40 billion annually. Therefore, pharmaceutical companies and institutions throughout the world are implementing plant screening programs as a primary means of identifying new drugs.

Colorectal cancer (CRC) is the second most common cause of cancer death in the European Union (EU), with an enormous health and economic burden. Around 436,000 new cases and 212,000 deaths occur each year in Europe. Death of CRC patients is usually caused by metastatic disease rather than from the primary tumor itself.

Matrix metalloproteinases (MMPs) are a family of zinc-dependent endopeptidases which are engaged in the remodeling of connective tissue (Markle *et al.,* 2010). A subgroup of MMPs, also called gelatinases (MMP-2 and MMP-9), have been shown to be largely implicated in CRC in animal models and patients. Their inhibitors (MMPIs) were demonstrated to be effective in reducing cancer progression/metastasis in *in vitro* assays and animal models and appear to be mostly effective at early stages of cancer or in preventing development of undetected micrometastases after surgery (Coussens *et al.,* 2002; Mook *et al.,* 2004; Zucker & Vacirca, 2004; Sang *et al.,* 2006; Herszényi *et al.,* 2012). In the last decade the development of synthetic MMPIs became an important branch of research in both academic and industrial settings and numerous MMP inhibitors have been tested in different clinical trials, especially MMP-2 inhibitors (Hidalgo & Eckhardt, 2001). Because of this, and in view of MMP-2 and MMP-9 involvement in various diseases, inhibition of specific MMPs up-regulation is believed to be able to improve clinical symptoms of patients. However, targeting MMPs in disease treatment has proven itself difficult by the fact that MMPs are ubiquitously indispensable for normal development and physiology, and previous efforts to inhibit MMP activity in the treatment of cancer patients yielded very unsatisfactory results with severe adverse side effects (Coussens *et al.,* 2002; Ndinguri *et al.,* 2012). Due to this, synthetic peptide inhibitors based on MMPs structure quickly became a hot spot of study on specific inhibitors.

Because MMPs are initially synthesized as zymogens (and therefore inactive), with pro-peptides that must be removed from a pro-peptide domain before the enzyme is active (Lu et *al.,* 2012; Ndinguri *et al.,* 2012), peptide drugs can inhibit extracellular MMPs activation directly, without affecting intracellular MMPs expression and therefore avoid generalized, deleterious side-effects (Lu *et al.,* 2012).

Nowadays, peptide research on drug design and discovery is one of the most promising fields in the development of new drugs. Compared to small molecule compounds, peptide drugs offer various advantages, such as high specificity and low toxicity (Lu *et al.,* 2012). In the last decade a substantial amount of research has turned towards novel plant MMPIs which are clinically active against various types of cancer cells. However, for reasons which are not understood, such studies often neglected peptides and small proteins. Several plant species are known to present specific bioactive peptides and small proteins, with functions such as defense against pathogen attack, or proteolytic inhibition. Such is the case, for example, of legume seeds (Park *et al.,* 2007). The fact that many of these inhibitors derive from plant foodstuffs makes them perfect candidates to use as nutraceuticals and in cancer-preventing diets, particularly in the case of colon cancer. Compared with the traditional cancer treatments such as chemotherapy or radioactive treatment, peptides and small proteins with high specificity against cancer cells or against tumor promoters may present the way of killing cancer cells while protecting normal cells and helping patients to recover rapidly (Park *et al.,* 2007).

The present findings have led to the discovery that peptides and small proteins from some edible seeds exhibit a strong inhibitory activity against MMP enzymes. Advantageously, in certain embodiments, they may pass unaltered through the human digestive tract and can therefore be used for colon cancer treatment.

The use of digestion-resistant, peptides and small proteins MMPIs in studies on CRC control and treatment seem particularly suitable, because the inside of the colon may actually be considered `outside the body', a situation in which the MMPIs will not show side-effects unless they are absorbed into the blood stream.

### MMPs and Inflammation

Worldwide incidence and prevalence of inflammatory bowel diseases (IBD) have increased dramatically over time, evidencing its emergence as a global disease (Molodecky *et al.,* 2012; Burisch *et al.,* 2014). Because mortality in IBD is low (Duricova *et al.,* 2010; Burisch *et al.,* 2014) and the disease is most often diagnosed in the young (Loftus *et al.,* 2002; Burisch *et al.,* 2014), it is predicted that the global prevalence of IBD will continue to rise substantially in the next years (Abraham & Cho, 2009; Molodecky *et al.,* 2012). Although the etiology of IBD has been extensively studied in the past few decades (Podolsky, 2002), disease pathogenesis is notyet fully understood (Jones *et al.,* 2008; Mikhailov & Furner, 2009).

IBD encompasses three types of diseases: Crohn's disease (CD), ulcerative colitis (UC), and inflammatory bowel diseases undefined (IBDU). All of them are mainly characterized by chronic mucosal inflammation in pathologic histology of the gastrointestinal tract in susceptible individuals (Podolsky, 2002; Danese *et al.,* 2004; Abraham & Cho, 2009; Mikhailov & Furner, 2009). Whilst IBD significantly reduce the patient's quality of life and is likely to develop into pre-cancerous states (Xie & Itzkowitz, 2008; Triantafillidis *et al.,* 2009), overall IBD clinical treatments are prone to induce side effects and present unspecific targets, are extremely costly and their curative effects are not satisfying (Dyson *et al.,* 2012). According to Xie and Itzkowitz (2008), patients with long-standing IBD have an increased risk of developing CRC. Indeed, many of the molecular alterations responsible for sporadic CRC also play a role in colitis-associated colon carcinogenesis (Xie and Itzkowitz, 2008).

Numerous studies have documented the involvement of MMPs in inflammatory processes in animal models, cell lines, altered tissue cultures and biopsies of patients (Baugh *et al.,* 1999; Parks *et al.,* 2004; Murphy & Nagase, 2008; Lee *et al.,* 2013). The gelatinases MMP-9 and MMP-2 have for long been recognized as playing important roles in the turnover and degradation of extracellular matrix proteins during cellular recruitment in inflammation (Malla *et al.,* 2008) and in other pathological-associated oncologic processes, such as tumourigenesis, cell adhesion and metastasis (Herszényi *et al.,* 2012). Although similar in their substrate selectivity, MMP-2 is constitutively expressed in fibroblasts, endothelial cells and epithelial cells and is only moderately involved in inflammatory diseases (Huhtala *et al.,* 1991), whereas MMP-9 expression is observed primarily in leukocytes (Van den Steen *et al.,* 2002), being highly induced in response to a variety of inflammatory pathologies (Van den Steen *et al.,* 2002), and is the main gelatinase induced during ulcerative colitis and other IBD (Garg *et al.,* 2009; Moore *et al.,* 2011).

These findings turned MMP-9 into a desirable therapeutic target in IBD prevention and treatment, as well as in the prevention of earlier cancer stages and metastatic migration. However, studies relating MMP-9 inhibition to pre-clinical and clinical IBD reduction are very few and targeting MMPs has proven itself difficult. This could be achieved, at least partly, through the long-term ingestion of natural food-born specific MMP-9 inhibitors that are colon-available, rather than serum-bioavailable. In the last years, it has been highlighted that some foods with a nutritive function provide beneficial health effects in the prevention and treatment of certain diseases (Ortega, 2006; Sirtori *et al.,* 2009) and in the last decade a substantial amount of research has turned towards novel plant foods presenting MMPIs.

### MMPIs from Plant Seeds

For over 30 years now, MMPs have been considered by researchers across the world as attractive cancer targets. As a result, many chemical MMPIs were developed as potential anticancer drugs. Well known examples are provided by tetracylines, zoledronate, ethylenediaminetetraacetic acid (EDTA), 1,10-phenanthroline, 2S,3R-3-amino-2-hydroxy-4-(4-nitrophenyl)butanoyl-L-leucine, and neovastat (registered trade mark) (isolated from shark cartilage). Up to now, a myriad of MMPI has already been synthesized, some of which have been used as potential therapeutic agents to limit tumour progression (Bourguet *et al.,* 2012). However, only a few small MMPIs entered the clinical trial stage, most of which (e.g. batimastat^{®}, marimastat^{®}, solimastat^{®}, galardin^{®}, trocade^{®}, prinomastat^{®}, tanomastat^{®}, rebimastat^{®}) terminated prematurely either due to lack of benefits or to strong adverse side effects (Wang *et al.,* 2012). Thus, so far, most of the clinical trials in cancer were rather disappointing. Small, non-natural peptides have also been synthesized in an attempt to find novel MMPIs. Thus, for example, two non-natural dodecapeptides were identified as MMP-2 inhibitors by Lu *et al.* (2012), whereas an octadecapeptide was found to be an MMP-9 inhibitor (Qiu *et al.,* 2013).

A distinct and more recent strategy is to search for MMPIs among the multitude of natural products that nature placed at our disposal.

Plant organs and tissues, including seeds, have long been known to contain metabolites, peptides and proteins with an array of potentially useful bioactivities, many of which await discovery. One such bioactivity relates to their capacity to inhibit MMPs. In this respect, tables 2 and 4 from the work reported by Cyr (2001) provide a huge list of plants (either stressed and non-stressed) whose aqueous, ethanolic and organic extracts exhibit inhibitory activity upon human MMP-2 and MMP-9 enzymes, respectively. These extracts surely encompass secondary metabolites, as illustrated in the following additional examples. Withaferin A is a steroidal lactone, derived from Acnistus arborescens, Withania somnifera and other members of Solanaceae family, as well as some of its stable derivatives (e.g. 3-azido withaferin A; Rah *et al.,* 2012), abolished secretory MMP-2 expression and activity. The flavonoids chrysin, apigenin, genistein and their homoleptic copper(II) complexes have also been reported to attenuate the expression and secretion of the metastasis-relevant matrix metalloproteinases MMP-2 and MMP-9 (Spoerlein *et al.,* 2013).

Many seeds have been reported to contain MMPIs, such as those from grape (La *et al.,* 2009), soybean, sunflower (Ceccoli *et al.,* 2010), and dried longan (Euphoria longana Lam.) (Panyathep *et al.,* 2013). In the case of grapevine, proanthocyanidins are the MMP inhibitors (Vayalil & Mittal, 2004), whereas in the case of soybean, the flavonoid genistein and the peptide lunasin (see below) seem to be the active ingredients.

In addition, seeds, and legume seeds in particular, have been long recognized by containing a variety of proteinaceous enzyme inhibitors, such as the trypsin inhibitors and the Bowman-Birk inhibitors. Those from soybean and chickpea exhibit insect activity. On the other hand, Bowman-Birk inhibitors have been reported to prevent tumourigenesis and to affect the antimicrobial activity. As a result, Birk (1996) concluded that the in vitro effects of proteinase inhibitors on animals should be interpreted with caution when related to humans. However, it should be noted that all these proteinaceous enzyme inhibitors are inactivated by thermic (including cooking) treatment In this respect are proteins or glycoproteins from soya, rice, pea or lupine, and other plant extracts, which are known to inhibitMMPs (Stuhlmann & Joppe, 2013).

### A Specific Mention to Lunasin

Lunasin from soybean deserves a special mention. Lunasin is a 43 amino acid residue, chemopreventive peptide initially identified in soybean and then claimed to be present also in barley, wheat, rye, triticale, Solanum nigrum and Amaranthus seeds (Jeong *et al.,* 2007). Using monoclonal antibodies prepared against the 43 amino acid residue soybean lunasin, Herrera (2009) conducted a detailed study to detect this peptide in total protein extracts as well as soluble fractions from the seeds of cultivated and wild species of *Lupinus.* The applicant failed to detect lunasin in the albumin or globulin fractions from *L. albus.* In the analyzed species of the genus *Lupinus* a positive immunological signal was obtained for lunasin in the prolamin fraction of seeds of *L. albus* with testa and in the albumin and glutelin fractions from seeds without testa of L. montanus and L. stipulates, respectively. Lunasin was not detected in protein extracts of seeds without testa of *L. albus* and of seeds with and without testa of L. mutabilis. An immunological reaction was obtained for polypeptide bands greater than 25 kDa, a result probably derived from the presence of lectins or storage proteins exhibiting lectin acitivity in lupin cotyledons which are known to recognize and bind to the glycosylated IgGs after undergoing fractionation by SDS-PAGE and blotting into a membrane. Mitchell *et al.* (2013) could not find a gene (or a gene fragment) encoding lunasin in cereals and confirmed its presence in soybean and peanut.

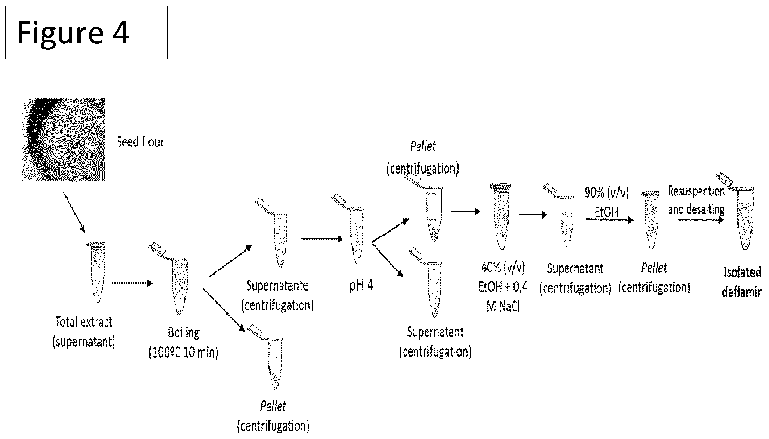

Lunasin is a small subunit peptide (SEQ ID NO: 191) derived from the larger cotyledon-specific 2S seed albumin (Gm2S-1) complex that has both anticancer and anti-inflammatory activities. Large-scale animal studies and human clinical trials to determine the efficacy of lunasin in vivo have been hampered by the cost of synthetic lunasin and the lack of a method for obtaining gram quantities of highly purified lunasin from plant sources (Seber *et al.,* 2012). A scalable method was developed that utilizes the sequential application of anion-exchange chromatography, ultrafiltration, and reversed-phase chromatography. This method generates lunasin preparations of 0.99% purity with a yield of 442 mg/kg defatted soy flour. The proposed mode of lunasin action, as presented by the authors in Figure 4 of Kyle *et al.* (2012), does not include a role of MMP inhibition, i.e. lunasin does not seem to interact physically with MMPs. Rather, physical interactions seem to take place between lunasin and chromatin and histones (Jiang *et al.,* 2016).

### A Brief Reference to the Lupinus Seed Storage Proteins

The main seed storage proteins in lupins, referred to as conglutins, have been classified into four families: α-, β-, γ- and δ-conglutins. β-Conglutin, the main seed globulin in lupins, is the vicilin or 7S member of the seed storage proteins, whereas α-conglutin is the legumin or 11S member of the seed storage proteins. In narrow-leafed lupin (*Lupinus angustifolius*), a total of three α-conglutin, seven β-conglutin, two γ-conglutin and four δ-conglutin encoding genes were previously identified (Foley *et al.,* 2011, 2015). These genes have been referred to as conglutin alpha 1, 2 and 3, conglutin beta 1, 2, 3, 4, 5, 6 and 7, conglutin gamma 1 and 2, and conglutin delta 1, 2, 3 and 4, respectively. In addition, the resulting polypeptides undergo extensive and complex processing and assembly processes, resulting in the high degree of microheterogeneity which characterizes these proteins.

A special reference to the *Lupinus* 2S protein, in this genus specifically termed δ-conglutin δ-Conglutin belongs to the 2S sulphur-rich albumin family. *Lupinus* seeds 2S albumin, also termed δ-conglutin (Sironi *et al.,* 2005), is a monomeric protein which comprises two small polypeptide chains linked by two interchain disulfide bonds: a smaller polypeptide chain, which consists of 37 amino acid residues resulting in a molecular mass of 4.4 kDa, and a larger polypeptide chain containing 75 amino acid residues with a molecular mass of 8.8 kDa (Salmanowich & Weder, 1997). The sole amino acid sequence of *L. albus* δ-conglutin has been inferred from the gene sequence. The larger polypeptide chain contains two intrachain disulfide bridges and one free sulfhydryl group (Salmanowich & Weder, 1997). This protein presents specific unique features among the proteins from *L. albus:* besides its high cystein content, it exhibits a low absorbance at 280 nm.

As far as the physiological role of δ-conglutin is concerned, a storage function has been proposed for this class of proteins. Structural similarity with the plant cereal inhibitor family, which includes bi-functional trypsin/alpha-amylase inhibitors, may suggest a defence function for this protein in addition to its storage role. Its presence in *L. albus* seeds was assessed to be around 10 to 12%, but more recent data suggest a lower content of around 3 to 4%. The *Lupinus* seed 2S albumin is typically present in both the albumin and the globulin fractions (Salmanowich & Przybylska, 1994), thus explaining our results obtained previously for the MMP inhibitory activity in the two protein fractions (Lima *et al.,* 2016).

### A Concise Definition of Blad

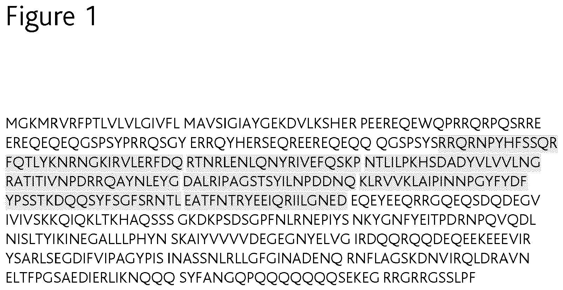

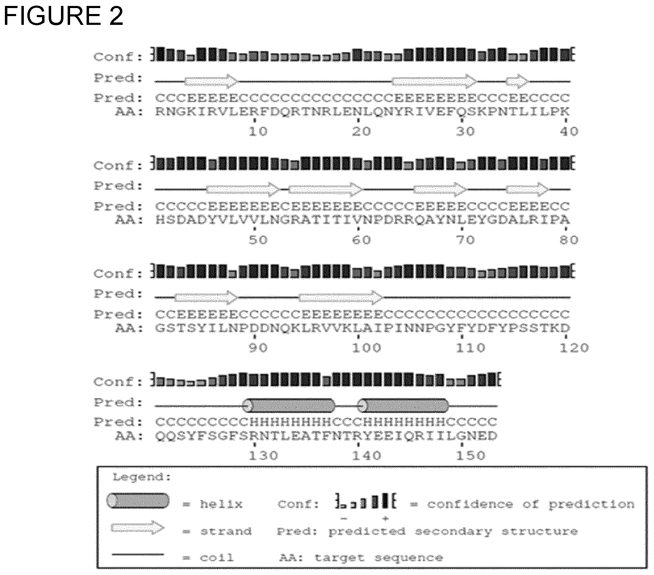

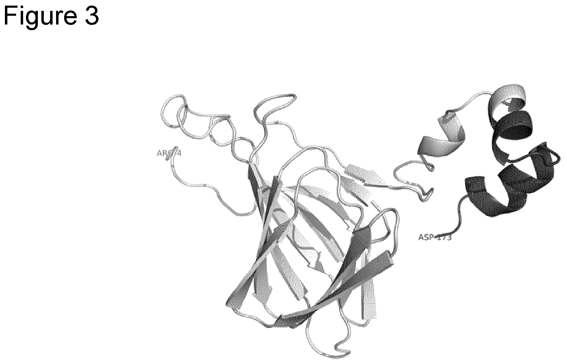

Blad is a 20,408.95 Da, 173 amino acid residue polypeptide which comprises residues 109 to 281 of the precursor of β-conglutin (i.e. pro-β-conglutin). β-Conglutin is a globulin and the major storage protein from *Lupinus* seeds (Figures 1, 2 and 3; Monteiro *et al.,* 2003, 2006). Under natural conditions, Blad accumulates in the cotyledons of *Lupinus* seedlings between the 4th and 14th day after the onset of germination.

### Anticancer Activities Present in Foodstuffs, with a Special Reference to Soybean

There is abundant evidence in the published literature concerning the anticancer activities of most edible foodstuffs. Legume seeds are no exception and these studies have focused primarily on soybean. Thus, there is much evidence suggesting that compounds present in soybeans can prevent cancer in many different organ systems. These include the Bowman-Birk inhibitor, the trypsin inhibitor, phytic acid, β-sitosterol, isoflavones (e.g. genistein and daidzein) and saponins (Kennedy, 1995). Legume seed proteins and soybean proteins in particular (including BBIs) have been reported to exhibit a role at the levels of anticancer and antimetastasis in various animal models (Roy *et al.,* 2010). Champ (2002) reported that BBI derived from soybean inhibited or prevented the development of chemically induced cancers of the liver, lung, colon, mouth and oesophagus in mice, rats and hamsters. Kennedy & Wan (2002) observed in vitro that 50 to 100 µg soybean BBI / mL decreased the prostate cancer cell migration. BBI inhibits MMPs and demonstrates efficacy against tumor cells in vitro, animal models, and human phase IIa clinical trials (Losso, 2008).

Currently, there are no prescription drugs that specifically target chronic inflammation. (There are, of course, over-the-counter medications that treat the minor and temporary inflammation and accompanying pain caused by injuries or procedures, such as surgery. However, these are not meant to treat chronic inflammation.) Some drugs, such as hydroxychloroquine, once used to battle malaria, are useful in treating some lupus patients, but they don't cure the disease. Aspirin and statins have shown promise in reducing inflammation in some people, but researchers aren't sure how broadly useful such drugs are in that role. With the exception of far-from-perfect anti-inflammatory drugs, such as prednisone, a corticosteroid that brings with it a slew of side effects, scientists are still researching how best to contain inflammation.

### Cell Invasion and Integrins

It is generally established that death of colorectal cancer patients is usually caused by metastatic disease rather than the primary tumor itself. Metastasis involves the release of the cancer cells from the primary tumour and attachment to another tissue or organ. Cancer cell invasion is therefore a key element in metastasis and requires a) Integrins for adhesion/de-adhesion and b) Matrix metalloproteinases (MMPs) for focalized proteolysis.

Focalized proteolysis is required to open up the path in the extracellular matrix for the cancer cells to travel across. The wound healing assays provide an estimate of the ability that cells have for cell invasion. Usually, MMP-9 activities are highly related to cancer cell invasion, hence the reduction in MMP-9 activity inhibits cell invasion and the two activities are usually paired. This is why MMP-9 inhibition is so desired, because it directly inhibits cell invasion, therefore inhibits death by metastasis.

On the other hand, cells adhere to a substrate through specific proteins called integrins, transmembrane receptors that are the bridges for cell-cell and cell-extracellular matrix (ECM) interactions. One important function of integrins on cells in tissue culture is their role in cell migration. Recent studies demonstrate that integrins are modulated by tumour progression and metastasis and are tightly connected to both MMP-9 and MMP-2 activities (Hood & Cheresh, 2002). Targeting and disabling integrins in cancer cells membranes can also be desirable because when cells are released from the tumor, they lack the ability to attach to another tissue. This will inhibit metastasis as well and can even help to disaggregate the primary tumor.

### Cell Proliferation and Metabolism

Another target for many studies is the specific cytotoxicity against cancer cells. Many bioactive compounds, such as phenolic compounds strongly reduce cell growth and impair metabolism, reaching toxic levels at high doses. The measurement of cell growth and cell metabolism in the presence of a bioactive compound is a direct measure of its toxicity to the cell. The MTT assay uses a specific coloring agent which needs to be absorbed to the living cells, and then metabolized by them in order to produce a purple color, which is then quantified by spectrophotometry. If the cell is dead, or metabolically impaired, it will not produce the coloring agent. Hence, higher levels of color are indicative of a higher number of living, metabolically active cells. If a compound reduces cell growth, or kills the cells, there will be a lower level in color.

Targeting and killing cancer cells is, in theory, a good approach. However, it can only work if there is a high specificity towards the cancer cells and not towards healthy, normal (i.e. non-cancer) cells. Most compounds that destroy cancer cells will also destroy normal healthy cells at a given dose, and although many studies focus only on the ability of a metabolite (e.g. phenolic compounds) to reduce cancer cell growth, they don't often take into account their effects on the control of healthy cells. One of the reasons why this is rather common, relates to the fact that unlike normal, healthy cells, it is relatively straightforward to culture cancer cells under laboratory conditions. Consequently, later-on, at the level of pre-clinical or even clinical assays, the use of these compounds becomes hampered by dose-limiting toxicity, insufficient clinical benefits and extremely adverse side-effects.

Therefore, a bioactive agent which reduces cell invasion but does not affect the cells normal metabolism (as appears to be the case with deflamin) will most likely produce less side-effects, and will be safer to use in preventive, long-term administrations, because it will not exert cytotoxicity towards regular colon cells.

It was not until about twenty years ago that the biological sciences `woke up' to secondary metabolism. This encompasses the more basic biochemistry and molecular biology, as well as those areas closer to application such as agriculture and human health and nutrition. Over the last 20 years, 25 to 30% of the new drugs entering the US market were discovered in plants. Worldwide, the over-the-counter value of these drugs is estimated at more than $40 billion annually. However, there is a major difference in dose-dependent bioactivity between bioactive secondary metabolites and small proteins / polypeptides such as the ones addressed in the present document

Bioactive beneficial secondary metabolites (e.g. antioxidant polyphenols) are typically effective at low concentrations. However, above a specific threshold they become highly toxic. On the contrary, small proteins / polypeptides are naturally either beneficial (e.g. deflamin) or toxic (e.g. ricin and the proteins present in the venoms of snakes and scorpions).

### MATERIALS AND METHODS

### Materials, Solvents and Reagents

2,4,6-Trinitrobenzenesulfonic acid (TNBS) 5% (w/v) aqueous solution was purchased from Sigma Chemical Co. Ketamine (Imalgene^{®} 1000) and xilazine (Rompun^{®} 2%) were purchased from Bio2 Produtos Veterinários (Lisbon, Portugal). All other reagents were purchased from Sigma-Aldrich (St. Louis, USA). Dye-quenched (DQ)-gelatin was purchased from Invitrogen (Carlsbad, CA, USA).

### Measurement of Antibacterial Activity

Antibacterial activities of deflamin were assessed in sterile 96-well plates (Greiner Bio-one, Germany), using the micro dilution method as described by Bouhdid *et al.* (2010). Briefly, 50 µL of Müller-Hinton media (Biokar, France) and 50 µL of deflamin solution (to obtain a deflamin concentration of 100 µg/mL) were added to the first well and serial diluted 1:2 to each adjacent well, up to 10 dilutions. Subsequently 50 µL of the bacterial suspension with a concentration of 2 × 105 UFC/mL, were added to the wells. A positive control (50 µL of Müller-Hinton media + 50 µL bacterial suspension) and a negative control (100 µL Müller-Hinton media) were performed. Plates were incubated for 24 h, at 37 ºC, and the absorbance was read at 546 nm (Synergy HT, Biotek, USA) in the beginning of the inoculation and at the end of the assay.

### Measurement of Antifungal Activity

A spore suspension was prepared by adding 20 mL of sterile water to 1 week old fungal cultures grown in Petri dishes containing PDA (potato dextrose agar) as culture medium. The growth conditions were 25 OC ± 1 OC, in the dark. The spore suspension was filtered and adjusted to the concentration of 105 spores/mL using a hematocytometer. Spore suspension (100 µL) was added to Petri dishes containing PDA medium and thoroughly spread on the surface of the dish with a sterile rake. Discs made of sterile filter paper (diameter: 6 mm) were soaked in 6 µL of a deflamin solution (100 µg/mL) and deposited on the surface of the medium. Controls were made by soaking sterile filter paper discs with 6 µL of sterile water. The Petri dishes where then stored in an incubator (25 OC, in the dark) and the fungal growth was monitored during a 2 week period.

### Minimal Inhibitory Concentrations (MICs)

Minimal inhibitory concentrations (MICs) were assessed in sterile 96-well plates (Greiner Bio-one, Germany), using the micro dilution method as described before (Bouhdid *et al.,* 2010). Briefly, 50 µL of RPMI medium was added to each well. Then, 50 µL of each sample was added to the first well and serially diluted 1:2 to each adjacent well, up to 10 dilutions. Subsequently, 50 µL of the HT29 cell suspension with a concentration of 2 × 105 cells/mL, was added to the wells. A positive control (50 µL RPMI medium + 50 µL cell suspension) and a negative control (100 µL RPMI medium) were performed. Plates were incubated for 24 h, at 37 ºC, and cell growth was measured by the 3-(4,5-dimethylthiazol-2-yl)-2,5-diphenyltetrazolium bromide (MTT) assay (Carmichael *et al.,* 1987). For MMP-9 MIC determination, the media from each well was collected and gelatinolytic activities were determined with DQ-gelatin, as described below.

### Seeds

In certain embodiments, dry seeds of the following legume species were employed: white lupin (*Lupinus albus* L.), chickpea (*Cicerarietinum* L.) and soybean (*Glycine max* L.). Whenever required, other legume seeds were also used: lentil (*Lens culinaris M.*), common bean (*Phaseolus vulgaris L.),* pea (*Pisum sativum L.*), broad bean (*Vicia faba L.*), and cowpea (*Vigna unguiculata L.*)*.*

### Cooked Seeds

Legume seeds, as many other seeds are well-known to contain anti-nutritional factors, such as inhibitors of digestive enzymes, lectins, high phytate concentrations, non-protein amino acids, etc. Therefore, they must be ingested after cooking to ensure denaturation of the proteinaceous anti-nutritional factors. For these reasons and because deflamin was found to resist boiling, a number of initial experiments was performed using cooked seeds. To simulate cooking conditions, the dry seeds were boiled in distilled water (w/v) until acquiring a soft, suitable-to-eat texture (Xu & Chang, 2008).

### Extraction of Proteins and of Non-Protein Compounds Extraction of Total Soluble Protein Extraction of Total Soluble Protein

In certain embodiments, total soluble proteins from seeds were extracted by stirring for 2 to 3 h at room temperature, in 100 mM Tris-HCl buffer, pH 7.5, at a ratio of 1:5 (w/v), containing polyvinylpolypyrrolidone (0.5 g PVPP per 0.5 g fresh weight) and stirring for 4 h at 4 degrees Celsius. The slurry was then centrifuged at 12,000 g for 60 min at 4 degrees Celsius (Beckman J2 - 21M/E, rotor JA 20.000). The supernatant was kept and stored in a freezer at-20 °C.

### Extraction of Non-Protein Compounds

For total phenolic extractions, seeds were milled to flour, deffated with n-hexane and extracted by adding 10 mL acetone in water (50%, v/v) per 1 g fresh weight. Samples were stirred for 4 h at room temperature and centrifuged at 12,000 g for 10 min at 4 degrees Celsius (Beckman J2 - 21M/E, rotor JA 20.000). The procedure was repeated twice and the supernatants were collected, pooled and stored frozen for further analysis. The supernatants were evaporated at 60 ºC until dryness and the resulting extractwas ressuspended in reaction buffer (50 mM Tris-HCl buffer,pH 7.6, containing 150 mM NaCl, 5 mM CaCl2 and 0.01% v/v Tween 20 with 12% v/v ethanol).

### Extraction and Isolation of Deflamin from Seeds

Dry, mature seed of *Lupinus albus* L. (lupin), was used in this part of the work. In certain embodiments, identical procedures were followed for the seed of other species. The MMPI protein extract was isolated using its ability to resist boiling and acid denaturation. A method was developed to isolate deflamin from seeds which is suitable to undergo scaling-up to an industrial scale (Figure 4).

In an embodiment, the method to purify deflamin is a clean procedure which follows a sequential precipitation scheme. It is based on deflamin resistance to high temperatures, low pH and high ethanol concentrations, and involves the following steps:
- Approximately 100 g ± 0.1 g of dry lupin seed (without embryo and tegument) are milled to flour.
- Flour is deffated with n-hexane and then suspended in water (1:20 w/v; pH adjusted to pH 8.0-8.5), under stirring for 2-3 h at room temperature or
- the fat is simply removed from the top of the slurry after suspension of the flour in water (1:20 w/v; pH adjusted to pH 8.0-8.5), under stirring for 2-3 h at room temperature. As an alternative to water, at a lab scale, the flour may be suspended in 50 mM Tris-HCl buffer, pH 7.5. After stirring for 4 h (or overnight) at 4 degrees C, the soluble proteins are obtained by centrifugation at 13,500 g for 30 min at 4 degrees C. The pellet is discarded.
- The protein solution is boiled at 100 degrees C for 10 min and centrifuged at 13500 g for 20 min at 4 degrees C. The pellet is discarded. The supernatant was collected and provided the Heat treated extract (HT).
- Polypeptides/proteins in the supernatant are precipitated by addition of diluted HCl down to pH 4.0. Upon centrifugation at 13,500 g for 20 min at 4 degrees C, the supernatant is discarded.
- The pellet is re-suspended in 40% (v/v) ethanol containing 0.4 M NaCl, stirred for 1 h at room temperature and centrifuged at 13,500 g for 30 min at 4 degrees C. This treatment brings deflamin into solution, unlike the vast majority of the seed storage proteins. The pellet is discarded.
- The supernatant is made to 90% (v/v) ethanol and stored at -20 degrees C overnight, precipitating deflamin, followed by centrifugation at 13,500 g for 30 min at 4 degrees C. The supernatant is discarded.

For best results and to obtain a purer and cleaner deflamin fraction, the 40 to 90% (v/v) ethanol differential precipitation should be repeated. Thus,
- Deflamin present in the pellet is once again dissolved in 40% (v/v) ethanol containing 0.4 M NaCl, stirred for 1 h at room temperature and centrifuged at 13,500 g for 30 min at 4 degrees C. This second wash cleans deflamin from final contaminants. The pellet is again discarded.
- The supernatant is once more made to 90% (v/v) ethanol and stored at -20 degrees C overnight, precipitating deflamin, followed by centrifugation at 13,500 g for 30 min at 4 degrees C. The supernatant is discarded.
- The final pellet contains pure deflamin, which is subsequently dissolved in the smallest possible volume of Milli-Q water. At a lab scale, the deflamin solution is finally desalted into water in Sephadex G-25 columns (NAP-10 columns, GE Healthcare Life Sciences) to remove low molecular mass contaminants.
- The extract obtained was stored in falcon tubes at -20 degrees C.

This methodology was successful in isolating deflamin from *L. albus, Cicerarietinum* and *Glycine max.*

### Deflamin Fractionation by High-Performance Liquid Chromatography

In certain embodiments, deflamin constituent polypeptides were fractionated in a High-Performance Liquid Chromatography (HPLC) device (Waters 2695 Separations Module) equipped with a Waters 2998 Photodiode Array Detector. Protein samples were separated in a C18 reverse phase column, Zorbax 300SB 5 µm, 250 mm × 4.6 mm. The elution was made with eluant A (0.1% v/v trifluoroacetic acid, TFA) and solvent B (acetonitrile in 0.1% v/v TFA). Peak detection was made at both 214 nm and 280 nm.

### Sodium Dodecyl Sulphate-Polyacrylamide Gel Electrophoresis (SDS-PAGE)

In certain embodiments, samples were treated with 100 mM Tris-HCl buffer, pH 6.8, containing 100 mM β-mercaptoethanol, 2% (w/v) SDS, 15% (v/v) glycerol and 0.006% (w/v) m-cresol purple, and heated at 100 degrees C for 5 min. One-dimension electrophoresis was carried out, following the method described by Laemmli (1970) in a 12.5% (w/v) acrylamide resolving gel and a 5% (w/v) acrylamide stacking gel, and performed in a vertical electrophoresis unit at 100 V and 20 mA per gel. Gels were fixed for 20 min in 10% (w/v) TCA, and stained in 0.25% (w/v) Coomassie Brilliant Blue R-250 (CBB R-250), 25% (v/v) 2-propanol and 10% (v/v) acetic acid. Destaining was carried in a solution of 25% (v/v) 2-propanol and 10% (v/v) acetic acid.

### Mass Spectrometry (MS) Analysis

In certain embodiments, selected isolated peaks were analyzed by LC/MS on a 5600 TripleTOF (ABSciex^{®}) in information-dependent acquisition (IDA) mode. Peptides were resolved by liquid chromatography (nanoLC Ultra 2D, Eksigent^{®}) on a MicroLC column ChromXPTM C18CL reverse phase column (300 µm ID × 15 cm length, 3 µm particles, 120 Å pore size, Eksigent^{®}) at 5 µL/min. Peptides were eluted into the mass spectrometer with a multistep gradient: 0-2 min linear gradient from 5 to 10%, 2-45 min linear gradient from 10% to 30%, and 45-46 min to 35% of acetonitrile in 0.1% FA. Peptides were eluted into the mass spectrometer using an electrospray ionization source (DuoSpray^{™} Source, AB Sciex) with a 50 µm internal diameter (ID) stainless steel emitter (New Objective).

For information dependent acquisition (IDA) experiments the mass spectrometer was set to scanning full spectra (350-1250 m/z) for 250 ms, followed by up to 100 MS/MS scans (100-1500 m/z from a dynamic accumulation time - minimum 30 ms for precursor above the intensity threshold of 1000 - in order to maintain a cycle time of 3.3 s). Candidate ions with a charge state between +2 and +5 and counts above a minimum threshold of 10 counts per second were isolated for fragmentation and one MS/MS spectra was collected before adding those ions to the exclusion list for 25 s (mass spectrometer operated by Analyst^{®} TF 1.6, ABSciex^{®}). Rolling collision was used with a collision energy spread of 5. Two IDA experiments were performed for each sample with the second analysis performed with an exclusion list of the peptides previously identified.

Protein identification was obtained using Protein Pilot^{™} software (v 5.0, ABSciex^{®}) with the following search parameters: identification from uniprot database from March 2016, with no alkylation or digestion for the peptide samples. As a criteria for protein filtering we used 1.3 unused score value and a 95% peptide confidence filtering and >0 contribution.

### Cell Cultures

### Biological Material

The human colon adenocarcinoma cell line, HT29 (ECACC 85061109), obtained from an adenocarcinoma from a 44 year old Caucasian female, was used throughout this work. HT29 cell lines were maintained in RPMI medium supplemented with 10% (w/v) heat-inactivated fetal bovine serum (FBS) and 200 mM glutamine, 2x104 IU.mL-1 penicillin and 20 mg.mL-1 streptomycin at 37 °C in a humidified atmosphere of 5% (v/v) CO2. Routine observation for cell viability was performed by inverted microscopy.

### Cell Proliferation, Adhesion and Viability Assays

HT29 cultured cells were seeded on 96-well plates (2X104/well), samples were added to the growth media at the required concentrations (e.g. 100 µg/mL) and incubated for 24 h. After each treatment, the extracellular media was collected, and cells were washed with phosphate buffered saline (PBS) to remove unattached cells. Cells that were attached to the bottom were harvested with 0.15% (w/v) trypsin in phosphate buffer solution. Cell proliferation and viability were determined using the standard 3-(4,5-dimethylthiazol-2-yl)-2,5-diphenyltetrazolium bromide (MTT) assay as described by Carmichael *et al.* (1987). Cell number was determined using a hemocytometer, with trypan blue staining, which allowed to quantify a) cell adhesion, b) cytotoxicity, and c) cell growth. All treatments were done in duplicate in at least 3 independent experiments.

### Cell Migration Assays: in vitro Wound Assay

In certain embodiments, for cell migration analysis, the wound healing assay was performed. HT29 cells (5×105 cells/well) were seeded in 6-well plates and allowed to reach to 80% confluence. 'Wounds' were performed by making a scratch across the cell monolayer to create an open gap, thus mimicking a wound. Cells were then washed twice with PBS to remove floating debris. Each well was subsequently filled with fresh media with or without the presence of different concentrations of potential inhibitors, at different concentrations (e.g. 100 µg/mL), and allowed to grow up to 48 h. At the end of treatments, the invaded area or the number of cells in the scratch area of each well was determined and compared to the initial area at 0 h, and cells were photographed under a phase-contrast microscope. The area covered by migrating cells into the denuded zone at the beginning of treatment was computed. This comparison allowed us to assess the inhibitory effect (if any) exerted by each protein fraction on the HT29 cell migrating capacity. The area of cell migration was counted in three to five random fields from each triplicate treatment and expressed as percentage related to time 0 (covering area by migrated cells to the denuded zone at the beginning of the treatment).

### Enzymatic Assays: the effect of deflamin on MMP-9 and MMP-2 catalytic activities

### Gelatinolytic Activity Quantification with MMP-9

For the fluorogenic gelatin assay, DQ-gelatin was purchased from Invitrogen (Carlsbad, CA, USA) and dissolved in water at 1 mg/mL. All solutions and dilutions were prepared in assay-buffer (50 mM Tris-HCl buffer, pH 7.6, containing 150 mM NaCl, 5 mM CaCl2 and 0.01% v/v Tween 20). In all experiments, DQ-gelatin was used at a concentration of 2.5 µg/mL.

To a 96-well plate Macro-assay plate (chimney, 96-well, black), 0.1 nM (for a final volume of 200 µL) of the enzyme (MMP-9, Sigma) was added. For inhibitor tests, the required amount of inhibitor was added (e.g. 100 µg/mL) and the plate was incubated for 1 h at 37 degrees C. Subsequently, DQ-gelatin at a final concentration of 2.5 µg/mL was added and allowed to incubate again for 1 h. Fluorescence levels were then measured (ex. 485 nm/em. 530 nm). In each experiment, both positive (no inhibitor) and negative (no enzyme) controls were included to correct for possible proteolytic activities present in the protein samples. All data were corrected by subtraction of their respective negative controls.

### Gelatinolytic Activity Quantification in Cell Media

Extracellular HT29 media was collected to quantify the gelatinolytic activities present after exposure to the different inhibitors. The activity detected is due to the presence of both enzymes, MMP-9 and MM-2. The assay was conducted the same way as described previously, except that HT29 media from each treatment was added to each well (150 µL).

### Total Gelatinolytic Activity from Colonic Tissues

Combined MMP-9 and MMP-2 activities were determined in the colons from mice exposed to deflamin extract treatments, as described by Medina *et al.* (2006) with few alterations. Briefly, colonic tissue was homogenized in a 1/100 ratio (w/v) in 50 mM Tris-HCl buffer, pH7.6, containing 150 mM NaCl. Samples were sonicated three times for 10 s each at 1 min intervals. After 10 min on ice, protein extracts were centrifuged for 10 min at 13,000 g and 4 °C, the supernatants were preserved, and protein concentrations were determined by a modification of the Lowry method (Lowry *et al.,* 1951). Samples were stored at-80 °C until assayed. Protein extraction from each colon, as described above, was used to quantify the respective gelatinolytic activities. The fluorogenic substrate dye-quenched (DQ)-gelatin purchased from Invitrogen (Carlsbad, CA, USA) was used to quantify MMP-9 and MMP-2 activities. DQ gelatin was dissolved in water at 1 mg/mL as per the manufacturer's instructions. All solutions and dilutions were prepared in assay-buffer (50 mM Tris-HCl buffer, pH 7.6, containing 150 mM NaCl, 5 mM CaCl2 and 0.01% v/v Tween 20). A 96-well micro-assay plate (chimney, 96-well, black) was used. Each colonic tissue supernatant from each treatment was loaded (100 µL) in each well. Subsequently, DQ-gelatin (at a final concentration of 2.5 µg/mL) was added to each well and the plate was allowed to incubate for 1 h. Fluorescence levels were measured (ex. 485 nm/em. 530 nm). All data were corrected by subtracting their corresponding negative controls.

### Zymography

In the zymographic assays, two enzymes (MMP-9 and MMP-2, commonly known as gelatinases) were detected as well as their corresponding zymogens or pro-enzymes (pro-MMP-9 and pro-MMP-2). By definition, a zymogen or a pro-enzyme is inactive due to the presence of an amino acid short sequence (termed pro-sequence) which typically blocks access to the active site. In the present situation, MMP enzymes are synthesized in this form to prevent, for example, that they start degrading the ribosome while still attached to it during protein synthesis. In this way, these proteins are synthesized in an inactive form and are converted into their mature, native form in their site of action. In the zymography assays described here, the pro-gelatinases also become active because they are denaturated by the SDS, thus exposing the catalytic site - Hence the slightly higher mass of the pro-enzymes in the zymographic gels because they still maintain the short amino acid sequence of the cysteine switch.

### Gelatin Zymography

Gelatin-zymography was performed according to standard methods with the following modifications: to determine the metalloproteinase activity in the culture supernatants of HT29 cancer cell lines, SDS-polyacrylamide gels (12.5% w/v acrylamide) copolymerized with 1% (w/v) gelatin were prepared. The cell culture supernatants were treated with a non-reducing buffer containing 62.6 mM Tris-HCl pH 6.8, 2% (w/v) SDS, 10% (v/v) glycerol and 0.01% (w/v) bromophenol blue, and were loaded into each well. Electrophoresis was carried out at 100 V for 2 h. After electrophoresis, gels were washed three times in renaturing buffer (2.5% v/v Triton X-100) for 60 min each, to remove the SDS. The gels were then incubated overnight with developing buffer (50 mM Tris-HCl buffer, pH 7.4, containing 5 mM CaCl2, 1 µM ZnCl2 and 0.01% w/v sodium azide). The gels were stained with 0.5% (w/v) Coomassie Brilliant Blue G-250 for 30 min and destained with a solution of methanol: acetic acid : water (50 : 10 : 40). Protein band intensities were determined by densitometry measuring the peak areas using an image processing software.

### Reverse Gelatin Zymography

Reverse gelatin zymography, used to detect and quantify MMPI proteins in different samples, was performed as described in Hawkes *et al.* (2001, 2010), with some modifications. Protein samples were treated with zymographic buffer (313 mM Tris-HCl buffer, pH 6.8, containing 10% (w/v) SDS, 50% (v/v) glycerol and 0.05% (w/v) bromophenol blue) and were loaded in SDS-polyacrylamide (12.5% w/v acrylamide) slab gels copolymerized with gelatin (1% w/v) and conditioned medium (1.0 mL) from a cell line expressing MMP-2 and MMP-9 or with different MMP-9 concentrations (e.g. 1 µmol/mL). Electrophoresis was performed as described above and the gels were washed three times (for 60 min each) in 2.5% (v/v) Triton X-100, to remove the SDS, and incubated overnight as described above for substrate zymography. The gels were stained with 0.5% (w/v) Coomassie brilliant blue G-250. Dark zones marked the MMPI-mediated inhibition of gelatin degradation. Dark bands visible against a white background marked the MMPI-mediated inhibition of gelatin degradation (Hawkes, 2001).

### Gelatin Zymography of Colon Extracts

To determine the specific metalloproteinase activities in colon extraction supernatants, a gelatin-zymography was performed according to standard methods (Toth *et al.,* 2012), with the following modifications: SDS-polyacrylamide gels (12.5% w/v acrylamide) were copolymerized with 1% (w/v) gelatin. Colon extraction supernatants, previously treated with a non-reducing buffer containing 62.6 mM Tris-HCl buffer, pH 6.8, 2% (w/v) SDS, 10% (v/v) glycerol and 0.01% (w/v) bromophenol blue, were loaded into each well of the SDS-gel. Electrophoresis was carried out as described above, in a 12.5% (w/v) acrylamide resolving gel and a 5% (w/v) acrylamide stacking gel, performed in a vertical electrophoresis unit at 200 V and 20 mA per gel. After electrophoresis, gels were washed three times in 2.5% (v/v) Triton X-100 for 60 min each, to remove the SDS. Gels were then incubated for two days with developing buffer (50 mM Tris-HCl buffer, pH 7.4, containing 5 mM CaCl2, 1 µM ZnCl2 and 0.01% w/v sodium azide), stained for 30 min with Coomassie Brilliant Blue G-250 0.5% (w/v) in 50% (v/v) methanol and 10% (v/v) acetic acid, and destained with a solution of 50% (v/v) methanol, 10% (v/v) acetic acid. White bands visible against a blue background marked the gelatinase activity of each proteinases (Toth *et al.,* 2012).

### Animal Models of Experimental Inflammatory Diseases

### Colitis Model of Inflammation

### Animals

Male CD-1 mice, 25 to 30 g in weight and 5 to 6 weeks of age (Harlan Iberica, Barcelona, Spain) were housed in standard polypropylene cages with ad libitum access to food and water, under a controlled environment in a room kept at 22 degrees C ± 1 degree C with a 12 h light, 12 h dark cycle at the Faculty of Pharmacy, Central Animal Facility, University of Lisbon.

### Animal Care and Maintenance for the in vivo Experiments

Experiments were conducted according to the Home Office Guidance in the Operation of Animals (Scientific Procedures) Act 1986, published by Her Majesty's Stationary Office, London, UK and the Institutional Animal Research Committee Guide for the Care and Use of Laboratory Animals published by the US National Institutes of Health (NIH Publication no. 85-23, revised 1996), as well as to the currently adopted EC regulations (Directive 2010/63/EU). Finally, the studies performed are in compliance with the ARRIVE Guidelines for Reporting Animal Research' summarized at http://www.nc3rs.org.uk. This experimental protocol was also endorsed by the Ethics Committee of the Faculty of Pharmacy, University of Lisbon. In addition, colleagues from the Faculty of Pharmacy, University of Lisbon, are licensed by the Portuguese General Directorate of Veterinary to coordinate and conduct independent animal research. All studies were carried out using male, 5 weeks old Wistar rats, weighing 100 to 150 g (Harlan Iberica, Barcelona, Spain). All animals received a standard diet and water ad libitum.

### Colitis Model Using Oral Administration (p.o.) of Deflamin

### Induction of Experimental Colitis

2,4,6-Trinitrobenzene sulphonic acid (TNBS) was instilled as an intracolonic single dose as previously described before (Impellizzeri *et al.,* 2015). Briefly, mice were left unfed during 24 h. On the induction day (day 0), mice were anesthetized with 100 mg/kg ketamine and 10 mg/kg xilazine. Then, 100 µL of TNBS solution was administered through a catheter carefully inserted until 4,5 cm into the colon. Mice were kept for 1 min in a Tredelenburg position to avoid reflux. Four days after induction, mice were anesthetized, and blood samples were collected by cardiac puncture. Mice were euthanized by cervical dislocation and necropsied. The abdomen was opened by a midline incision. The colon was removed, freed from surrounding tissues and opened longitudinally for observation and classification of diarrhea severity. Afterwards, the colon was washed with phosphate buffered saline for macroscopical observation of the tissue and subjected to biochemical analyses or subsequently fixed in paraformaldehyde for further processing.

### Experimental Groups

Animals were randomly allocated into four experimental groups as described:
1. Sham group (n = 6): animals were subjected to the procedures described above except the intracolonic administration was with 100 µL of saline solution. During the 4 days of the protocol the animals were administered orally with 10 mL/kg of distilled water.
2. Ethanol group (n = 6): animals were subjected to the procedures described above except the intracolonic administration was with 100 µL of a 50% (v/v) ethanol/water solution. During the 4 days of the protocol the animals were administered orally with 10 mL/kg of distilled water.
3. TNBS group (n = 10): animals were administered 100 µL of 2.5% (w/v) TNBS in 50% (v/v) ethanol. During the 4 days of the protocol the animals were administered orally with 10 mL/kg of distilled water.
4. TNBS + extract (or deflamin) group (n =10): animals were administered with 100 µL of 2.5% (w/v) TNBS in 50% (v/v) ethanol. During the 4 days of the protocol the animals were administered orally (p.o.) with extract (or deflamin; 15 mg/kg).

Oral administrations were performed daily, starting from 3 h after the initial administration of TNBS, by gastric gavage.

### Macroscopic Evaluation of Colitis Severity

After colon removal, a longitudinal incision was performed for observation of content and classification of diarrhea severity by an observer blinded regarding the experimental groups. Afterwards, the colon was rinsed with saline and observed macroscopically through a surgical microscope for closer observation of the tissue and capture of lesion pictures. The colon was then measured, as well as the extent of injury (if present).

### Evaluation of Hemorrhagic Injury

### Fecal hemoglobin, as an index of hemorrhagic injury, was measured using a quantitative method by immunoturbidimetry (Kroma Systems)

### Histology and Immunohistochemistry Procedures

Colons were removed, fixed in 4% (w/v) paraformaldehyde in PBS for 72 h at room temperature, dehydrated through a graded ethanol series and embedded in paraffin (n = 3 per group). Hematoxilin & Eosin (H&E) staining was performed as previously described (Rocha *et al.,* 2015) and images were acquired using a bright field Axioscop microscope (Zeiss, Göttingen, Germany).

The degree of inflammation and colon damage on microscopic cross-sections was graded semi-quantitatively from 0 to 3: 0, normal colon with no lesions, mucosa of uniform thickness, crypts straight, normal crypt architecture, no cellular infiltration, edema or exudate meaning no signs of inflammation; 1, colon with mild lesions, mucosal erosion and small superficial ulcers scattered along the length of the colon, with slight crypt loss and mononuclear cell infiltration; 2, colon with moderate lesions, intestines with extensive erosion and ulceration, with moderate crypt loss and neutrophil infiltration; 3, colon with very severe ulceration, thin mucosa with loss of crypts and markedly increased infiltration of neutrophils and acute inflammatory exudate.

For immunostaining, 6 µm thick sections were submitted to antigen retrieval in 20 mM citrate buffer with 1.5% (v/v) H2O2 for 15 min at room temperature in the dark, incubated for 10 min in Tris/EDTA buffer at 84 °C and blocked for 1 h at room temperature in 1% (w/v) bovine serum albumin (BSA) in PBS. Primary antibodies [rabbit anti-COX2 (Cell Signaling #4842, 1:100) and mouse anti-iNOS (BD Transduction Laboratories #610328, 1:100)] were used in 0.5% (w/v) BSA in PBS overnight at 4 degrees C. After washing in PBS, sections were incubated for 1 h at room temperature with antibodies anti-rabbit coupled to horseradish peroxidase (Santa Cruz Biotechnology, 1:5000) in 0.5% (w/v) BSA in PBS, incubated for 10 min in SIGMAFAST DAB with Metal Enhancer (Sigma, USA) and mounted with Entellan (Merck, Germany). Tissue sections were visualized with a AxioScope brightfield microscope (Zeiss, Göttingen, Germany).

### Colitis Model Using Intraperitoneal Injection (i.p.) Administration of Deflamin

The anti-inflammatory effect of deflamin against colitis administrated intraperitoneally was also tested. Mice were treated exactly as described above, except that deflamin extract was administered via intraperitoneal injection. The following experimental groups were tested:
1. Sham group (n = 6): animals were subjected to the procedures described above except the intracolonic administration was with 100 µL of saline solution. During the 4 days of the protocol the animals were administered orally with 10 mL/kg of distilled water or injected intraperitoneally with the same amount of saline.
2. Ethanol group (n = 6): animals were subjected to the procedures described above except the intracolonic administration was with 100 µL of a 50% (v/v) ethanol/water solution. During the 4 days of the protocol the animals were administered orally with 10 mL/kg of distilled water injected intraperitoneally with the same amount of saline.
3. TNBS group (n = 10): animals were administered with 100 µL of 2.5% (w/v) TNBS in 50% (v/v) ethanol. During the 4 days of the protocol the animals were administered orally with 10 mL/kg of distilled water injected intraperitoneally with the same amount of saline.
4. TNBS + extract (or deflamin) group (n = 10): animals were administered with 100 µL of 2.5% (w/v) TNBS in 50% (v/v) ethanol. During the 4 days of the protocol the animals were injected intraperitoneally (i.p.) with extract (or deflamin; 10 mg/kg).

Intraperitoneal injection administrations were performed daily, starting from 3 h after the initial administration of TNBS, by gastric gavage, as described before, and the same evaluations were performed, as described for oral administrations.

### Preventive Effects vs Curative Effects of Deflamin

To compare the preventive effect of deflamin with its curative effect, mice were maintained and treated as described above and randomly allocated into four experimental groups:
1. Sham group (n = 6): animals were subjected to the procedures described above except the intracolonic administration was with 100 µL of saline solution. During the 4 days of the protocol the animals were administered orally with 10 mL/kg of distilled water.
2. TNBS group (n = 10): animals were administered with 100 µL of 2.5% (w/v) TNBS in 50% (v/v) ethanol. During the 4 days of the protocol the animals were administered orally with 10 mL/kg of distilled water.
3. TNBS + deflamin p.o. (n = 9): animals were administered with 100 µL of 2.5% (w/v) TNBS in 50% (v/v) ethanol. During the 4 days of the protocol the animals were administered orally with deflamin (15 mg/kg).
4. Deflamin preventive treatments + TNBS (n = 10): Three days before of TNBS induction, animals were administered orally with deflamin (15 mg/kg). Animals were then administered with 100 µL of 2.5% (w/v) TNBS in 50% (v/v) ethanol.

During the 4 days of the protocol the animals were administered orally with deflamin (15 mg/kg).

After the 4 days experiment, macroscopic evaluation of colitis severity was performed as described above.

### Carrageenan-Induced Paw Oedema Model of Inflammation

### Animal Care and Maintenance for the in vivo Experiments

Experiments were conducted according to the Home Office Guidance in the Operation of Animals (Scientific Procedures) Act 1986, published by Her Majesty's Stationary Office, London, UK and the Institutional Animal Research Committee Guide for the Care and Use of Laboratory Animals published by the US National Institutes of Health (NIH Publication no. 85-23, revised 1996), as well as to the currently adopted EC regulations. Finally, the studies are in compliance with the ARRIVE Guidelines for Reporting Animal Research' summarized at http://www.nc3rs.org.uk. Hence, the Ethics Committee of the Research Institute endorsed the animal study protocol, considering also that authors Sepodes and Rocha are licensed by the Portuguese General Directorate of Veterinary to coordinate and conduct independent animal research. All studies were carried out using male Wistar rats of 5 weeks of age weighing 100 to 150 g (Harlan Iberica, Barcelona, Spain). All animals received a standard diet and water *ad libitum.*

### Paw Oedema Model of Inflammation Using Oral Administration of Deflamin

Induction of experimental paw oedema and evaluation of oedema severity. The carrageenan-induced paw oedema of the rat hind paw is a suitable model to study acute local inflammation and widely considered to be one of the most useful models in the evaluation of anti-inflammatory activity. This model was used to test the anti-inflammatory activity of deflamin administered orally or topically.

### The Paw Oedema Model

Paw oedema was induced by a single sub-plantar injection into the rat left hind paw of 0.1 mL of a 1% (w/v) λ-carrageenan sterile saline solution. The paw volume was measured by means of a volume displacement method using a plethysmometer (Digital Plethysmometer LE7500; Letica Scientific Instruments, Letica, Spain). The pawvolume was measured immediately after the injection of carrageenan (V0 or basal volume) and 6 h later (V6 h). The increase in paw volume was taken as the oedema volume.

### Experimental Groups

Animals were randomly allocated into the following groups as described:
1. Control group (n = 6): animals were subjected to subplantar injection into the rat left hind paw of 0.1 mL sterile saline and administered with saline (1 mL/kg, i.p.).
2. Carrageenan group (n = 6): animals were subjected to paw oedema induction and administered with saline (1 mL/kg, i.p.).
3. Deflamin group (n = 5): animals were subjected to paw oedema induction and pre-treated with the deflamin extract (15 mg crude extract per kg, administered orally and i.p.) 30 min before λ-carrageenan injection.
4. Indomethacin group (n = 6): animals were subjected to paw oedema induction and pre-treated with indomethacin (10 mg/kg, i.p.) 30 min before λ-carrageenan injection.
5. Trolox group (n = 6): animals were subjected to paw oedema induction and pre-treated with Trolox (30 mg/kg p.o.) 30 min before λ-carrageenan injection.

### Colitis Model Using Topical Administration of Deflamin

Animals were essentially treated as described before, and allocated in groups as described:
1. Control group (n = 6): animals were subjected to subplantar injection into the rat left hind paw of 0.1 mL sterile saline and administered with saline (1 mL/kg, i.p.).
2. Carrageenan group (n = 6): animals were subjected to paw oedema induction and administered with saline (1 mL/kg, i.p.).
3. Deflamin group (n = 5): animals were subjected to paw oedema induction and topically treated with the deflamin extract (dissolved in water and glycerol 30:70, v/v) after λ-carrageenan injection.
4. Control group (n = 6): animals were subjected to paw oedema induction and topically treated with the glycerol solution (water and glycerol 30:70, v/v) after λ-carrageenan injection.

### Statistical Analysis

For the animal colitis model, all results were expressed as mean ± SEM of n observations, where n represents the number of animals studied. Results were compared using a one-factorial ANOVA test, followed by a Bonferroni's post hoc test using GraphPad Prism 5.0 software (GraphPad, San Diego, CA, USA). For gelatinolytic activities, all experiments were performed in triplicate, in at least three independent times and the data were expressed as the mean 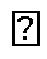 standard deviation (SD). SigmaPlot software (version 12.5) was used for comparing different treatments, using one-way and two-way analysis of variance (ANOVA). Tukey's test was used to compare differences between groups and the statistical differences with P value less than 0.05 where considered statistically significant.

### RESULTS

### MMPI Activities in Total Extracts from Seeds of Legume Species

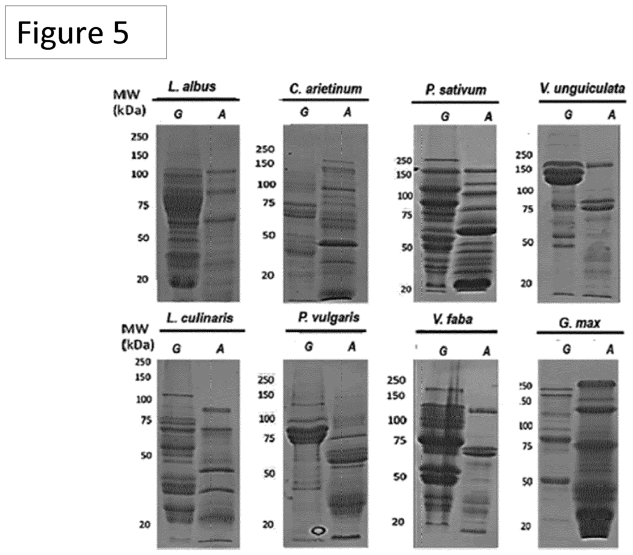

In a preliminary study recently published in Food Chemistry (Lima *et al.,* 2016) and before discovering deflamin, the Applicant screened a number of total seed extracts for the presence of inhibitory MMP activities. These seeds under study were: *Cicerarietinum* L. (chickpea), *Glycine max* L., (soybean), Lens culinaris M. (lentil), *Lupinus albus* L. (lupin), Phaseolus vulgaris L. (common bean), Pisum sativum L. (pea), Vicia faba L. (faba bean), and Vigna unguiculata L. (cowpea). The most promising ones, which we continued to study and will be addressed here were chickpea, lupin and soybean (Figures 5 and 6-11; Lima *et al.,* 2016). Figure 5 compares the albumin and globulin polypeptide profiles for each of the eight legume seeds initially analysed. Figure 5 shows representative images of the polypeptide distribution between albumins and globulins from eight species of legume seeds separated by SDS-PAGE. G - globulins, A - albumins. Protein extracts (40 µg per lane) were loaded onto 12.5% (w/v acrylamide) polyacrylamide gels under reducing conditions (Lima *et al.,* 2016).

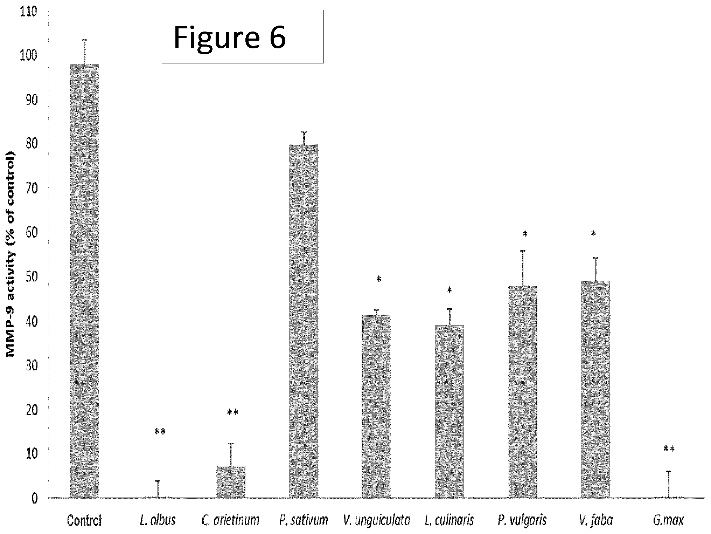

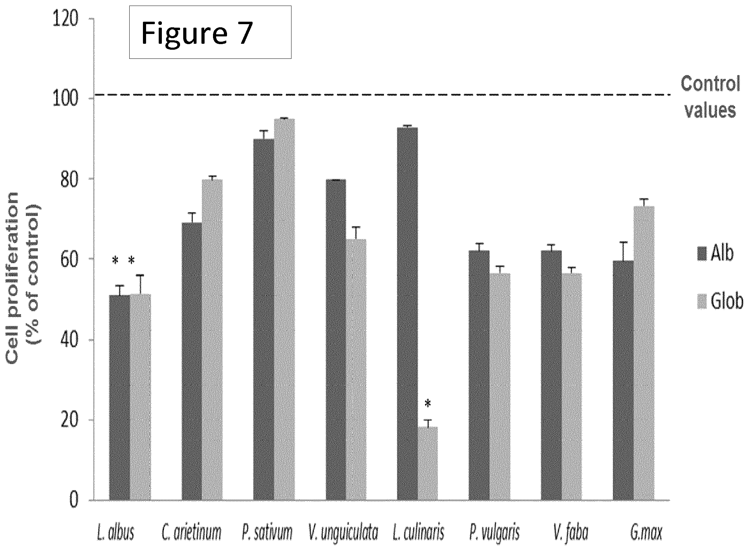

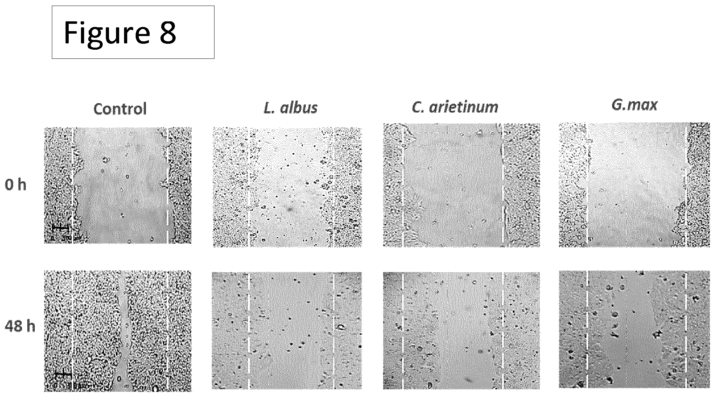

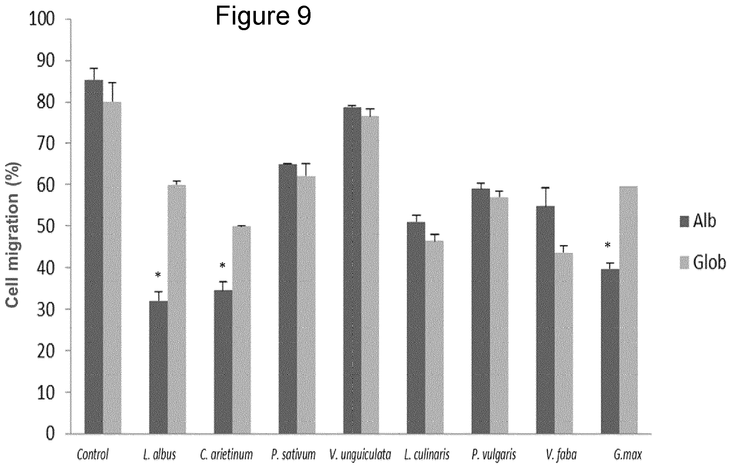

It should be noted that extractions of total seed proteins, total albumins and total globulins were performed under native conditions. Therefore, Figure 6-11 addresses the effects of a number of seed subfractions upon MMP activities present in the seeds from the eight species:
- Total soluble extracts (thus including low molecular mass metabolites such as flavonoids and other polyphenols, and peptides and proteins, presumably including lunasin) in Figure 6; Figure 6 concerns MMP-9 inhibitory activity. The effect of total soluble extracts from eight different legume seeds (*Lupinus albus, Cicer arietinum, Vicia faba, Phaseolus vulgaris, Pisum sativum, Vigna unguiculata, Lens culinaris* and *Glycine max*) on the proteolytic activity of MMP-9. Total soluble extracts were added to a reaction mixture containing MMP-9 and gelatinolytic activity was by the DQ fluorogenic assay.
- Albumins and globulins (a 24 h exposure) upon HT29 cell proliferation in Figure 7; Figure 7 concerns HT29 cell proliferation assay. HT29 cells were grown for 24 h in the presence of albumin or globulin fractions previously extracted from the eight seed species.
- Albumins and globulins (a 24 h exposure) upon HT29 cell migration in Figure 8 and 9; Figure 8 concerns the HT29 cell migration wound assay. Cells were grown until reaching 80% confluence and the monolayer was scratched with a pipette tip (day 0). Cell migration was determined after a 48 h exposure of HT29 cells to albumin or globulin fractions from the eight seed species. Examples of cell migration obtained for the highest inhibitory seed extracts: *L*. *albus, C. arietinum* and *G*. *max.*

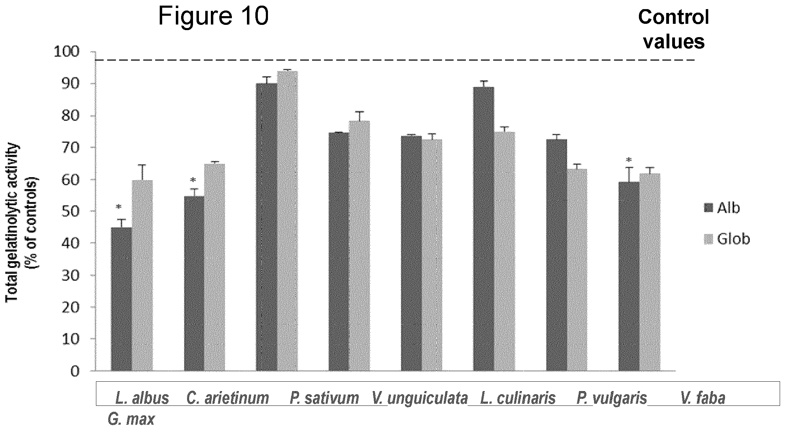

Figure 9 concerns the HT29 cell migration wound assay. Cells were grown until reaching 80% confluence and the monolayer was scratched with a pipette tip (day 0). Cell migration was determined after a 48 h exposure of HT29 cells to albumin or globulin fractions from the eight seed species. Relative migration rates (D),
- Albumins and globulins (a 48 h exposure) upon the proteolytic activity of gelatinases present in the HT29 extracellular media in Figure 10;

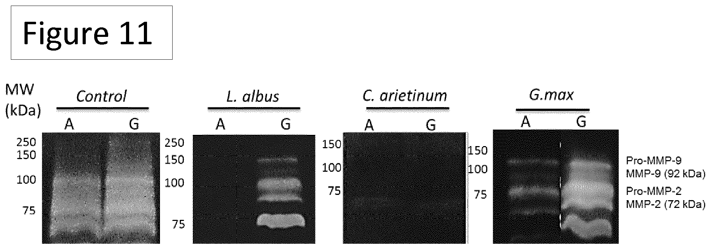

Figure 10 concerns proteolytic activity of gelatinases present in the HT29 extracellular media after a 48 h exposure to albumin or globulin fractions isolated from the eight seed species, as quantified by the DQ fluorogenic method.
- Zymographic profiles of MMP-9 and MMP-2 activities present in HT29 cell extracellular media after a 48 h exposure to albumins or globulins in Figure 11.

Figure 11 concerns zymographic profiles of the MMP-9 and MMP-2 activities present in HT29 extracellular media after a 48 h exposure of the cells to albumin or globulin protein fractions. Only the seed extracts producing the most marked inhibitions (i.e. *L. albus, C. arietinum* and *G. max*) are shown. Polyacrylamide gels (12.5% w/v acrylamide) were co-polymerized with 1% (w/v) gelatin. Relative activities of MMP-9 and MMP-2 bands were calculated as a % of controls. G, Glob - total globulin fraction containing 100 µg protein/mL; A, Alb - total albumin fraction, containing 100 µg protein/mL. All values represented are the mean of at least three replicate experiments ± SD, and are expressed as a percentage of the corresponding control. Vertical bars represent SD. *P < 0.05, **P < 0.001.

It is important to mention that none of these studies/extract involved cooked seeds, boiled extracts, extracts exposed to low pH values and/or extracts subjected to the digestive process. The results obtained clearly indicate the presence of MMP inhibitory activity or activities in the seeds from the eight seeds studied, with larger prominence, by decreasing order of magnitude, in lupin, soybean and chickpea. Such MMP inhibitory activities were attributed both to secondary metabolites, widely reported in the literature (e.g. genistein from soybean), and to a low molecular mass protein (i.e. lunasin).

Although sweet lupin seed consumption is increasing throughout the world, with a multitude of applications by the food industry, wild (i.e. bitter or alkaloid containing) seeds have been used as human food since ancient times and continue to do so as a snack in Mediterranean countries, provided they are boiled and the alkaloids washed away by immersion under running water. Interestingly, the very same alkaloids which make the wild-type seeds toxic seem to exert beneficial effects on diabetes mellitus. Nevertheless, in our everyday western life-style, other legume seeds are eaten more often and in larger quantities than lupins, such as chickpea and soybean. Embodiments of the invention focus on these last three species, with some additional results focusing also on some non-legume seeds.

### Cooking and the Richness of Seeds in Bioactive Metabolites

As mentioned in the Materials and Methods section above, dry seeds including those of cereals and legumes are not ingested as such for a couple of reasons. First, they are quite hard for us to chew. Second and most important, legume seeds, as many other seeds are well-known to contain anti-nutritional factors, such as inhibitors of digestive enzymes, lectins, high phytate concentrations, non-protein amino acids, etc. Therefore, they must be ingested after cooking to ensure denaturation of the proteinaceous anti-nutritional factors, as well as destruction or leaching of some non-protein constituents. For these reasons and because deflamin was found to resist boiling, a number of initial experiments was performed using cooked seeds.

In addition to proteins, plant seeds contain a wide array of bioactive secondary metabolites, most of which retain for the most part their biological activity after cooking.

The following examples on phytin, saponins, phenolic compounds and protein exemplify their concentration in both raw and cooked seeds from several legume species. It should be taken into account, as referred above, that the reduction (expressed per unit dry weight) of a specific compound induced by cooking, from phytin to proteins, is typically due to both molecular destruction and leaching into the surrounding water. Hence the popular knowledge that the water in which vegetables are cooked should be used to make soup.

In the next block of experiments, Pisum sativum and Vigna unguiculata were abandoned due to their poor results in what concerns MMP inhibition (see above). Therefore, embodiments relate to six species only, namely *Cicerarietinum, Glycine max,* Lens culinaris, *Lupinus albus, Phaseolus vulgaris* and *Vicia faba.*

### Phytin

Phytin (also referred to as phytate, PA, inositol hexaphosphate and IP6), for example, is considered both an anti-nutrient, since when present in excess inhibits digestive enzymes (e.g. trypsin, pepsin, α-amylase and β-glucosidase) and binds to certain minerals (most notably zinc and to a lesser extent calcium and chromium), thus interfering with their bioavailability (internet site 1).

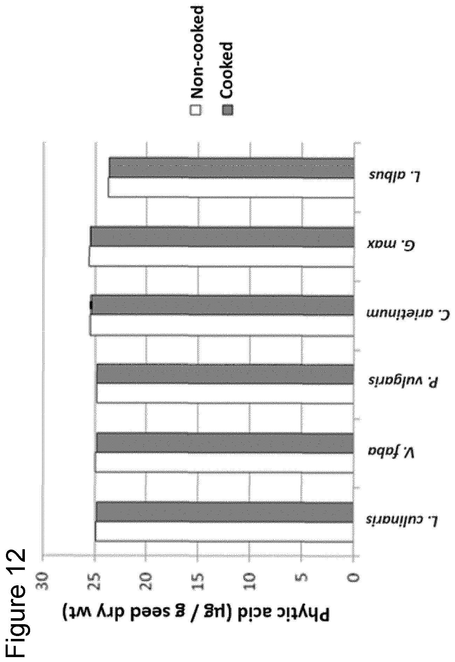

Figure 12 shows the phytin seed content of several species under study. Seed phytin content seems to be fairly constant among the species studied and is not affected by cooking in any significant way.

Figure 12 shows phytin concentration in the seeds of several legumes, as quantified by the method described by Gao *et al.* (2007). Vertical bars represent the mean ± SD of at least three biological replicates.

### Saponins

Saponins have considerable potential as pharmaceutical and/or nutraceutical agents in natural or synthetic form. They have been shown to exhibit anticarcinogenic, neuroprotective, anti-inflammatory and anti-oxidant activities, among others (Rao & Gurfinkel, 2000).

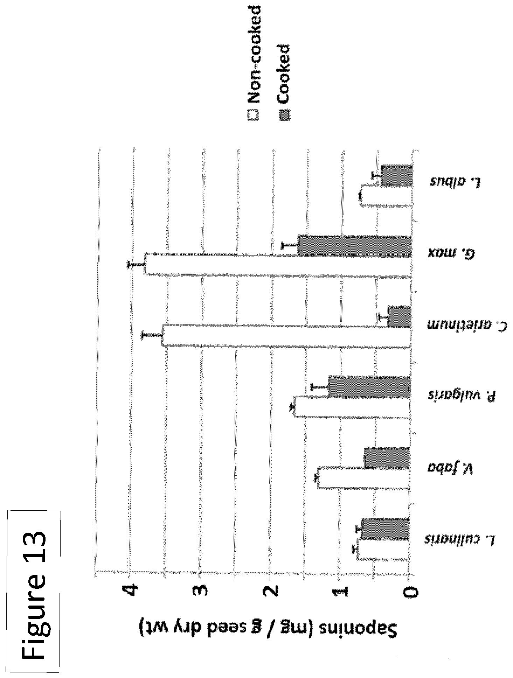

Figure 13 shows the saponin seed content of several species under study. Unlike those of phytin, saponin content varies significantly among the species analysed, with the highest values obtained for soybean and chickpea and the lowest ones for lupin. Boiling reduced consistently the amount of saponins present, with losses ranging from ca. 7% (lentil) to over 90% (chickpea). Figure 13 shows Saponin concentration in the seeds of several legumes, as quantified by the method described by Hiai *et al.* (1976). Vertical bars represent the mean ± SD of at least three biological replicates.

### Phenolic Compounds

Phenolic compounds occur universally in plants, and are known to exhibit high antioxidant ability and free radical scavenging capacity. They are therefore generally regarded as potential agents for preventing and treating many oxidative stress-related diseases, such as cardiovascular diseases, cancer, ageing, diabetes mellitus and neurodegenerative diseases, mostly due to their cardioprotection, anticancer, anti-inflammation and antimicrobial bioactivities (Li *et al.,* 2014). However, major concerns involve their bioavailability and potential toxicity, with the vast majority of studies not considering their resistance to cooking and to the digestive process. In addition, unlike bioactive proteins, their beneficial/harmful bioeffects are typically dose-dependent

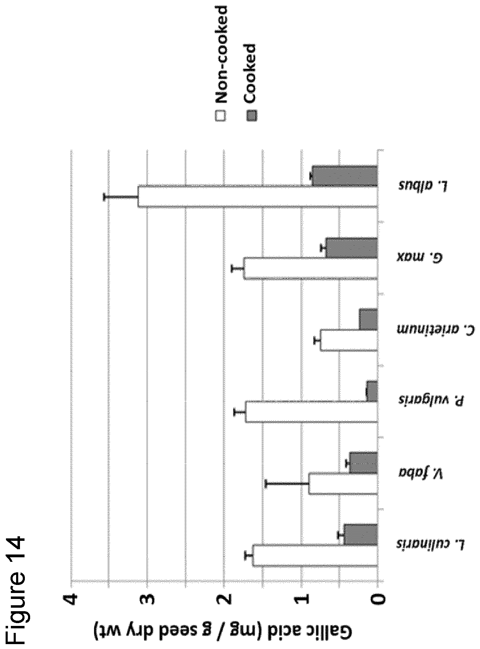

The seed concentration in phenolic compounds was greater in lupin, followed by soybean, common bean and lentil (Figure 14). Supposedly, the greater the seed level in polyphenols, the greater its anticancer bioactivity. Cooking reduced dramatically (between 60 and 90% depending on the species) the amount of seed phenolics. Figure 14 show concentration in phenolic compounds in the seeds from several legumes, as quantified by the Folin-Ciocalteau method (Attard, 2013). Vertical bars represent the mean ± SD of at least three biological replicates.

### Total Soluble Protein

Proteins compose an amazing class of biomolecules fulfilling an enormous variety of bioactivities with no parallel in any other class of molecules. Selecting a previously unknown protein and determining its biological function is not only difficult but also one of the most challenging and interesting tasks of biological and chemical researchers. In addition to executing the biological role for which they evolved, proteins many also be used for the benefice of mankind due to a wide range of beneficial activitites. Thus, for example, the Food and Drug Administration (FDA) authorized the use, on food labels and in food labeling, of health claims on the association between soy protein and reduced risk of coronary heart disease (FDA, 1999).

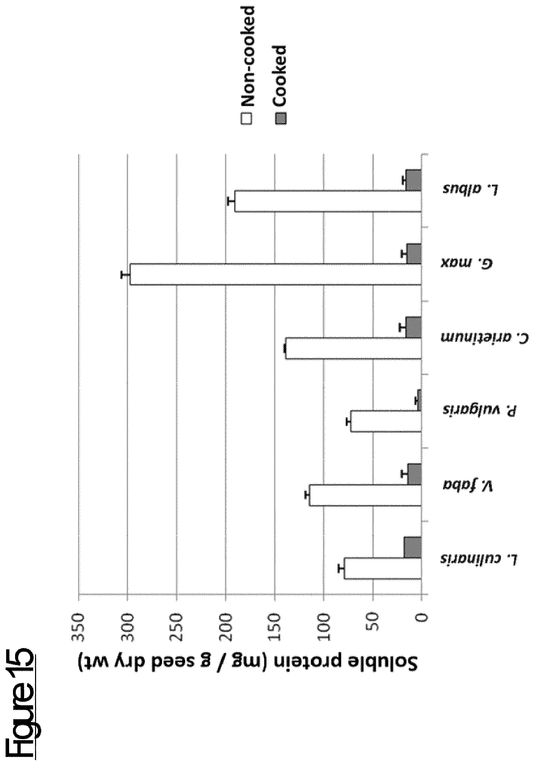

Soybean seeds contain, as expected, the highest amount of total soluble protein (297.4 mg/g dry wt), followed, by decreasing protein concentration, by lupin (190.4 mg/g dry wt), chickpea (138.2 mg/g dry wt), broad bean (114.5 mg/g dry wt), lentil (79.0 mg/g dry wt) and common bean (720.0 mg/g dry wt); Figure 15). Upon cooking, these values were reduced in all cases to values below 20 mg/g dry wt, with the highest protein concentrations obtained for lentil, lupin, chickpea and soybean. It is important to note that Figure 15 refers to soluble protein and that cooked seeds certainly contain most of their protein in a denatured, insoluble form.

Figure 15 shows total soluble protein concentration in seeds from several legumes, as quantified by the Bradford method (Nobel, 2000). Vertical bars represent the mean ± SD of at least three biological replicates.

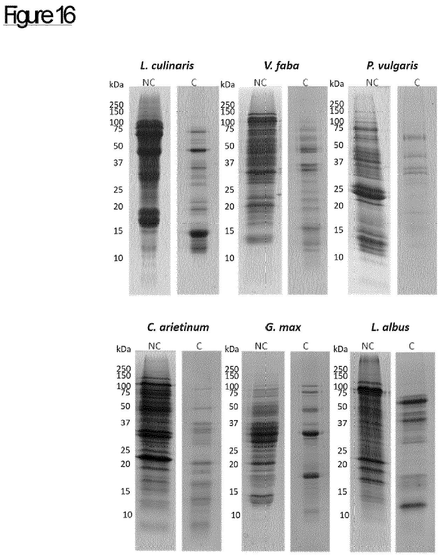

The data presented in Figure 16 allows a comparison for each species between the polypeptide profiles of the soluble proteins from intact seeds with those from the corresponding cooked seeds. As expected, the profiles are completely different with the polypeptides which survived cooking in each case present in lanes C. Note that lanes in Figure 16 do not contain the same amount of protein. Rather, they correspond to a fixed amount of seed dry weight. Therefore, direct quantitative and qualitative comparisons can be made for each species.

Figure 16 shows representative polypeptide profiles obtained by R-SDS-PAGE (17.5% w/v acrylamide supplemented com 10% v/v glycerol; reducing conditions) of soluble protein fractions extracted from raw (NC) and cooked (C) seeds. To allow both quantitative and qualitative comparative analyses, fractions NC and C were resuspended and loaded in the gel in equal volumes.

Embodiments of the invention concern primarily i.e. *Cicerarietinum*, *Glycine max* and *Lupinus albus.*

### Cell Migration

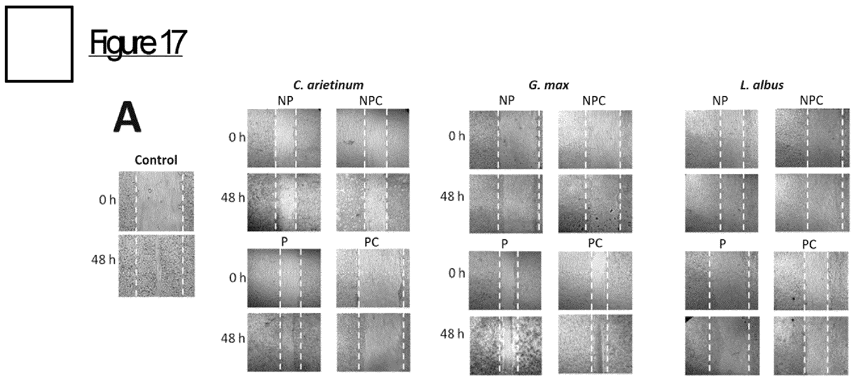

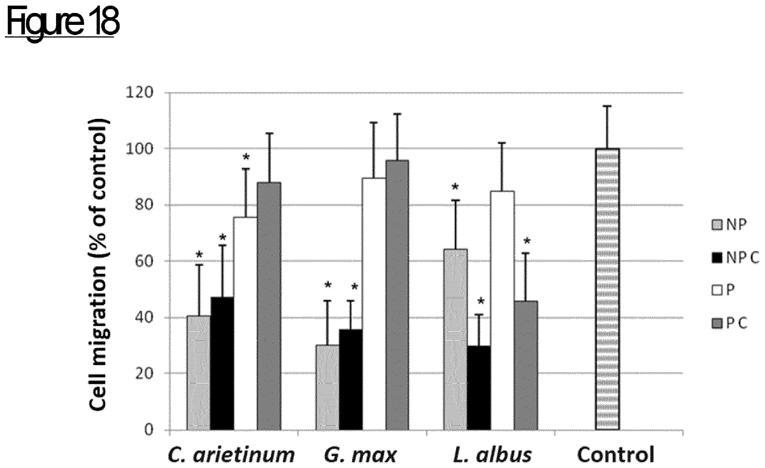

The data presented in Figure 17 and Figure 18 shows that the soluble extracts prepared from C. *arietinum*, G. max and *L. albus* inhibit the migration of HT29 cells. However, considerable differences were found among extracts, species and the condition (i.e. cooking or not) of the seeds. Thus, all non-protein extracts examined exhibited a marked inhibition upon cell migration. Among the soluble protein extracts prepared from raw seeds, the cell migrating inhibitory activity was almost negligible for soybean, intermediate for lupin and slightly higher for chickpea. However, it was amongst the soluble protein extracts prepared from cooked seeds that an astonishing and surprising result was achieved: unlike chickpea and soybean, cooking lupin seeds has a dramatic effect in enhancing the cell migrating inhibitory activity of the resulting soluble protein extract. Strangely enough and once again unlike chickpea and soybean, exactly the same effect is achieved in what concerns the non-protein extract prepared from cooked lupin seeds. These data suggest that lupin seeds contain both protein and low molecular mass non-protein compounds which exhibit a HT29 cell migrating activity whose effect is markedly enhanced by previous cooking the seeds, something which make them particularly suitable in what human nutrition is concerned.

This study may be the first to consider the effect of cooking (a topic of utmost importance with respect to human health and nutrition) legume seeds on the migrating potential of cancer cells.

Several hypotheses may be advanced to try to explain the increment in cell migrating inhibitory activity of both non-protein and protein soluble extracts prepared from cooked lupin seeds versus raw seeds.
1. A concentration effect. We know that most lupin seed proteins are denatured and leach out into the surrounding water during cooking. It is also known that many (if not most) low molecular mass metabolites are either destroyed or leach into the boiling water. In the the wound healing assays depicted in Figure 17 and 18, 100 µg soluble protein/mL or 10 mg soluble non-protein biomolecules/mL were used. When compared to extracts prepared from raw seeds, this means a large concentration effect for those metabolites and proteins that survived the cooking process.
2. A heat-induced and irreversible dissociation effect in the case of oligomeric proteins in case the individual subunits or a remaining part of the original oligomer is soluble and exhibits enhanced bioactivity.
3. It is also possible for deflamin to occur both in the soluble protein fraction and in the soluble low molecular mass non-protein fraction.

Nevertheless, the same effect was not observed for either chickpea or soybean, something which makes lupin somewhat 'special'.

Figure 17 shows representative images of HT29 cell migration as assessed by the wound healing assay (A). Cells were grown until reaching 80% confluence and the monolayer was scratched with a pipette tip (day 0). Cell migration was determined after a 48 h exposure of HT29 cells to buffer (control), to non-protein extracts (10 mg/mL) or to protein extracts (100 µg/mL) from the three seed species under study. Figure 18 shows relative migration rates are plotted in (B). NP: soluble non-protein extract prepared from raw seeds; NPC: soluble non-protein extract prepared from cooked seeds; P: soluble protein extract prepared from raw seeds; PC: soluble protein extract prepared from cooked seeds. Vertical bars represent the mean ± SD of at least three biological replicates. *P < 0.05.

Unlike the soluble low molecular mass non-protein compounds, the soluble soybean protein fraction prepared from raw seeds did not show a significant inhibitory activity on the migration of HT29 cells. This may come as a surprise due to the well-known presence of Bowman-Birk inhibitors (BBI) in G. max seeds. One possible explanation may lay on the fact that Fereidunian and co-workers (Fereidunian *et al.*, 2014) purified 12 mg BBI / g soybean seed, a value corresponding to about 9% of the soya total soluble protein. A simple extrapolation tells us that ca. 9 µg of soybean BBI were included in the soluble protein extract prepared from raw soybean seeds used in the assay (Figures 17 and 18), an amount far below that used by Fereidunian and colleagues.

### Cell Viability and Proliferation

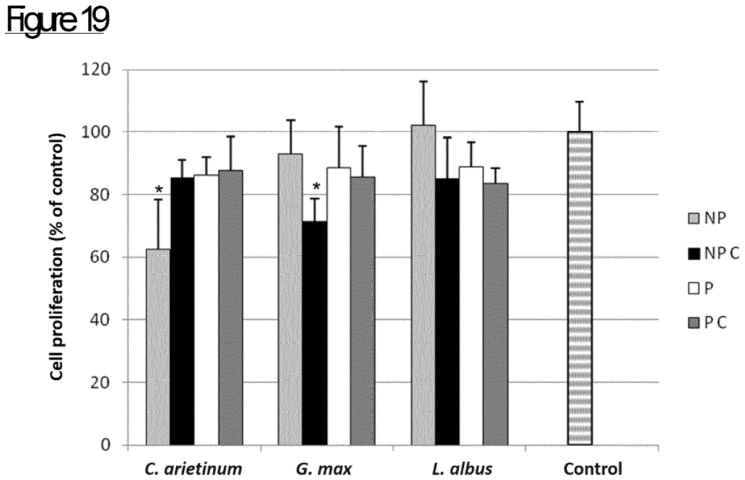

The results presented in Figure 19 show the effect of the different extracts, species and the condition (i.e. cooking or not) of the seeds on HT29 cell proliferation. With the possible exception of the soluble non-protein extract prepared from chickpea seeds, it seems reasonable to conclude that all extracts present low toxicity to HT29 cells, since these cells remain viable after a 24 h exposure. In addition, they do not seem to inhibit cell proliferation. Giron and co-workers (Giron-Calle *et al.*, 2004) detected a potent inhibition of Caco-2 cell proliferation in the presence of the acetone soluble metabolites extracted from chickpea seeds. Figure 19 show a cell proliferation assay. HT29 cells were grown for 24 h in the presence of buffer (control), non-protein extracts (10 mg/mL) or protein extracts (100 µg/mL) from the three seed species under study. NP: soluble non-protein extract prepared from raw seeds; NPC: soluble non-protein extract prepared from cooked seeds; P: soluble protein extract prepared from raw seeds; PC: soluble protein extract prepared from cooked seeds. Vertical bars represent the mean ± SD of at least three biological replicates. *P < 0.05. In contrast to the results shown in Figure 19, data published on legume seed proteins point to an inhibitory role at the level of cell proliferation. Fereidunian and co-workers (Fereidunian *et al.*, 2014) observed over 50% reduction in HT29 cell proliferation in the presence of 200 µg BBI / mL. This value increased to over 80% for a BBI concentration of 400 µg / mL. BBI from chickpea has been reported to inhibit breast cancer cell proliferation in vitro, whereas BBIs from common bean, soybean and chickpea reduced prostate cancer cell proliferation also in vitro (Magee *et al.*, 2012).

Bawadi and colleagues (Bawadi *et al.*, 2005) reported the inhibition of prostatic cancer cell proliferation and cell migration, as well as of the secretion of MMP-9 and MMP-2 by water-soluble black bean condensed tannins. Park *et al.* (2013) reported that fisetin obtained from a methanolic extract of Dalbergia odorifera inhibits MMPs, proliferation and invasiveness of fibrosarcoma HT-1080 cells, and Aparicio-Fernandez *et al.* (2006) using human adenocarcinoma HeLa cells and human premalignantkeratinocytes (HaCaT) found that 100% methanol crude extract from the seed coats of black Jamapa beans exhibits an inhibitory effect on the proliferation of HeLa cancer cells but is less aggressive on HaCaT premalignant cells. Zhou *et al.* (1999) showed that soybean isoflavones (genistein or daidzein) or soybean phytochemical concentrate inhibit the growth of prostate cancer cells LNCaP, DU 145 and PC-3 *in vitro.*

### Gelatinolytic Activity in HT29 Extracellular Media

Under normal conditions (e.g. in the absence of inhibitors), cancer cells secrete MMP-9 and MMP-2 into the external miliue to degrade the matrix proteins, thus allowing cells to migrate. Any condition which inhibits the gelatinases will inhibit metastases formation. Discovering natural inhibitors of MMPs is therefore of potential interest.

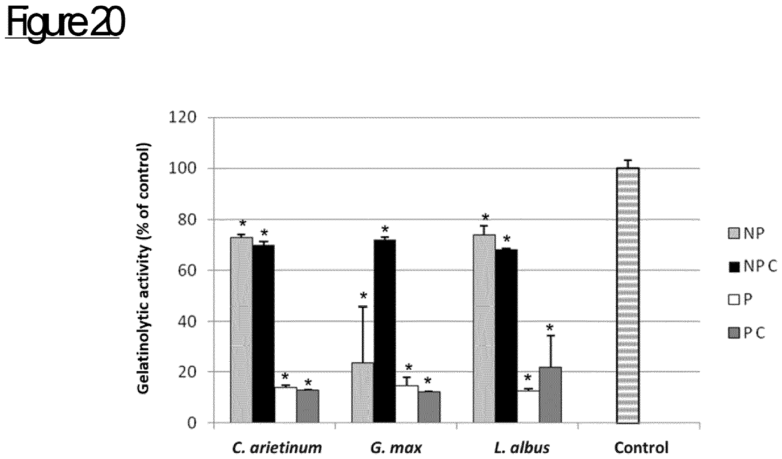

As demonstrated in the previous section, some soluble extracts containing proteins and low molecular mass metabolites prepared from lupin, chickpea and soybean seeds exhibit a strong inhibitory effect on HT29 cell migration but not on HT29 cell proliferation. This difference may be explained by their mechanism of action. One of the major factors associated to cancer cell invasiveness is the activity of MMP-9 and MMP-2 enzymes. Therefore, these activities were determined in the HT29 extracellular media after a 48 h exposure to several extracts (Figure 20), as quantified by the DQ fluorogenic method.

Figure 20 show proteolytic activity of total MMP activity in HT29 extracellular media as quantified by the DQ fluorogenic method. HT29 cells were grown for 48 h in the presence of buffer (control), non-protein extracts (10 mg/mL) or protein extracts (100 µg/mL) from the three seed species under study. NP: soluble non-protein extract prepared from raw seeds; NPC: soluble non-protein extract prepared from cooked seeds; P: soluble protein extract prepared from raw seeds; PC: soluble protein extract prepared from cooked seeds. Vertical bars represent the mean ± SD of at least three biological replicates. *P < 0.05.

The results presented in Figure 20 show that all extracts tested exhibited some degree of inhibition upon the level of gelatinase activities present on HT29 extracellular media. However, when compared to the control, an extremely strong inhibition (between 80 and 90%) was found for all soluble protein extracts (either prepared from raw or cooked seeds) and for the soluble non-protein extract prepared from soybean raw seeds.

In general, it is interesting to note that HT29 cell migration was mostly affected by soluble non-protein metabolites (Figures 17 and 18), whereas total MMP inhibitory capacity in the HT29 extracellular media was essentially conditioned by the soluble proteins (Figure 20). These results suggest thatlegume proteins are more efficient at inhibiting MMP activity, while soluble non-protein metabolites seem to inhibit cell migration using other(s) mechanism(s) of action.

The assays illustrated in Figure 20 measure the total gelatinolytic activity, i.e. those of MMP-9 (92 kDa) and MMP-2 (72 kDa) combined. To separate these two activities, a different approach must be used, such as zymography or reverse zymography. The results of such experiments (data not shown) indicate that MMP-9 is the primary target Indeed, zimography of the extracellular media collected following a 48 h incubation of HT29 cells with each one of the four extracts used in Figure 20 showed an inhibitory activity essentially targeting MMP-9 and pro-MMP-9 (83 kDa).

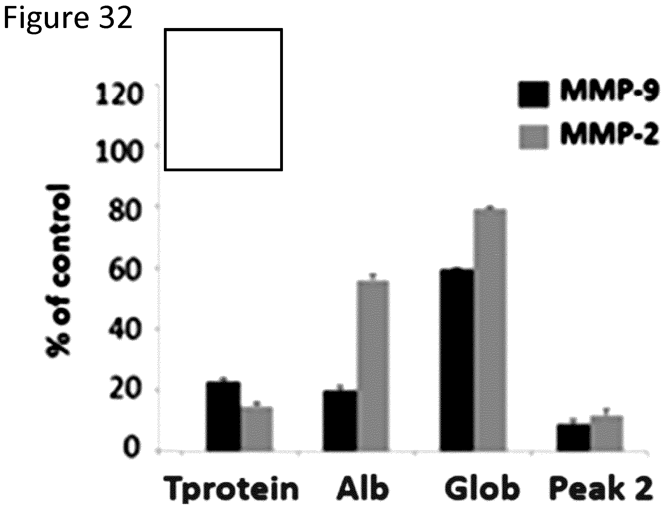

This block of experiments seems to indicate that the soluble proteins from lupin, chickpea and soybean inhibit HT29 cell migration essentially via MMP-9 inhibition. However, it should be noted that different reports on the gelatinases inhibitory activities produce contradictory results even inside this very same study. Thus, the experiments described immediately above indicate that both the protein fraction and the metabolite fraction from lupin, chickpea and soybean inhibit considerably MMP-9 but not MMP-2. In contrast, the experiment illustrated in Figure 32 shows that the total protein fraction from lupin strongly inhibits both gelatinases. Identical results are presented in the available literature. Soybean BBI inhibits both MMP-9 and MMP-2 at concentrations of 200 e 400 µg/mL (concentrations far higher than those utilized in the present study; Fereidunian *et al.*, 2014). Bawadi *et al.* (2005) reported that a 24 h incubation of Caco-2 colon, MCF-7 and Hs578T breast, and DU 145 prostatic cancer cells with water-soluble black bean condensed tannins resulted in a sharp decrease in the levels of active MMP-2 and MMP-9 secreted into the culture medium for tannin concentrations above 12 µM. At 15 µM, fisetin inhibits 50% MMP-9 and other MMPs, but apparently not MMP-2 (Park *et al.*, 2013). Phytin at 2.5 mM inhibited the expression of MMP-9, MMP-2 and other MMPs in colon cancer Caco-2 cells stimulated with phorbol-12-myristate 13-acetate (PMA); Kapral *et al.*, 2012). Treatment of fibrosarcoma HT-1080 cells with soybean saponins inhibited the mRNA expression of and reduced the amounts of secreted MMP-2 and MMP-9 (Kang *et al.*, 2008).

One striking observation is apparent: with the exception of protease inhibitors (e.g. trypsin inhibitor and BBI), few experiments have been conducted to assess the potential inhibitory effect of proteins on MMPs activities.

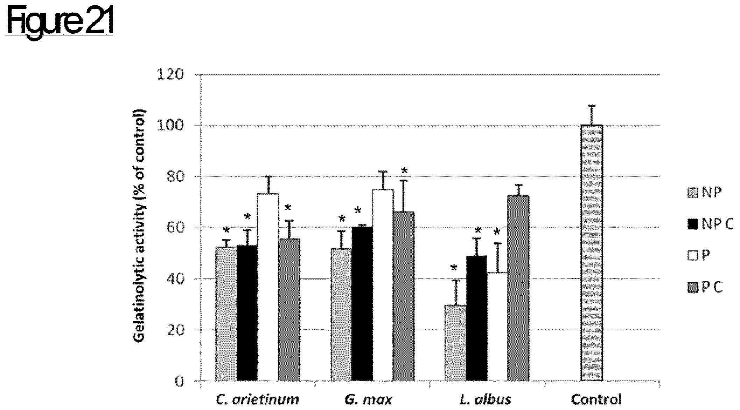

Figure 21 illustrates one other experiment in which the inhibitory activity of chickpea, soybean and lupin seed extracts on commercial MMP-9 was assessed using the same seed mass in all cases, thus allowing a direct comparison and mimicking the ingestion of these seeds. Figure 21 shows the inhibitory effect of soluble seed extracts on the proteolytic activity of MMP-9. Extracts were added to a reaction mixture containing commercial MMP-9 and gelatinolytic activity was determined by the DQ fluorogenic assay. The volume of extract (100 µL) added to each reaction mixture corresponded to the same seed mass. NP: soluble non-protein extract prepared from raw seeds; NPC: soluble non-protein extract prepared from cooked seeds; P: soluble protein extract prepared from raw seeds; PC: soluble protein extract prepared from cooked seeds. Vertical bars represent the mean ± SD of at least three biological replicates. *P < 0.05.

Overall, this experiment shows that both the protein fraction and the non-protein fraction inhibit MMP-9 activity. This inhibitory action seems to be higher for the non-protein fraction and stronger in lupins than in chickpea or soybean. As expected, the effect of cooking the seeds decreased, but only marginally, the inhibitory power of all fractions. These results explain the effect observed in Figures 17 and 18, where the higher HT29 cell migration (as assessed by the wound healing assay) obtained for the non-protein and protein fractions from cooked lupin seeds when compared to raw seeds can be attributed to a concentration effect of the cooking-resistant, active ingredients.

Isolation of Potentially Novel MMP-9 and MMP-2 Inhibitors from *Lupinus albus* Seeds.

### Discovery of Deflamin

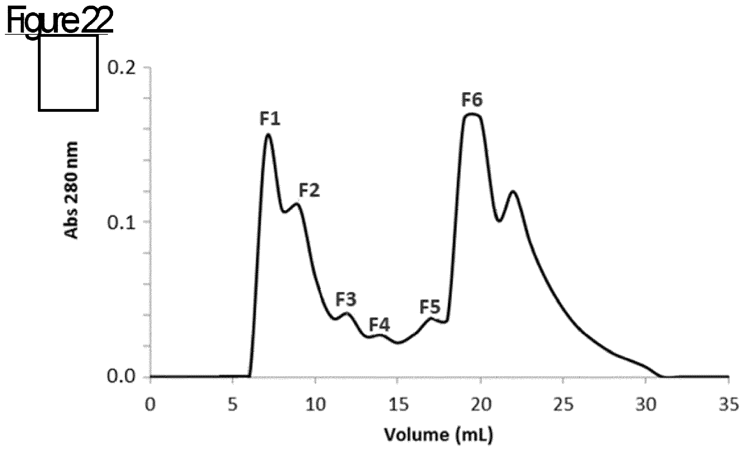

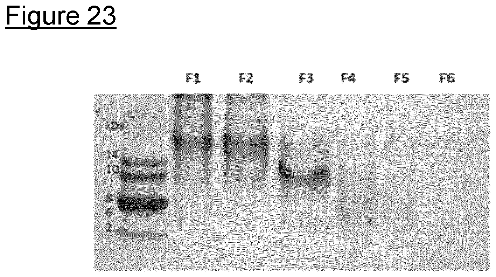

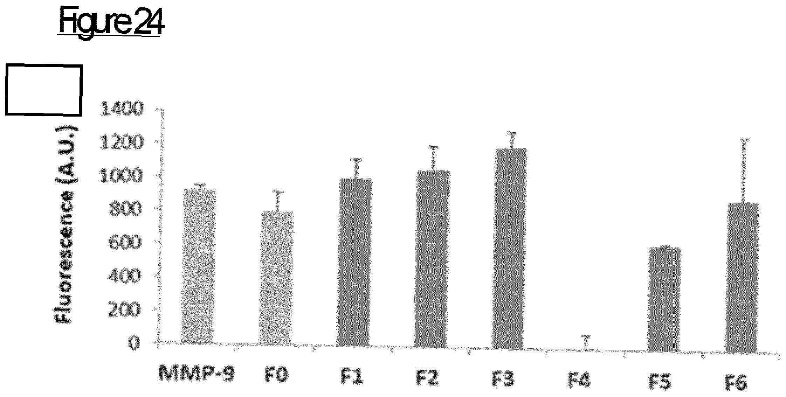

In order to isolate the protein fractions, found to be responsible for MMP-9 inhibition, the proteins of *L. albus* were separated according to their native size, and tested their inhibitory activity against MMP-9. Figures 22 to 24 show the size exclusion chromatography (SEC) obtained for the *L. albus* total protein extraction, the corresponding electrophoretic protein profiles of the collected fractions F1 to F6, and the MMP-9 inhibitory activity of each fraction. Peptides, polypeptides and proteins were extracted from *L. albus* seeds as described in the Material and Methods section. The desalted extract, containing the total protein extract, was fractionated using the Äkta system by size exclusion chromatography in a Superdex 75 column. Protein peaks were collected as fractions F1 to F6 (as shown in Figure 22). Proteins/polypeptides were in certain embodiments separated according to their molecular size using a urea and dithiothreitol (DTT) containing buffer, which allowed the separation of different low molecular mass fractions. Other buffers were tested which could not allow an effective separation of *L. albus* proteins/polypeptides in this size range, which was concordant with previous results obtained in our lab. Each fraction was then tested for MMP-9 inhibitory activity, using the DQ gelatin assay. Figure 24 shows the effect in MMP-9 activity of each fraction (F1 - F6).

Figure 24 clearly shows that fraction 4 cause 100% inhibiton of MMPs catalytic activities. This fraction was subsequently confirmed to contain deflamin.

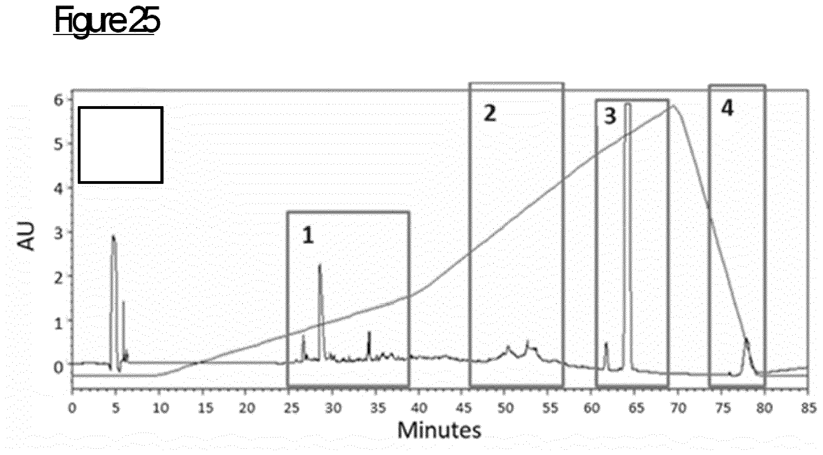

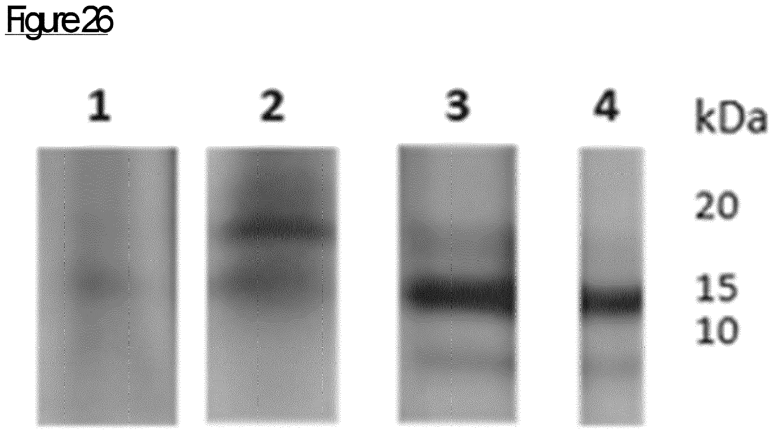

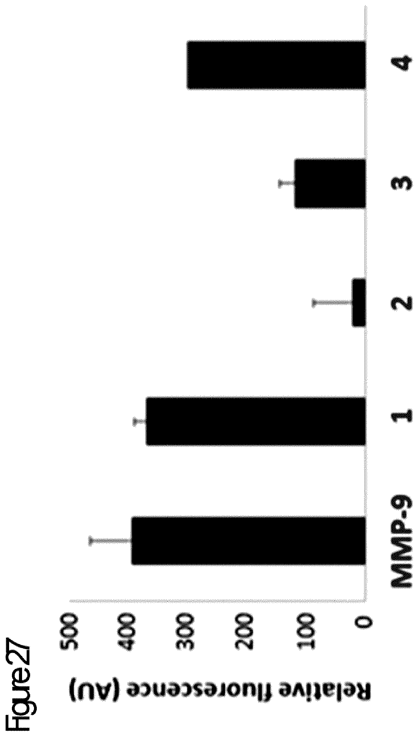

Figures 22-24 show the peptides, polypeptides and proteins were extracted from *L. albus* seeds as described in the Material and Methods section. The desalted extract, containing the total protein extract, was fractionated using the Äkta system by size exclusion chromatography in a Superdex 75. Protein peaks were collected as fractions F1 to F6 (22) and separated by Tricine SDS-PAGE under reducing conditions (23). (24) Each fraction was tested for MMP-9 inhibitory activity, using the DQ gelatin assay. Results are expressed in arbitrary units of fluorescence and represent an average of three replicates ± SD. As observed in Figures 22 to 24, only fraction 4 presented a very high level of MMP-9 inhibition. This sample was further fractionated by reverse phase (RP)-HPLC, in order to analyze the specific peptides responsible for this activity. Figures 25 to 27 shows the HPLC profiles obtained for fraction 4. Essentially four peaks were obtained, each of which was analysed by SDS-PAGE and its MMP-9 inhibitory activity assessed by the DQ gelatin assay. Peak 2 exhibited the highest MMP-9 inhibitory activity and, to a lesser extent, also peak 3 (Figure 27). It is interesting to note that at this stage, we conducted peak 2 to be composed of at least two, probably more polypeptides (box 2 in Figure 25).

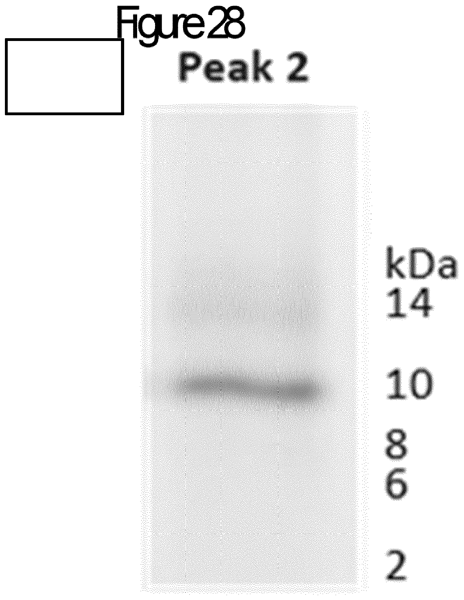

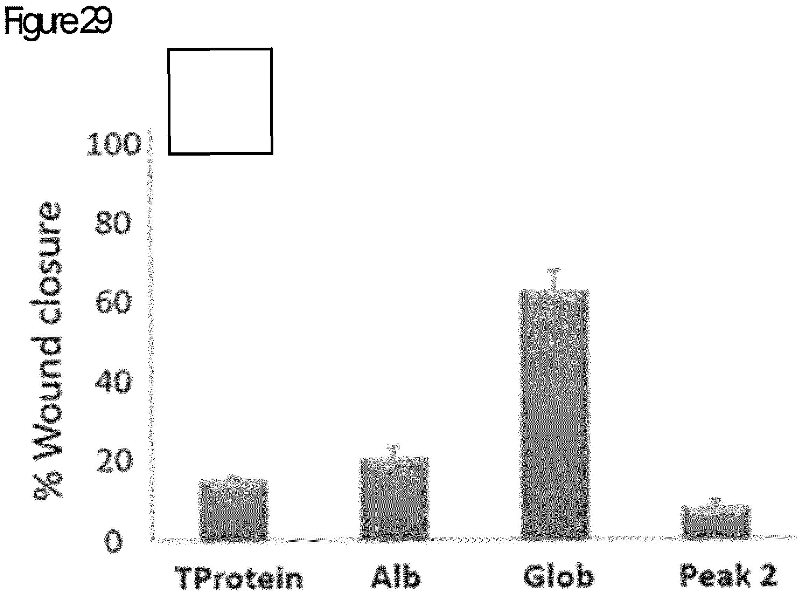

Note that all protein fractions utilized in Figures 28 to 32 were previously subjected to boiling and low pH values, in order to determine its resistance to denaturation whilst at the same time simulating the digestive process, as well as part of the isolation procedure, and were subsequently used for HT29 cell wound closure and gelatinolytic assays, as well as zymographic analysis. This clearly indicates the resistance of deflamin to boiling and to low pH values.

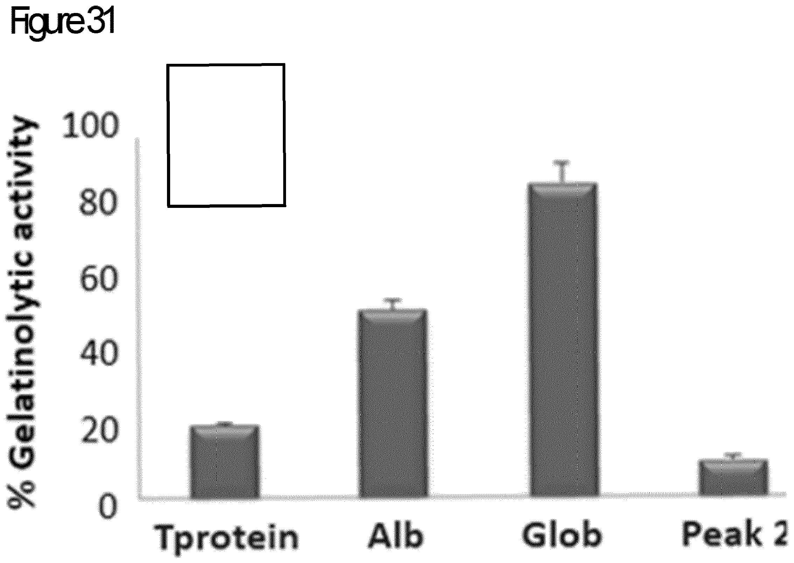

Figure 31 shows the gelatinolytic activities of HT29 cell media in the presence of the same protein fractions, whereas Figure 32 assesses the activities of both MMP-9 and MMP-2 in the zymographic separations. Results evidence that the polypeptides comprising peak 2 collected from the HPLC chromatogram depicted in Figure 25 are indeed strong MMP inhibitors, particularly after heat treatments, and that they can inhibit both MMP-9 and MMP-2 catalytic activities.

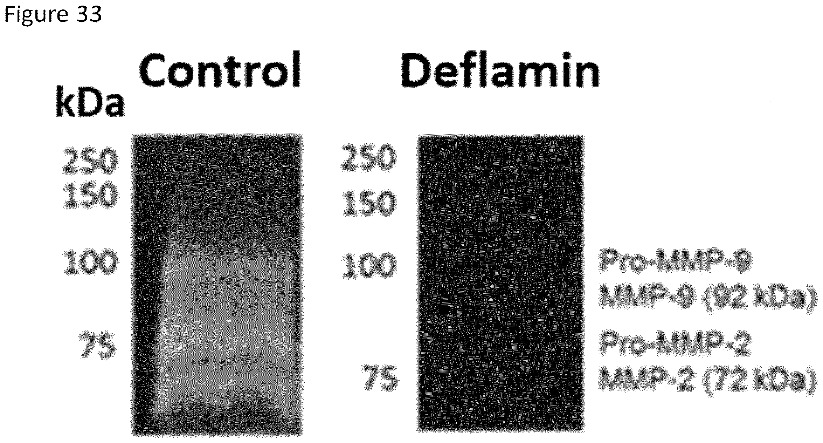

Analysis of HT29 extracellular media after a 48 h incubation of the cells with peak 2 protein (100 µg protein/mL) revealed that 'deflamin' inhibits both MMP-9 and MMP-2 activity, as shown by the DQ-gelatin fluorogenic method (Figure 32) and by zymography (Figure 33). The black (blue when viewed in colour) background visible in Figure 33 is due to heavy staining of gelatin by Coomassie Brilliant Blue, which co-polymerized with acrylamide to form the gel matrix. The presence of banded active MMP enzymes or pro-MMP proenzymes degrades locally the matrix-embedded gelatin, resulting in a white band. The presence of an inhibitor (e.g. 'deflamin') blocks the proteolytic action of MMPs, allowing staining of the unaltered gelatin. Therefore, the absence of white bands in the lane 'De flam in' from Figure 33 reveals that 'deflamin' at 100 µg/mL fully inhibited the activity of all MMP forms present in the HT29 extracellular media and indicates that this inhibition did not revert during the zimographic assay.

It should be taken into account that the fraction loaded in the gels depicted in Figure 33 is not exactly deflamin - Hence the notation 'deflamin'. Indeed in certain embodiments, deflamin is a proteinaceous fraction that is obtained from seeds following the isolation methodology detailed in Figure 4 - in certain embodiments this is part of its definition. The bioactive fraction utilized in Figure 33 was purified using a different procedure.

Figure 33 shows representative images of the zymographic profiles of MMP-9 and MMP-2 enzyme activity in HT29 extracellular media after a 48 h exposure of the cells to 'deflamin' (100 µg protein/mL). Control: HT29 cells incubated for 48 h in the absence of 'deflamin'.

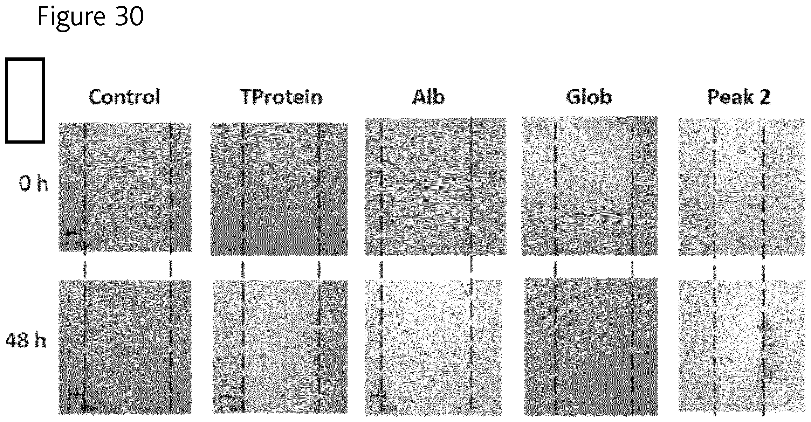

Although capable of inhibiting cellular invasion (Figure 30), the same concentration of 'deflamin' did not affect cell multiplication, suggesting absence of cytotoxicity.

### Purification and Characterization of Deflamin from Lupinus albus -I

An embodiment of the inventive methodology was developed (see Figure 4) to extract and purify deflamin from seeds that is suitable to undergo up-scaling, allowing its mass production in industrial facilities. The procedure is described in a detailed manner in the Methods section.

It should be borne in mind that the HPLC step referred in the Methods section is utilized to fractionate deflamin constituent polypeptides and not to isolate deflamin.

Sequential extractions allow the isolation of *L. albus* deflamin which presents higher MMPI activity than total extracts.

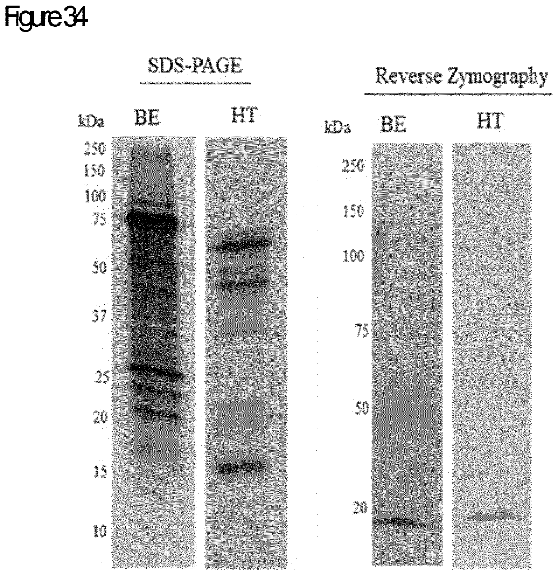

Figure 34 shows a representative image of the polypeptide distribution between *Lupinus albus* seeds simply extracted with buffer (buffer extraction; BE) or after heat treatment (HT), and visualized by SDS-PAGE (left) and the reverse gelatin zymography (right).

Protein extracts (50 µg/mL) were loaded onto 17.5% (w/v acrylamide) polyacrylamide gels, copolymerized with gelatin and MMP-9 in the case of reverse zymography.

The polypeptide band visible in both lanes BE and HT with a molecular mass lower than 20 kDa corresponds to deflamin. As shown in Figure 34, deflamin maintains its biological activity after the heat treatment.

Preliminary results suggested that the MMPI protein fraction from *L. albus* (i.e. deflamin) was highly soluble in water and exhibited resistance to heat denaturation. Therefore, a method of isolation with sequential precipitations (appropriate for the future scaling-up to an industrial scale) was established.

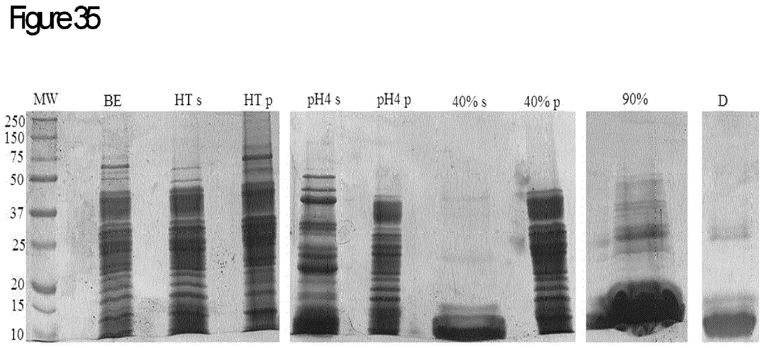

Representative images of the electrophoretic profiles obtained following several sequential extractions to isolate the MMPI active protein fraction are shown in Figure 35.

Figure 35 shows representative images of the polypeptide profiles obtained after each step of the purification method as specified on the top of the gels. The protein samples (25 µg) were loaded onto 17.5% (w/v acrylamide) polyacrylamide gels. MW- Molecular mass markers; BE - Buffer Extration; HT s - Heat Treatment, supernatant; HT p - Heat Treatment, pellet; pH4 s - Acid precipitation, supernatant; pH4 p - acid precipitation, pellet; 40% s - 40% v/v Ethanol containing 0.4 M NaCl, supernatant; 40% p - 40% v/v Ethanol containing 0.4 M NaCl, pellet; 90% - 90% v/v Ethanol overnight at -20 ºC, pellet; and D - Deflamin.

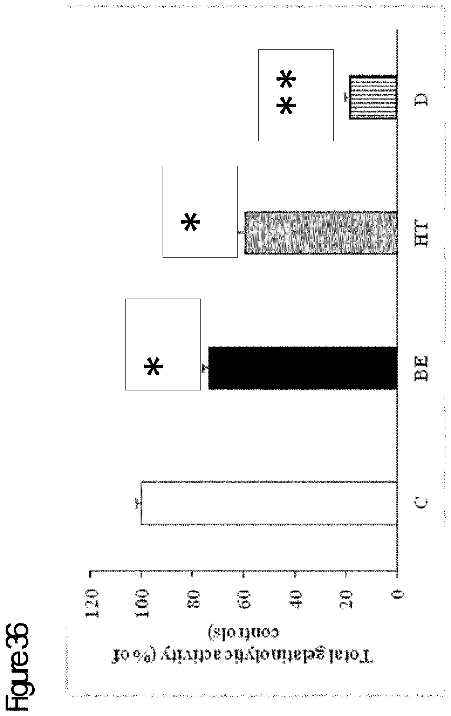

Analysis of the polypeptide profiles following each step of the isolation method depicted in Figure 4 revealed the gradual purification of a protein fraction with a molecular mass below 20 kDa, which was termed deflamin. The main protein fractions obtained, i.e. total protein / buffer extract (BE), heat-treated extract (HT) and isolated deflamin fraction were compared for their MMPI activities using the DQ gelatin assay. The results obtained are shown in Figure 36.

At the protein concentration tested (50 µg/mL), Figure 36 shows that all samples were able to significantly inhibit MMP-9 proteolytic activity. However, significant differences (P<0.05) were observed among the samples analysed, with the highest inhibition (P<0.05) detected for deflamin, which induced a reduction greater than 80% of MMP-9 activity.

In other words, in Figure 36, the buffer extraction (BE), heat treatment (HT) and deflamin (D) protein fractions were obtained from *L. albus* seeds and used to assess their inhibitory activity upon the proteolytic activity of MMP-9 on DQ-gelatin. The negative control (C) does not inhibit MMP-9, resulting in 100% proteolytic activity for this protease. Protein samples were added at a concentration of 50 µg/ml and gelatinolytic activity was measured by the DQ fluorogenic assay. MMP activities are expressed as relative fluorescence as a % of controls, and represent the averages of at least three replicate experiments (n = 3) ± SD. _{*} P < 0.05, _{**} P < 0.001.

In summary, as deflamin is gradually purified, its inhibitory effect as an MMPI increases.

### L. albus Deflamin is More Effective in Inhibiting Colon Cancer Cell Invasion and Proliferation

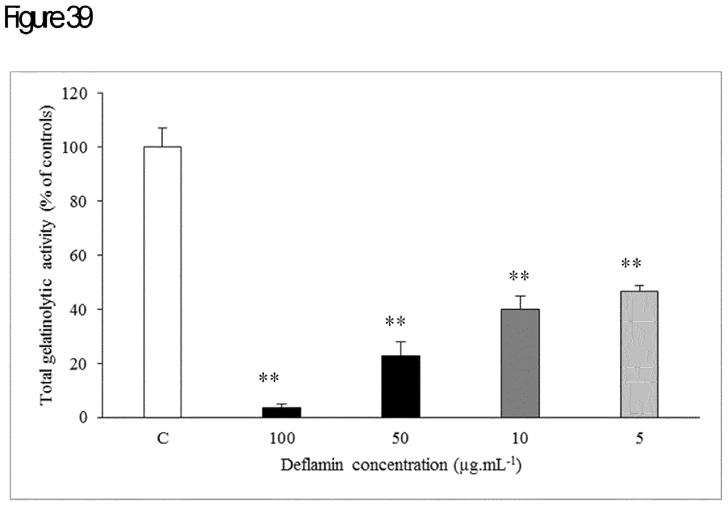

Note: the word 'more' in this title was used with a double sense:
1 - As the isolation methodology proceeds from the initial total protein extract to purified deflamin, its biological activity gradual increases, reaching a maximum with isolated deflamin. As comparative tests performed after each purification step use identical protein amounts of proteins from each sample, as the degree of deflamin purification increases, the amount of deflamin relative to total protein in each fraction also increases, justifying the increment in deflamin bioactivity when one moves from less pure to purer deflamin fractions.
2 - Deflamin is a poor inhibitor of cell multiplication (meaning a low cytotoxicity; see below; compare Figures 39 to 42), but a potent inhibitor of colon cancer cell invasion and proliferation.

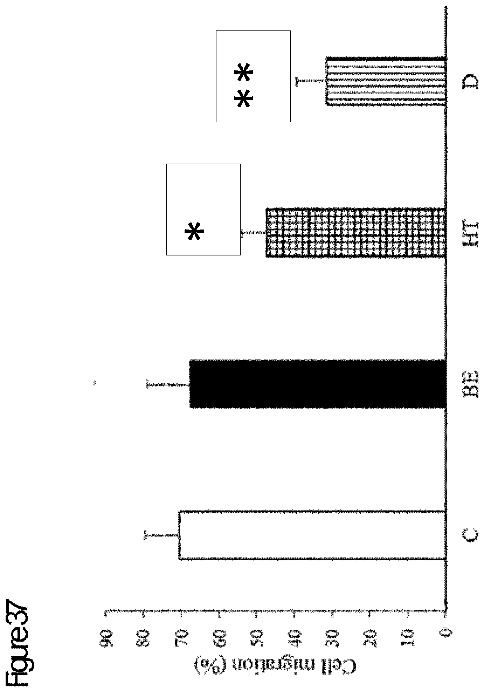

Isolated deflamin activities in HT29 cells were characterised while comparing it to the total extract and to the heat-treated extract of *L. albus.* Figures 37 to 38 shows the effect of each of these protein fractions on HT29 cell migration after 48 h of exposure to the total extract, to the heat treated extract and to isolated deflamin (50 µg protein/mL).

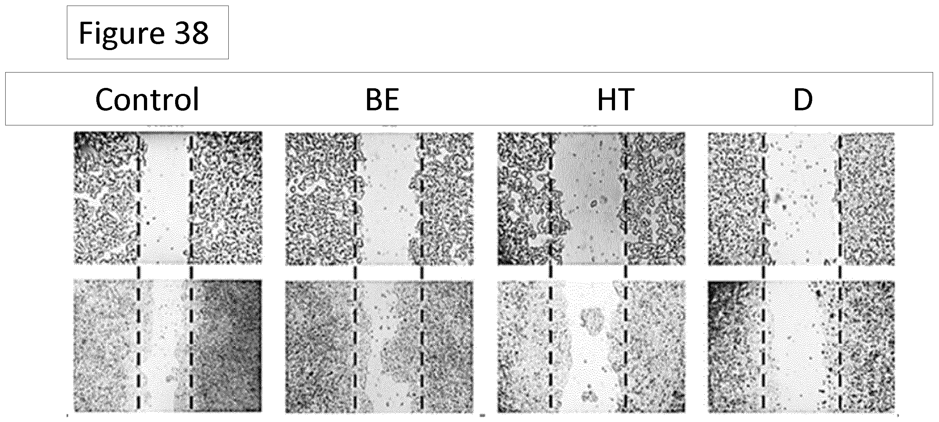

Figures 37 and 38 show HT29 cell migration after exposure to Buffer Extraction (BE), Heat treatment (HT) and isolated deflamin (D), as determined by wound healing assays. (Figure 37) - Relative migration rates. Values are the means of at least three replicate experiments ± SD, and are expressed as % wound closure in relation to day 0. (Figure 38) - Examples of cell migration obtained for the highest inhibitory protein fraction, i.e. deflamin. Cells were grown until reaching 80% confluence and the monolayer was scratched with a pipette tip (day 0). Cells were then exposed to 50 µg protein/ml extract and cell migration was recorded after 48 h. _{*} P < 0.05, _{**} P < 0.001.

These results show that deflamin presented the highest inhibition in migration rates when compared to the other protein samples studies (P<0.05), inducing a 60% reduction in cell migration rates. Furthermore, at the concentration used, HT and deflamin were statistically different from controls (P<0.05) whilst the BE sample remained statistically similar to controls (P>0.05). This result may explain, at least to some extent, why deflamin was not discovered before.

### L. albus Deflamin Activities Are Dose Dependent

The methodology developed to isolate deflamin (depicted in Figure 4 and described in detail in the Methods section) demonstrated to be highly efficient in isolating the MMPI fraction responsible for *L. albus* MMPI activities. The effect of this fraction (i.e. deflamin) was further tested to see if it was dose-dependent. A set of four different deflamin concentrations (100, 50, 10 and 5 µg/mL) were tested using the DQ gelatin method and the wound invasion assay in HT29 colon cancer cells and the results are expressed in Figures 39 and 40 to 41, respectively.

Figure 39 shows the effect of different concentrations of deflamin (100, 50, 10 and 5 µg/mL) on gelatinase activities. Four different concentrations of deflamin were obtained from *L. albus* seeds and used to assess their inhibitory activity upon the proteolytic activity of MMP-9 on DQ-gelatin. The negative control (C) does not inhibit MMP-9, resulting in 100% proteolytic activity for this protease. Deflamin was added at concentrations of 100, 50, 10 and 5 µg.mL-1 and gelatinolytic activity was measured by the DQ fluorogenic assay. Gelatinase activities are expressed as relative fluorescence as a % of controls, and represent the averages of at least three replicate experiments (n = 3) ± SD.

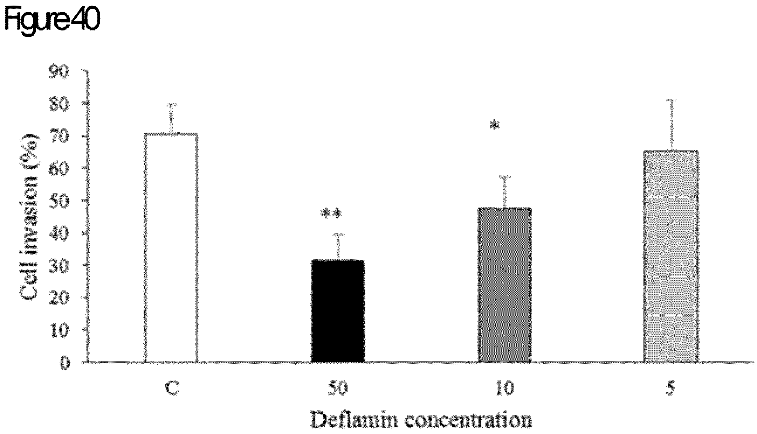

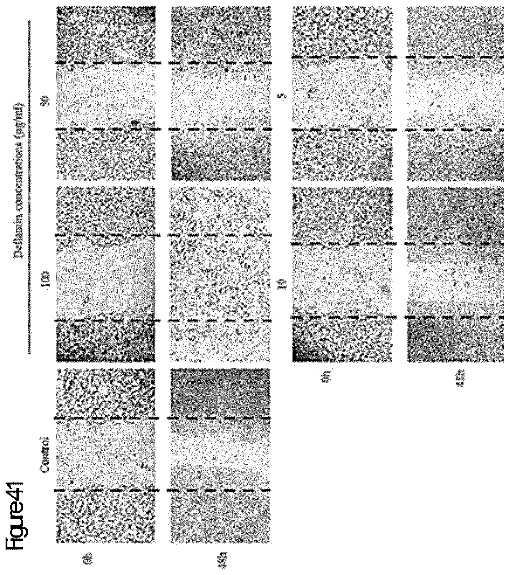

Figures 40 to 41 show HT29 cell migration after exposure to different concentrations of deflamin, as determined by wound healing assays. (Figure 40) - Relative migration rates. Values are the means of at least three replicate experiments ± SD, and are expressed as % wound closure in relation to time 0. (Figure 41) - Examples of cell migration obtained for the four deflamin concentrations tested plus the control. Cells were grown until reaching 80% confluence and the monolayer was scratched with a pipette tip (day 0). Cells were then exposed to 100, 50, 10 and 5 µg/mL deflamin and cell migration was recorded after 48 h. _{**} represents P<0.001 and _{*} represents P<0.05 when compared to controls.

Figure 40 shows that all concentrations tested (100, 50, 10 and 5 µg/mL) were able to significantly inhibit gelatinase proteolytic activity (P<00.1), when compared to controls. However, the inhibition level in each concentration differed, in a dose-dependent manner, with the highest inhibition detected for 100 µg/mL of deflamin, which induced a reduction greater than 90% of gelatinolytic activity. Figures 40 and 41 shows that the capacity of deflamin to inhibit colon cancer cell invasion.

Figures 40 and 41 shows that the capacity of deflamin to inhibit HT29 cell migrations gradually increases with deflamin concentration, from 5 to 50 µg deflamin/mL. However, for the highest deflamin concentration studied (100 µg/mL), a different and interesting result was obtained: HT29 cells were completely detached at 100 µg deflamin/mL (see Figures 40 and 41), justifying the absence of this concentration in the graph from Figure 40. *L. albus* deflamin does not reduce cell growth and metabolism in colon cancer cells.

To test whether deflamin was cytotoxic to HT29 cells, and if it influenced cell growth, we tested the same concentrations using a standard cell proliferation assay.

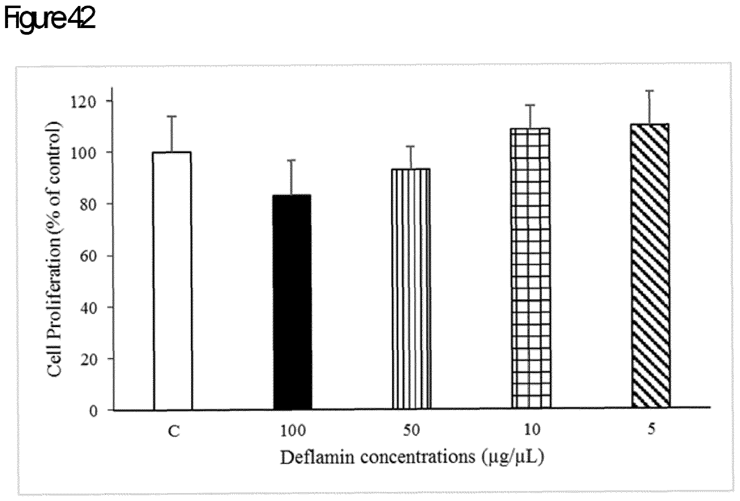

Figure 42 illustrates the number of HT29 living cells after growth in the presence of different deflamin concentrations (100, 50, 10 and 5 µg/mL), determined after staining with MTT (which can only be metabolized by living cells). The results show that a 2-day exposure to deflamin did not induce a significant reduction (P>0.001) in cell growth or in the number of living cells, when compared to controls. Furthermore, there were no visible cytotoxic effects. This result indicates that deflamin is relatively non-citotoxic to HT29 cells even at 100 µg deflamin/mL.

Figure 42 shows HT29 cell proliferation after a 24 h exposure to different concentrations of deflamin. Cells were grown for 24 h in the presence of 100, 50,10 and 5 µg protein/mL extract and stained with MTT. Values represented are the means of at least three replicate experiments (n = 3) ± SD and are expressed as a percentage of the control.

Since cell growth was not impaired by deflamin, minimal inhibitory concentrations (MICs) were determined for cell invasion, cell detachment, MMP inhibition and cell growth.

The results presented in Figure 42 show that a 2-day exposure to deflamin did not induce a significant reduction (P>0.001) in cell growth or in the number of living cells, when compared to controls. Furthermore, there were no visible cytotoxic effects (data not shown). This result indicates that deflamin is relatively non-cytotoxic to HT29 cells even at 100 µg deflamin/mL and that it does not interfere with the normal cellular metabolism.

However, for the highest deflamin dose, HT29 cells were detached (Figures 40-41) which if not related to any degree of cytotoxicity, it might possibly be related to cell adhesion. It is known that cells adhere to a substrate via their integrins, i.e. transmembrane receptors that are the bridges for cell-cell and cell-extracellular matrix (ECM) interactions. One important function of integrins on cells in tissue culture is their role in cell migration. Recent studies demonstrated that integrins are modulated by tumour progression and metastasis and are tightly connected to both MMP-9 and MMP-2 activities. Nevertheless, few studies have shown a cell detachment effect in the presence of MMPIs. These results suggest that deflamin's mode of action might involve a broader mechanism than induces more than just gelatinase inhibiting. The observation that the highest deflamin dose tested (i.e. 100 µg/mL) causes no apparent cytotoxic effect suggests it is not harmful to the digestive system and may therefore be used in preventive diets, without any secondary effects.

Since cell growth was not impaired with deflamin, we set out to determine the minimal inhibitory concentrations (MICs) and the concentration necessary to induce 50% effect (EC50) of deflamin, in the different tests: cell growth, cell invasion, cell detachment and MMP inhibition. Results are present in Table 1.

**Table1. Determination of Minimal Inhibitory Concentrations (MICs) and the concentrations which induce a 50% effect (EC50) for deflamin bioactivities on cell growth, cell invasion, cell detachment and MMP inhibition. Results are expressed in µg/mL. ND: not determined.**

| | MIC (µg.mL⁻¹) | EC50 (µg.mL⁻¹) |
|---|---|---|
| **Cell Growth** | >100 | > 100 |
| **Cell Invasion** | <10 | 10 |
| **Cell Detachment** | 100 | ND |
| **MMP inhibition** | <5 | 10 |

Under the conditions tested, MIC values for cell invasion and MMP inhibition were lower than the MICs found for the other parameters studied. A 10 µg.mL-1 deflamin concentration was found enough to significantly inhibit 50% of cell invasion (P<0.05) making it the EC50 value for cell invasion. For MMP inhibition the EC50 is of 10 µg deflamin/mL as well. This is in accordance to the high relation between MMP-9 activities and cell invasion, and corroborates that MMP inhibition is at least one of the major modes of action of deflamin. Nonetheless, the MIC levels determined for cell invasion were lower than 10 µg/mL but were not statistically significant (P<0.05) at 5 µg/mL, whilst MMPs were already very significantly inhibited in the presence 5 µg/mL, which is why the MIC values are lower than this concentration. With MIC values lower for MMP inhibition than for cell invasion, it is expected that MMP inhibition only induces a noticeable cell invasion reduction after a certain degree of inhibition. On the other hand, the MIC for cell detachment was only achieved for >100 µg/mL, at the highest deflamin concentrations tested, at which no significant cell toxicity was detected.

Clearly, MMP inhibition and the reduction in cell invasion are the strongest activities of deflamin, when compared to cell growth impairment or cytotoxicity which were only affected in a very low degree. This could be of significant importance. MMPIs with high specificity and low side effects have been very hard to find, and most clinical trials yielded unsatisfactory results. On the other hand, in cancer preventing diets, reducing MMP-9 and -2 activities in low amounts is desired but low toxicity levels against colon cells even in higher doses are a very important requirement. Compared to low molecular mass compounds such as polyphenols, polypeptide MMPIs offer various advantages, such as high specificity and low toxicity. Compared to the traditional cancer treatments such as chemotherapy or radioactive treatment, peptides and small proteins with high specificity against tumor promoters such as MMPs that simultaneously present low toxicity may represent the future in cancer treatment / prevention.

### L. albus Deflamin is a Complex of Low Molecular Mass β-Conglutin and δ-Conglutin Fragments

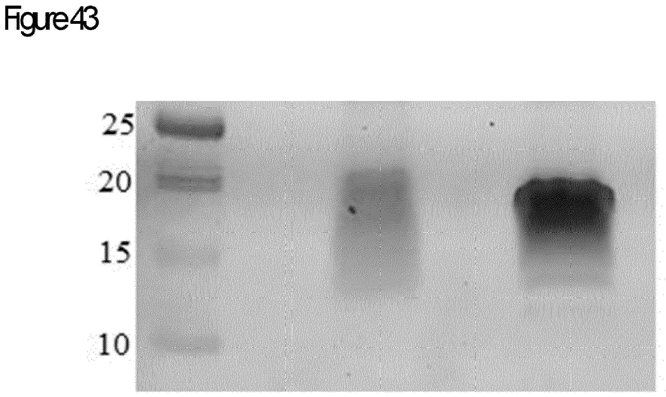

Since the deflamin fraction analysed in Figure 35 seemed to be composed by more than one polypeptide band, the same sample (i.e. isolated deflamin) was fractionated by electrophoresis, under reducing and non-reducing conditions to detecte its polypeptide composition as well as to determine the potential presence of disulphide bonds. The results obtained are presented in Figure 43.

Figure 43 shows the deflamin polypeptide profile under reducing and non-reducing conditions. Representative image of the polypeptide distribution of isolated deflamin from *Lupinus albus* seeds separated by SDS-PAGE under reducing (R) and non-reducing (NR) conditions. Deflamin (50 µg/mL) was loaded onto a 17.5% (w/v acrylamide) polyacrylamide gel with reducing buffer (100 mM Tris-HCl buffer, pH 6.8, containing 100 mM β-mercaptoethanol, 2% (w/v) SDS, 15% (v/v) glycerol and 0.006% (w/v) m-cresol purple) and non-reducing buffer (100 mM Tris-HCl buffer, pH 6.8, containing 2% (w/v) SDS, 15% (v/v) glycerol and 0.006% (w/v) *m*-cresol purple).

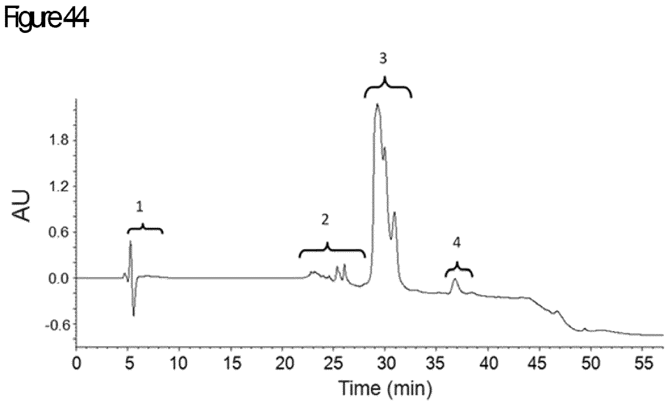

Deflamin was further analysed by reverse-phase HPLC, in order to separate its different polypeptide constituents. Figures 44 to 46 shows the chromatographic profiles obtained at 280 and 214 nm, and the respective electrophoretic patterns. Results show the presence of the deflamin standard bands, scattered throughout peaks 2 to 4.

The 280 nm peak eluting from the HPLC reverse phase column at 45 to 50 min does not contain neither protein nor bioactivity. For this reason, its study was discontinued.

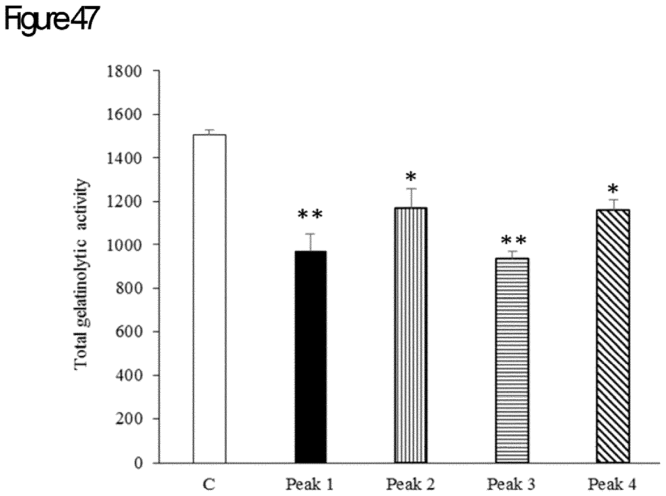

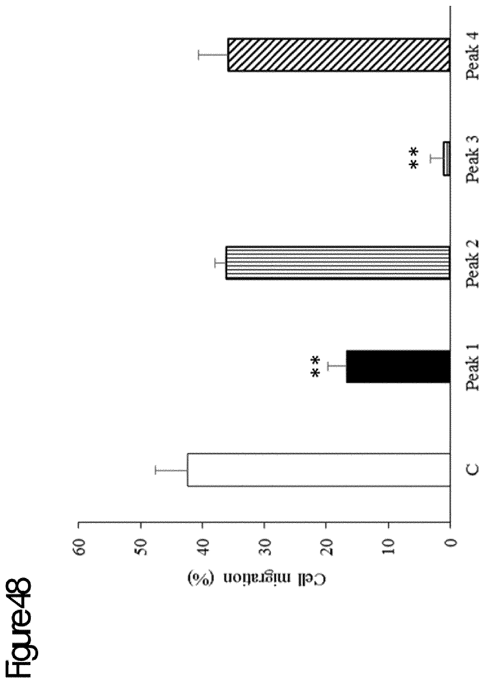

In order to determine the peak fraction with higher activity, we further determined the MMPI activities of the 4 peaks (Figures 47 and 48), using the DQ gelatin and the cell invasion assays. Results are shown in Figures 47 and 48, respectively.

Results seem to suggest that all the selected peaks presented some degree of MMP inhibition and also inhibited invasion, but peak 3 was the one with the highest observed activities. The 4 selected peaks were further analysed by mass spectrometry, for identification.

### Deflamin Is the First Proteinaceous MMPI Which Can Be Purified by a Cost-Effective and Up-Scalable Procedure

Legume seeds have been long recognized by containing a variety of proteinaceous enzyme inhibitors, such as the trypsin inhibitors and the BBIs. However, although the presence of MMPIs of natural occurrence may be considered ubiquitous in plant tissues, all of them present several disadvantages when considering their production for clinical purposes: toxicity in high concentrations or prolonged exposures; chemical inactivation (e.g. denaturation) or destruction (e.g. proteolysis) upon cooking and/or by the digestive process; the lack of a specific; and a high-cost and inefficient method of isolation, which prevent MMPIs in general to undergo efficient scaling-up to an industrial level. Isolated deflamin reported in this work surpasses all of these constraints, as it is resistant to boiling and is an enzyme inhibitor; on the other hand, the sequential precipitation method developed is simple, cost-effective and easily applied in an industrial context As a mixture of edible polypeptides which occur naturally in lupin seeds, it doesn't pose the problem of toxicity in higher doses, that most phenolic compounds and other bioactive secondary metabolites do, and the use of the acid and ethanol precipitations assures the removal of possible toxic contaminants as well as higher molecular mass proteins.

The yield of the extraction procedure is present in Table 2. Results show that per 100 g of dry seed we obtain 100 mg of deflamin, which correspond to around 0.5% of total protein content of the seed.

**Table 2. Yield (expressed in %) of dry L. albus seeds in deflamin.**

| | Deflamin | % Yield |
|---|---|---|
| Per 100 g of dry lupin seed | 100 mg | 0.1% |
| Per 100 mg of total protei n | 520 µg | 0.5% |

These results corroborate that deflamin is indeed present in very low concentrations in the seed, hence the lower activities observed in the BE fractions. It also suggests that the consumption of lupin alone may not provide enough deflamin to induce the same effects that its isolated form can provide.

It is important to notice that the low yields of the extraction procedure are not due to the method itself, but rather to the low amount of deflamin in the seed. Still, the relative easiness of the procedure and the possibility to up-scale to larger amounts, in a cost-effective and simple manner, using filtrations and flow centrifugation as well as low cost reagents such as ethanol suggest a high potential for industrial production.

Furthermore, given the potential of deflamin, our developed procedure is also of particular importance to pursue a more thorough characterization of this proteinaceous fraction, such as its identity, dose-response effects and EC50, as well as clinical and pre-clinical studies. Research on other varieties and species of lupins, on seeds from other legume species and on seeds from other plant families, as well as changes in their growth conditions may provide an increase in the amount of deflamine in the seed. The fact that isolated deflamin is efficient in inhibiting MMP-9 and reducing cancer cell invasion suggests its high potential for a vast array of clinical uses. Since MMP-9 is closely involved in inflammation as well as in oncologic processes, the MMPI deflamin could possibly be used in both anticancer approaches as well as anti-inflammatory treatments, especially those related to the digestive tract, such as colorectal cancer (CRC) and inflammable bowel diseases (IBDs).

### Identification of the Polypeptides Comprising L. albus Deflamin by Mass Spectrometry - I

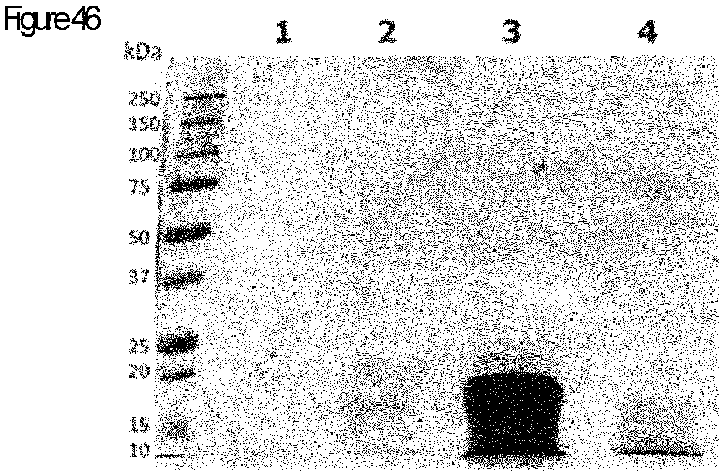

Deflamin was isolated from *Lupinus albus* seeds following the methodology detailed in the embodiment of Figure 4. Deflamin was subsequently fractionated by RP-HPLC into the four peaks depicted below. The polypeptides comprising each of these peaks as indicated in Figure 46 were identified by mass spectrometry. The results obtained are presented in (peak 1), (peak 2), (peak 3) and (peak 4) below and indicate the presence of the following peaks:
- Peak 1: fragments of conglutin beta 1, 2, 3, 4, 5 and 7. Note that β-conglutin fragments were detected which span the entire molecule. No δ-conglutin fragments were detected in peak 1.
- Peak 2: fragments of conglutin beta 1, 2 and 6, and conglutin delta-2 large chain. Note that β-conglutin and δ-conglutin fragments were detected which span the entire molecules of their precursors.
- Peak 3: fragments of conglutin beta 1 and conglutin delta-2 large chain. Note that β-conglutin and δ-conglutin fragments were detected which span the entire molecules of their precursors.
- Peak 4: fragments of conglutin beta 1, 2, 3, 6 and 7. Note that β-conglutin fragments were detected which span the entire molecule. No δ-conglutin fragments were detected in peak 1.

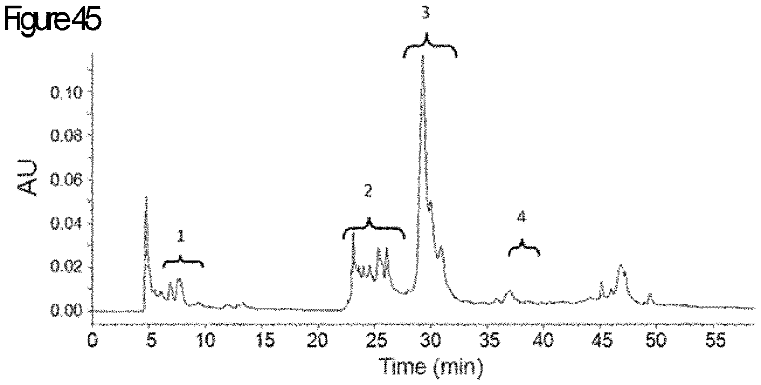

In other words, Figures 44 to 46 show representative images of deflamin fractionation by RP-HPLC and SDS-PAGE into its constituent polypeptides. Deflamin was extracted and purified from *Lupinus albus* seeds by the methodology developed and illustrated in Figure 4. (Figures 44 and 45) - Reverse Phase (RP)-HPLC chromatography of deflamin monitored at 214 nm (Figure 44) and at 280 nm (Figure 45). (Figure 46) Polypeptide profile of each peak collected from the HPLC run as visualized by SDS-PAGE (17.5% w/v acrylamide) performed under reducing conditions (R-SDS-PAGE). Protein peaks (50 µg) were loaded onto 17.5% (w/v acrylamide) polyacrylamide gels. Total polypeptides were stained with Coomassie Brilliant Blue.

Figure 47 shows MMP-9 proteolytic activity of fractions 1 to 4 obtained by HPLC fractionation of deflamin. Protein samples were added at a concentration of 25 µg/ml and gelatinolytic activity was measured by the DQ fluorogenic assay. Results are expressed in arbitrary units of fluorescence and represent the averages of at least three replicates experiments (n = 3) ±SD. _{**} Represents P<0.001 and _{*} represents P<0.05 when compared to controls.

Figure 48 shows HT29 cell migration after exposure to each of the selected deflamin peaks collected after RP-HPLC fractionation - Relative migration rates. Values are the means of at least three replicate experiments ± SD, and are expressed as % wound closure in relation to day 0. Cells were grown until reaching 80% confluence and the monolayer was scratched with a pipette tip (day 0). Cells were then exposed to 25 µg protein/ml extract and cell migration was recorded after 48 h. _{**} Represents P<0.05.

Two main conclusions may be drawn from all these results:
- *L. albus* deflamin is composed of a complex mixture of β-conglutin and δ-conglutin fragments;
- β-Conglutin and δ-conglutin are both precursors of *L. albus* deflamin.

Mass spectrometry analyses of the peak components (see Figure 44-46 and SEQ ID Nos: 194-197) of *L. albus* deflamin. The fractionation of *L. albus* deflamin by RP-HPLC of deflamin resulted in 4 peaks, each of which contain polypeptides that were identified by mass spectrometry.

Colour code indicates peptides confidence:
- green residues corresponds to peptides with 95% confidence;
- yellow for peptides with confidence between 50 and 95%;
- red for peptides with confidence bellow 50%;
- and grey corresponds to unidentified residues.

### Splitting L. albus Deflamin in Two Fractions with Ca2+ e Mg2+

Isolated deflamin from *L. albus* comprises two fractions (one derived from β-conglutin, the other from δ-conglutin) which are not separated by HPLC, but which can be in fact separated through the addition of Ca2+ and Mg2+, since β-conglutin binds these cations, self-aggregates and becomes insoluble.

### Methodology

The deflamin fraction isolated by HPLC was lyophilized and dissolved in water and agitated. To the deflamin solution CaCl2 and MgCl2 from a stock solution were added until reaching 10 mM CaCl2 and 10 mM MgCl2, followed by agitation for 4 h or overnight. The suspension was centrifuged for 1 h at 30,000 g. The supernatant and pellet were desalted on NAP-10 columns previously equilibrated in water and lyophilized for future analysis. All operations were performed at 4 °C.

Both lyophilized fractions were used for electrophoretic separation, and their MMP-9 inhibitory activity was determined using the fluorogenic DQ-gelatin assay, the wound healing assay in HT29 cells and substrate zymography, as described earlier.

### Results

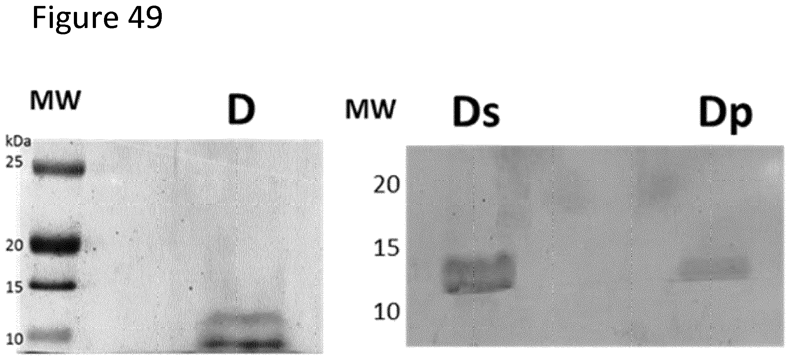

Figure 49 shows the electrophoretic profiles of both the Ca/Mg soluble and insoluble deflamin fractions. The figures show electrophoretic profiles of the Ca/Mg soluble and insoluble deflamin fractions. D - deflamin isolated by RP-HPLC; Ds - deflamin fraction which did not precipitate in the presence of the cations (supernatant); Dp- deflamin fraction which precipitated in the presence of the cations (pellet).

### Splitting L. albus Deflamin into Two Fractions Influences its Anticancer Activity

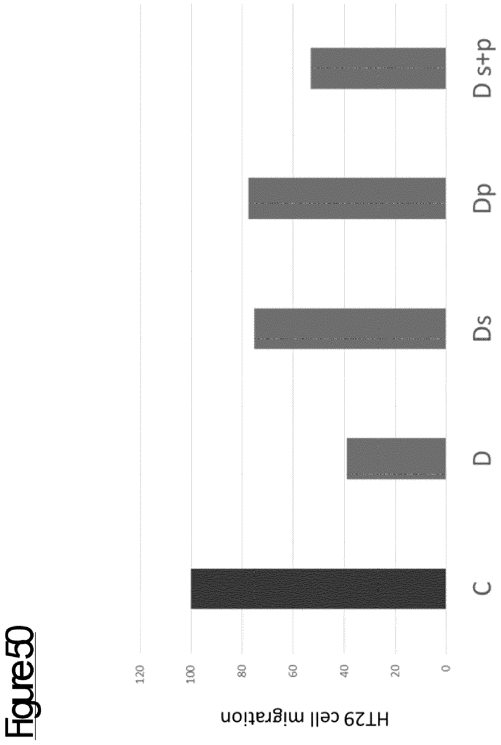

When split in two fractions, *L. albus* deflamin loses its activity of cell invasion inhibition. But when these fractions are combined again, it recovers part of the initial activity. These results, presented in Figure 50, suggest that the two fractions are essential for the expression of deflamin bioactivity.

Figure 50 shows Inhibition of cell invasion in HT29 cells by deflamin and deflamin subfractions, precipitated or not with a solution of 10 mM CaCl2 and 10 mM MgCl2. C - control; D - deflamin; Ds - fraction of deflamin which did not precipitate in the presence of cations (supernatant); Dp - fraction of deflamin that precipitated in the presence of the cations (pellet); and D s+p - both fractions, Dp and Ds, combined in equal parts.

### Influence of L. albus Deflamin on the Expression of Genes Related to Inflammation and Tumor Invasion

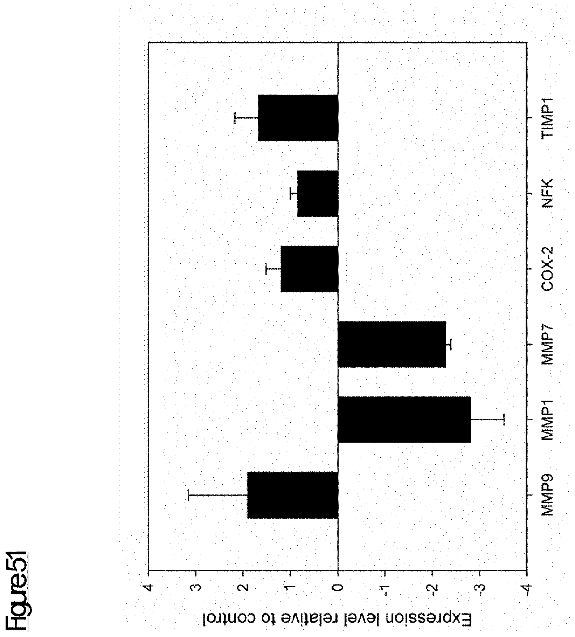

Based on the natural history of certain diseases and epidemiology studies, a strong association has been established between particular chronic inflammatory conditions and eventual tumor appearance. Certain genes are more highly expressed during these pathologies and the proteins they express are usually recognized as biomarkers of inflammation and tumorigenesis. Such examples are: several types of MMPs, such as MMP-1, MMP-7, MMP-9 and MMP-2, and also tumor necrosis factor alfa (TNFα), a cell signaling protein (cytokine) involved in systemic inflammation, nuclear-factor kappa B (NF-*κ*B), a protein complex that controls transcription of DNA, and TIMP1, an endogenous tissue inhibitor of metalloproteinases, as well as the inflammatory mediators iNOs and COX2. Some of these were tested in Figure 51.

### Methods

HT29 cells were exposed to 50 µL/g of deflamin and allowed to grow for 24 h. Total RNA was extracted from HT29 cells using the NZY Total RNA isolation kit (Nzytech) with some modifications, and quantification was carried out in a Synergy HT Multiplate Reader, with Gene5 software, using a Take 3 Multi-Volume Plate (Bio-Tek Instruments Inc. Winooski, USA). For reverse transcription, the RevertAid reverse transcriptase priming with oligod(T) kit was used (Thermo Scientific) according to the manufacturer's recommendations.

A set of primers for specific genes related to inflammation and cancer invasion were used. When amplification confirmed the expression, the transcripts were quantified by real-time PCR (qPCR), performed in 20 µL reaction volumes composed of cDNA derived from 2 µg of RNA, 0.5 µM gene-specific primers (Table 3), and SsoFast EvaGreen Supermixes (Bio-Rad, Hercules, CA) using an iQ5 Real-Time Thermal Cycler (BioRad, Hercules, CA). Reaction conditions for cycling were 95 °C for 3 min followed by 40 cycles at 95 °C for 10 s, 61 °C for 25 s, and 72 °C for 30 s. Melting curves were generated in each case to confirm the amplification of single products and absence of primer dimerization. Each analysis was performed in triplicate reactions, each in three biologic replicates (n = 9, in which each replicate is the average of three technical measurements). The corresponding quantification cycles (Cq) obtained by the iQ5 optical system software (Bio-Rad, Hercules, CA) were exported to a MS Excel spreadsheet (Microsoft Inc.) for quantification. Cq values of each gene of interest were normalized with respect to actin (Act). Relative gene expression values in deflamin exposures are presented in Figure 51 below, as fold-change values in relation to control conditions.

**Table 3 - Specific pairs of primers used to assess the transcription of the genes under study**

| **Accession number (NCBI)** | **ID attributed** | **Primer sequences** |
|---|---|---|
| | | |
| **NM_001145938** | HsMMP1 | TTCGGGGAGAAGTGATGTTC TTGTGGCCAGAAAACAGAAA |
| **NM_002423** | HsMMP7 | GTATGGGACATTCCTCTGATCC CCAATGAATGAATGAATGGATG |
| **NM_004994** | HsMMP9 | GCACGACGTCTTCCAGTACC CAGGATGTCATAGGTCACGTAGC |
| **NM_000963** | HsCOX-2 | TGAGCATCTACGGTTTGCTG AACTGCTCATCACCCCATTC |
| **NM_001165412** | HsNFKβ1 | TGGAGTCTGGGAAGGATTTG CGAAGCTGGACAAACACAGA |
| **NM_003254** | HsTIMP1 | AGGCTCTGAAAAGGGCTTCC GGACACTGTGCAGGCTTCAG |

### Results

The results presented in Figure 51 suggest that deflamin does not significantly alter the expression of specific genes related to inflammation and tumorigenesis, nor related to MMP-9, corroborating the hypothesis that deflamin has no significant direct activity on gene expression, but rather acts through direct interaction with MMP-9. Of note is a small inhibition of the expression of genes associated with MMP-1 and MMP-7, which usually show enhanced expression during advanced metastatic disease.

Figure 51 shows in other words transcriptional responses to deflamin in HT29 cells.

### Deflamin Activity in Food Products

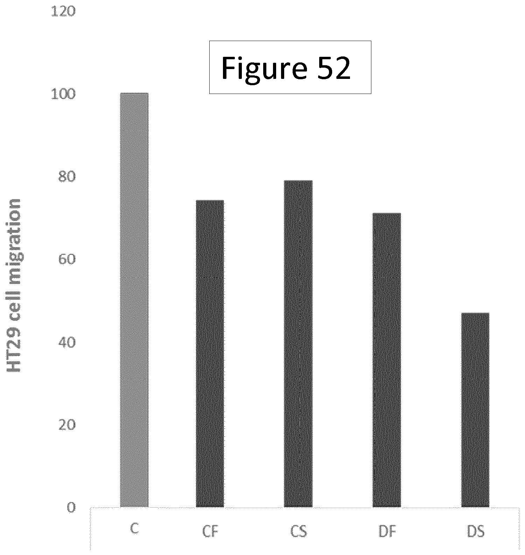

*L. albus* deflamin was used in the manufacture of salted cooked cookies. It is important to note that during this process, temperatures raised up to 180 oC. Nevertheless, the results obtained in Figure 52 shows that deflamin maintained its cancer cell invasion inhibitory activity in the savory cooked cookies.

Figure 52 shows the inhibition of cell invasion in HT29 cells by protein extracts prepared from cookies containing (D) or not (C) deflamin. C- control; CF - uncooked control cookies; CS - cooked cookies; DF - deflamin-containing uncooked cookies; DS - deflamin-containing cooked cookies.

### Isolation and Characterization of L. albus Deflamin - II

### Deflamin Analysis by RP-HPLC and Electrophoresis

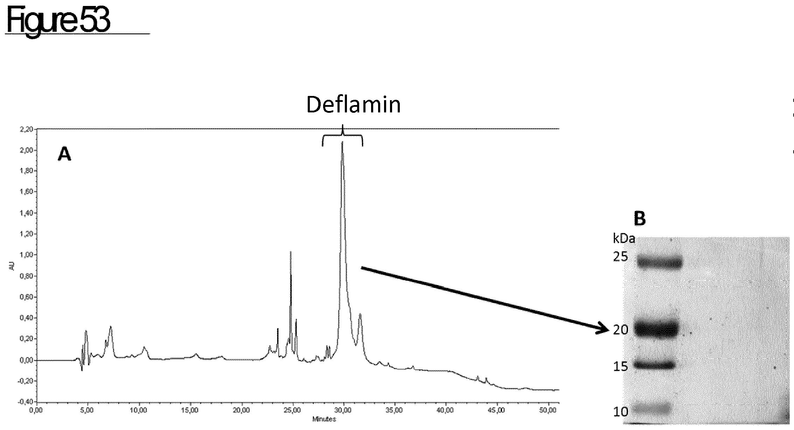

Figure 53 shows representative images of *L. albus* deflamin fractionation by RP-HPLC (eluants are acetonitrile and TFA) and the corresponding SDS-PAGE. Deflamin was extracted and purified from *Lupinus albus* seeds.

Figure 53 shows HPLC and Electrophoretic Profiles. A - Reverse Phase (RP)-HPLC chromatography monitored at 280 nm. Deflamin peak is identified by the arrow. B - Polypeptide profile of the deflamin peak collected from the HPLC run as visualized by SDS-PAGE (17.5% w/v acrylamide) performed under reducing conditions. The protein peak eluting at 30 min (50 µg) was loaded onto a 17.5% (w/v acrylamide) polyacrylamide gel and stained with Coomassie Brilliant Blue.

### Mass Spectrometry Analysis of the Two Fragments of Deflamin by MALDI-TOF - II

### Methods

The instrumentation comprised Ultraflex II MALDI-TOF TOF Bruker-Daltonics, equipped with a LIFT cell and N2laser.

### Ionization: MALDI

Operation mode: The mass spectrometer was operated with positive polarity in linear mode and spectra were acquired in the range of m/z 5000-20000. A total of 1000 spectra were acquired at each spot position at a laser frequency of 50 Hz. External calibration: a protein calibration standard I from Bruker ([M+H]+ of insulin (5734.51 m/z); ubiquitin I (8565.76 m/z), cytochrome c (12360.97 m/z), myoglobin (16952.30 m/z); [M+2H]2+ of cytochrome c (6180.99 m/z) and myoglobin (8476.65 m/z)).

### Results

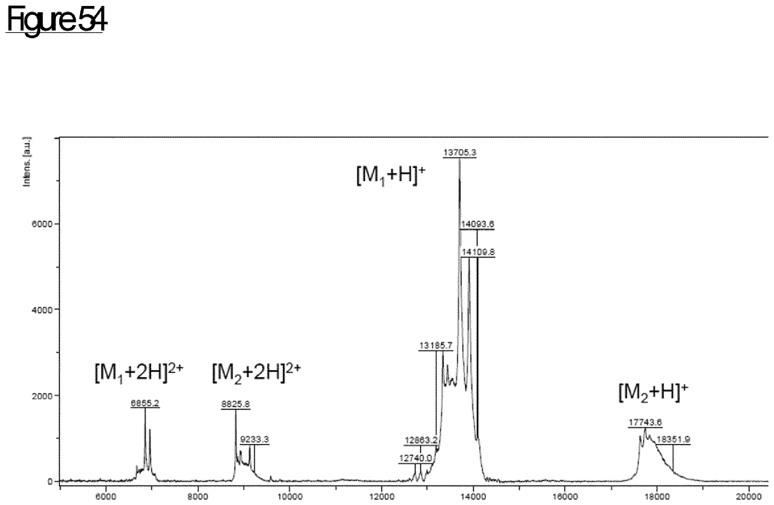

The MALDI-TOF analysis of *L. albus* deflamin clearly shows the presence of two blocks of polypeptide fragments with masses of 13 and 17 kDa, composed of several fragments homologous to each other, with slightly different lengths (Figure 54).

Figure 54 shows in other words deflamin from *L. albus* analysed by MALDI-TOF MS.

### Deflamin Antimicrobial Activity

Deflamin antimicrobial activity was tested against the following microorganisms and found to be null:
Gram+ bacteria:
   - Listeria monocytogenes (NCTC 11994)
   - Bacillus cereus (NCTC 7464)
   - Staphylococcus aureus (NCTC 10788)
Gram- bacteria:
   - *Escherichia coli* (NCTC 9001)
   - Salmonella Goldcoast (NCTC 13175).
Fungi:
   - *Alternaria alternata*
   - *Botrytis cinerea*
   - *Phaeoacremonium aleophilum*
   - *Alternaria sp.*
   - *Penicillium sp.*

### Animal Models of Colitis

### Preliminary Results on Anti-Colitis Effects of Deflamin

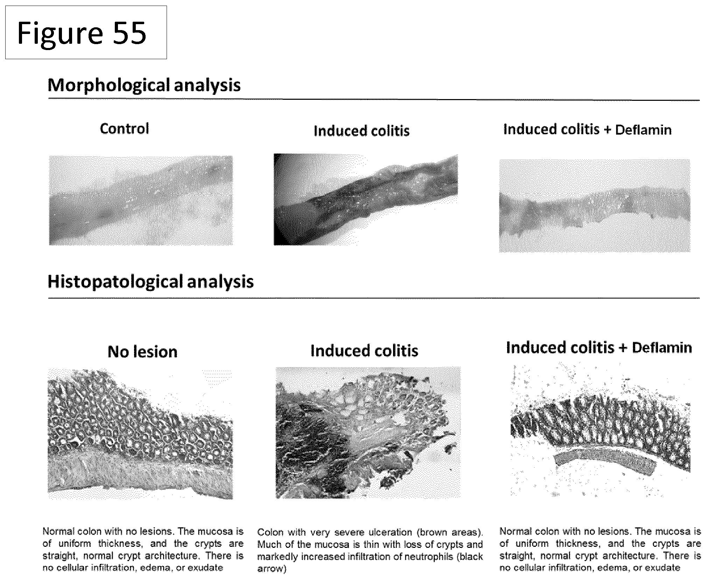

Preliminary results on the bioactivity of deflamin on colitis are shown in Figure 55. Preliminary results on anti-colitis effects of deflamin: representative images of the colitis-induced lesions in comparison with control and deflamin treatments in mice models. The assays were made as a preliminary study with only one concentration of deflamin introduced in their diets after the induction of colitis.

Deflamin administration reduces the macroscopical and functional signs of colitis injury

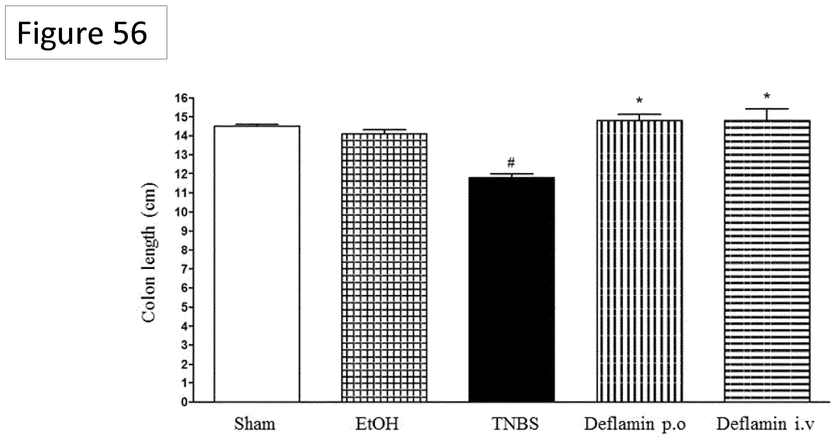

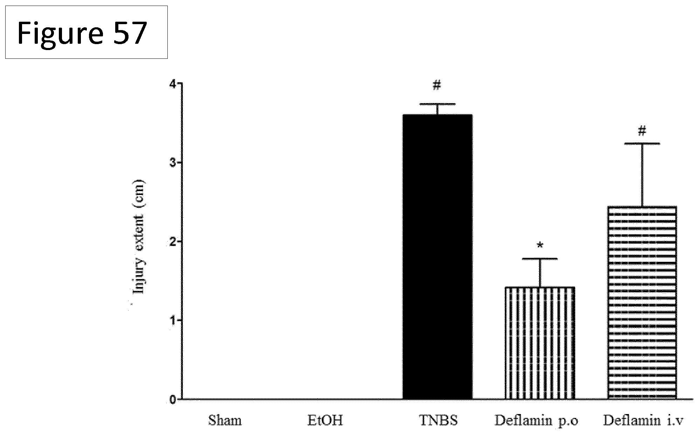

In order to ascertain the anti-inflammatory effects of deflamin, its effects were tested on mice with TNBS-induced colitis, using two types of administrations, oral administration (p.o.) and intraperitoneal injection (i.p.).Deflamin was administered 3 h after colitis induction. Figures 56 and 57 show the effect of deflamin on the length of colons (cm) and on the extent of intestine injury (cm), respectively.

Figure 56 shows the effect of deflamin administration on the length of colon (cm). Sham group (n = 6), EtOH group (n = 6), TNBS group (n = 10), TNBS+deflamin p.o. group (n = 9) and TNBS+deflamin i.p. group (n = 10). #P<0.001 vs Sham group, *P<0.001 vs TNBS group.

Figure 57 shows the effect of deflamin administration on the extent of intestine injury (cm). Sham group (n = 6), EtOH group (n = 6), TNBS group (n = 10), TNBS+deflamin p.o. group (n = 9), TNBS+deflamin i.p. group (n = 10). #P<0.001 vs Sham group, *P<0.01 vs TNBS group.

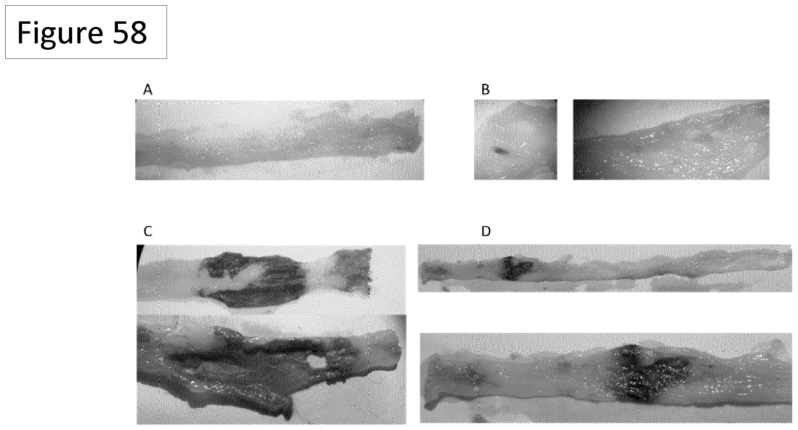

Figure 58 shows the effect of deflamin administration on the macroscopic observation of colon. (A) Sham group (n = 6), (B) EtOH group (n = 6), (C) TNBS group (n = 8), (D) TNBS+deflamin p.o. group (n=9). #P<0.001 vs Sham group, *P<0.01 vs TNBS group.

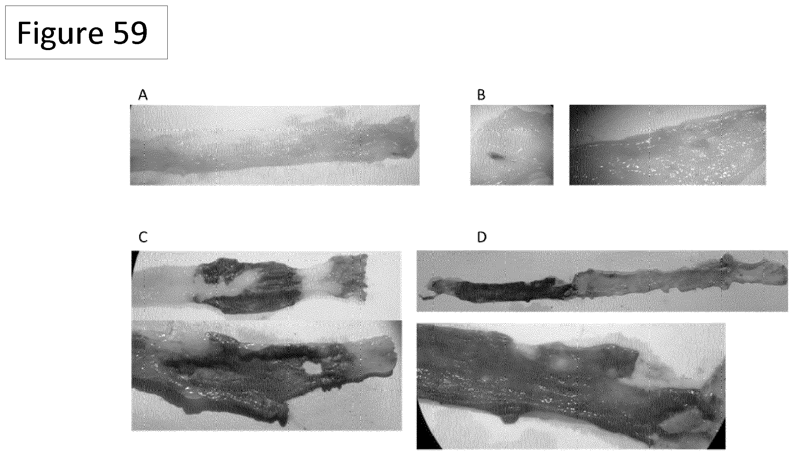

Figure 59 shows the effect of deflamin administration on the macroscopic observation of colon. (A) Sham group (n = 6), (B) EtOH group (n = 6), (C) TNBS group (n = 8), (D) TNBS+deflamin i.p. group (n = 10). #P<0.001 vs Sham group, *P<0.01 vs TNBS group.

The animals in the Sham and Ethanol Groups exhibited no macroscopical signs of colon injury, and presented no mortality, whilst intracolonic injection of TNBS/EtOH led to a very significant (P<0.05) decrease in colon length and an increase in the extent of visible injury (ulcer formation). In the deflamin-treatment group (both p.o. and i.p.) all of the macroscopical signs of colon injury were significantly reduced, comparing to the TNBS group (Figures 56 and 57).

The results show that the administration of deflamin (both i.p. and p.o.) led to an overall reduction in colon inflammation, and in the case of p.o., a significant (P<0.05) attenuation of colon length reduction, and a significant (P<0.05) reduction in the extent of visible injury (ulcer formation). These differences can be easily perceived by a microscopical observation of the fresh and rinsed colons immediately after colon collection at the end of the experiments. Figures 58 and 59 demonstrate representative pictures of these microscopic observations obtained by a bench surgical microscope of the colons isolated from the different treatments groups. Four days after intra-colonic administration of TNBS, the colons appeared flaccid and filled with liquid stool. Observations of images show a clear attenuation of colon injury in animal treated with deflamin when compared to the TNBS-induced colitis (Figure 42 and 43). Concerning the type of administration, some differences were observed between the two types of administrations when comparing the morphological signs of colitis and the extent of the colonic injury which were higher in i.p. administrations (Table 1 and Figure 59; P<0.05). The macroscopical observations also corroborate this trend when the two treatments were compared (i.e. p.o. vs i.p.; Figures 58 and 59).

Deflamin attenuates the histological features and inflammatory markers of colitis injury

In order to enlighten the mechanisms responsible for the effect of deflamin, we analyzed the severity of histological injuries and also determined the presence of specific markers of inflammation and cancer progression, COX2 and iNOS.

Decreased expressions of cyclooxygenase-2 (COX-2) and nitric oxide synthase (iNOS) in the colon tissue of experimentally induced colitis (D'Acquisto *et al.*, 2002), is associated to a reduction of the severity of colitis and an alleviation of the macroscopic and microscopic signs of the disease. Specifically in the intestine, up-regulation of the production of NO via expression of inducible iNOS represents part of a prompt intestinal antibacterial response; however, NO has also been associated with the initiation and maintenance of inflammation in human IBD (Kolios *et al.*, 2004) and studies have shown that the level of iNOS-derived NO correlates well with disease activity in ulcerative colitis (Cross & Wilson, 2003). Nitric oxide is produced and released locally in much greater quantities in the inflamed gut than in the noninflamed gut and was actually even suggested as a novel clinical biomarker for diagnosis and monitoring of IBD patients (Lundberg *et al.*, 2005). Similarly to COX-2, immunostaining assays in the experimental colitis study showed that there was in fact an increased expression of iNOS in animals subjected to colitis induction.

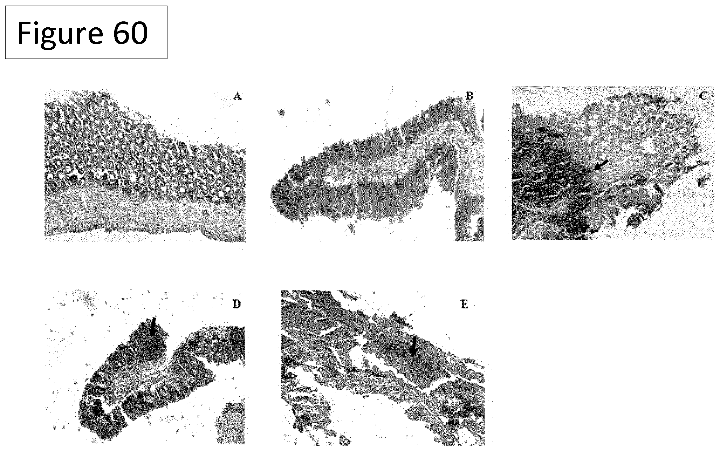

Histological evaluations are presented in Figure 60.

Figure 60 shows the effect of deflamin administration on the histological features of colon inflammation. (A) Sham group (n = 6), (B) EtOH group (n = 6), (C) TNBS group (n = 8), (D) TNBS+deflamin p.o. group (15 mg/kg, n = 9), (E) TNBS+deflamin i.p. group (10 mg/kg, n = 10).

While control samples showed a normal colon with no lesions, a mucosa of uniform thickness, normal crypt architecture and no signs of inflammation, in the TNBS treatments the colons exhibited severe ulceration with crypt loss and a thinner mucosa with a marked neutrophil infiltration, equivalent to score 3.

In comparison, the samples from animals administered with deflamin presented more moderate lesions with partial intact crypts and some neutrophil infiltration, indicating a lower damage score of 2, particularly in the p.o. administrations.

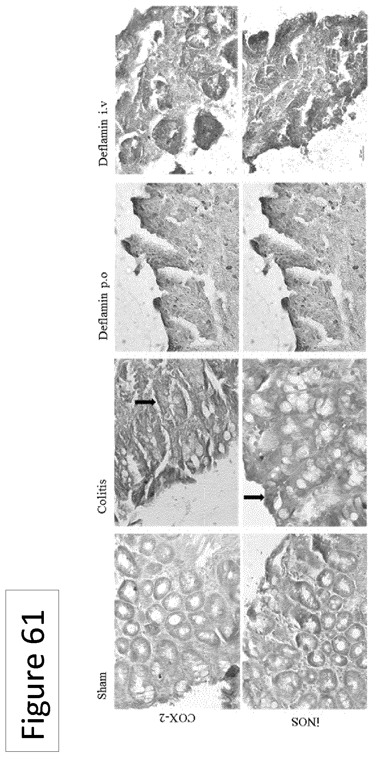

Figure 61 shows the effect of deflamin administration on the colon tissue expression of COX-2 and iNOS. (A) - COX-2 expression: (1) Sham group, (2) TNBS group, (3) TNBS+Deflamin p.o. group, (4) TNBS+ Deflamin i.p. group; (B) - iNOS expression: (1) Sham group, (2) TNBS group, (3) TNBS+ Deflamin p.o. group, (4) TNBS+ Deflamin i.p. group.

Figure 61 shows COX-2 and iNOS expression in colonic tissues. Results show that TNBS treatment induced a marked increase in COX-2 and iNOS expression along the remaining crypts, indicated by the brown color when compared with control samples. In accordance with the histological observations, animals treated with deflamin exhibited a reduced staining for both COX-2 and iNOS, indicating a reduction in the inflammatory processes on the colon, especially in the p.o. group.

### Deflamin Effects are Induced in Preventive as Well as in Curative Treatments

Since p.o. administrations induced better results, we further tested if a preventive approach through an oral diet supplementation with Deflamin, rather than a just a curative approach would present similar effects.

Table 4 shows the morphological and functional signs of colitis in both treatments, curative (D - p.o. administration of deflamin 3 h after TNBS induction) and preventive (Dp - o.p. administration of Deflamin 3 days prior to TNBS administration).

The animals in the Sham and Ethanol Groups exhibited no macroscopical signs of colon injury, and presented no mortality, whilst intracolonic injection of TNBS/EtOH led to a very significant (P<0.05) decrease in colon length and an increase in the extent of visible injury (ulcer formation) and diarrhea severity, exhibiting a mortality rate of 50%.

**Table 4. Morphologic and functional observations of the colon, immediately after collection, for both treatments, curative (D - p.o. administration of deflamin 3 h after TNBS induction) and preventive (Dp - p.o. administration of deflamin 3 days prior to TNBS administration). *p < 0.001 versus Sham group; #p < 0.05 versus TNBS group; and φp < 0.05 D group versus Dp group.**

| **Animal Group** | **Length of colon (cm)** | **Extent of injury (cm)** | **Presence/consistency of diarrhea** | **Mortality (%)** |
|---|---|---|---|---|
| Sham | 14.5 ± 0.08 | 0 | 0 | 0% |
| EtOH 50% | 14.1 ± 0.20 | 0 | 0 | 0% |
| TNBS | 11.8 ± 0.19* | 3.6 ± 0.14* | 3^{*} | 50% |
| NBS + D | 14.8 ± 0.33^{#} | 2.44 ± 0.84^{#} | 1.13 ± 0.35^{#} | 11% |
| TNBS + Dp | 13.11 1.98^{#} | 2.48 ± 1.24^{#} | 1.63 ± 0.74^{#} | 12% |

Results show that the administration of deflamin, both curative as well as preventive, led to an overall reduction in colon inflammation, with a significant (P<0.05) attenuation of colon length reduction, and a significant reduction (P<0.05) in the extent of visible injury (ulcer formation). Also, a significant decrease (P<0.05) in diarrhea severity, mortality rates and a reduction of general histological features of colon inflammation were observed when compared to the TNBS group. Furthermore, there were no significant differences (P>0.05) observed between preventive and curative approaches and between both deflamin treatments and the controls.

### Deflamin Reduces MMP-9 Activity in Fresh Colon Tissues

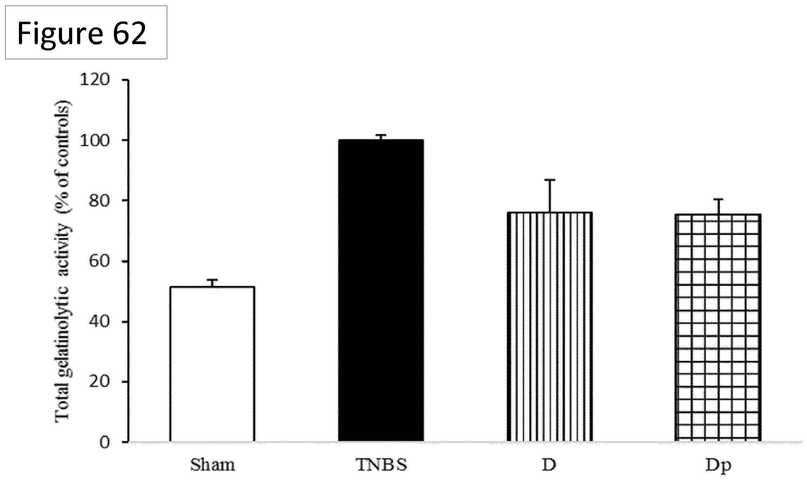

In order to test if the anti-inflammatory effects observed in the deflamin treatments were due to MMP-9 and/or MMP-2 inhibition, the gelatinolytic activities of these enzymes in the fresh colon tissues from the different experimental animal groups (curative and preventive) were tested, using the DQ-gelatin kit and zymographic assays. Figure 62 shows the total gelatinolytic activity present in the colon samples, as quantified by the quenched-dye DQ-gelatin method.

Figure 62 shows the effect of deflamin administration on the colon tissue gelatinase activities of MMP-2 and MMP-9 from colitis-induced mice. Proteolytic activity of the gelatinases presents in colons was quantified by the DQ fluorogenic method. Results are expressed as relative fluorescence as a % of controls and represent the average of at least three replicate experiments (n = 3) ± SD. TNBS group = C+; Sham group = C-; D = Curative treatment with deflamin (15 mg/kg, n = 9, p.o.); and Dp = Preventive treatment with deflamin (15 mg/kg, n = 6, p.o.). *p < 0.001 versus Sham group; #p < 0.05 versus TNBS group; and φp < 0.05 D group versus Dp group.

Figure 62 shows that the TNBS induced a very significant increase in gelatinolytic activities, when compared to controls (P<0.001), whereas both curative and preventive deflamin administrations reduced significantly (P<0.05) the total MMP-9 + MMP-2 activity when compared to the TNBS treatment, but there were no significant differences (P>005) between the curative and preventive administrations.

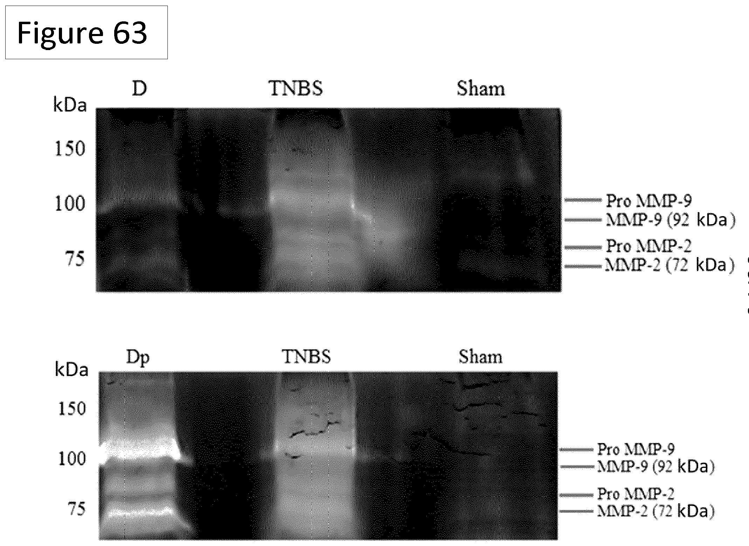

Given that the DQ-gelatin assay provides evidence for total gelatinolytic activity in the colon tissue (i.e. MMP-9 + MMP-2) with no possible distinction between the two gelatinases, we further tested their specificity through substrate zymography, where MMP-9 and MMP-2, in their native and zimogenic forms, can be readily resolved by electrophoresis. Figure 63 shows an example of a zymographic profile of the protein extracts obtained from the colonic tissues in the different experimental groups. White bands show the gelatinolytic activity of the specific bands.

Figure 63 shows the effect of deflamin administration on the colon tissue gelatinase activities of MMP-2 and MMP-9 from colitis-induced mice. Representative image of the zymographic profiles of MMP-9 and MMP-2 activities of the colons. Protein extracts of the colon were loaded on 12.5% (w/v acrylamide) polyacrylamide gels co-polymerized with 1% (w/v) gelatin. TNBS group (n = 10); Sham group (n = 6); D = colon from animals treated with deflamin in curative treatments (15 mg/kg, n = 9, p.o.) and Dp = colon from animals treated with deflamin in preventive treatments (15 mg/kg, n = 9, p.o.).

The zymographic profiles show how TNBS increased not only MMP-9 activity but also MMP-2 activity, both in the native and in the zymogenic forms of the enzymes, when compared to controls where there was low activity of the active forms of MMP-2 and MMP-9. However, in deflamin treatments there was an evident reduction in MMP-9 and MMP-2 activities (native and zymogenic forms), when compared to the TNBS group.

However, the specificity and intensity of the MMP inhibition differed between treatments. Whilst in the curative treatments both MMP-2 and MMP-9 were reduced in a similar fashion (and for both enzyme forms), this trend was not the same in the preventive approach, where MMP-9 was clearly and visibly more inhibited than pro-MMP-9, whereas pro-MMP-2 was inhibited, but MMP-2 was not (Figure 63).

### Deflamin is Also Bioactive on Other Models of Acute Inflammation

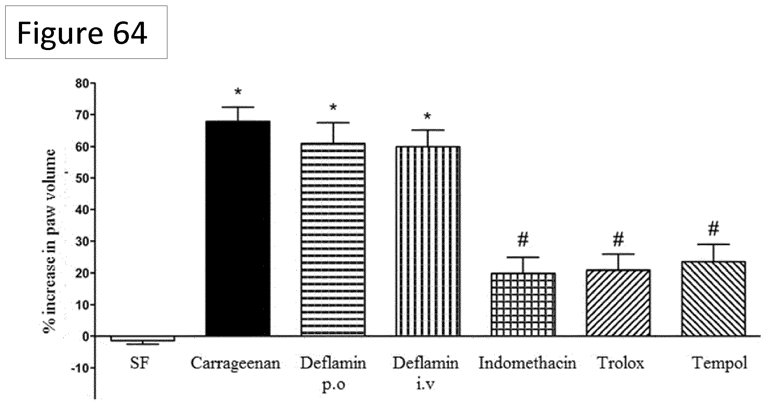

Figure 64 shows the effect of deflamin administration on the rat paw oedema development elicited by carrageenan 6 h after oedema induction. Effect of a single administration of deflamin extract (15 mg/kg; n = 5; p.o.) in comparison with the effect of a single administration of carrageenan (1 mL/kg; n = 6; i.p.), indomethacin (10 mg/kg; p.o.; n = 6), tempol (30 mg/kg; n = 8; p.o.), or Trolox (30 mg/kg; n = 8; p.o.). SF: subplantar injection of 0.1 mL sterile saline and administered with saline (1 mL/kg, i.p., n = 6). The data are presented as means with their standard errors. *p < 0.001 versus SF group; #p < 0.001 versus carrageenan group.

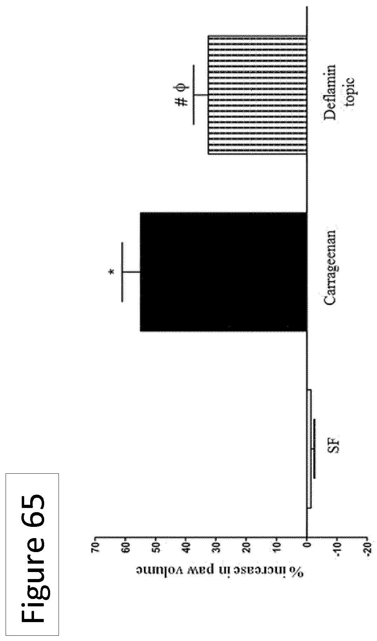

Figure 65 shows the effect of topic deflamin administration on paw oedema in rats 6 h after oedema induction by carrageenan. Effect of a single administration of deflamin extract (15 mg/kg; n = 3; p.o.) in comparison with the effect of a single administration of carrageenan (1 mL/kg; n = 6; i.p.). SF: subplantar injection of 0.1 mL sterile saline and administered with saline (1 mL/kg, i.p., n = 6). The data are presented as means with their standard errors. *p < 0.001 versus SF group; #p < 0.05 versus carrageenan group; and φp < 0.01 versus SF group. Deflamin was tested to see if it is able to reduce inflammation in another animal model of inflammation: the carrageenan-induced paw oedema, also using different administrations: via p.o., i.p. or applied topically. Figure 64 shows the effect of deflamin on the paw oedema under conditions of p.o. and i.p. administrations, and Figure 65 shows the result for the topic administration, represented in % of increase in paw volume. For the carrageenan group, a very significant increase (P<0.001) in the % of paw volume was observed in both Figures, whereas treatment with anti-inflammatory controls reduced it significantly (P<0.05).

Results show that deflamin treatments reduced the percent increase in paw volume after carrageenan administration, but were only significant (P<0.05) for the topic applications (Figure 65), whereas in the i.p. and p.o. administrations (Figure 58) the effect was not significantly different from the carrageenan group (P>0.05).

### Deflamin Digestibility

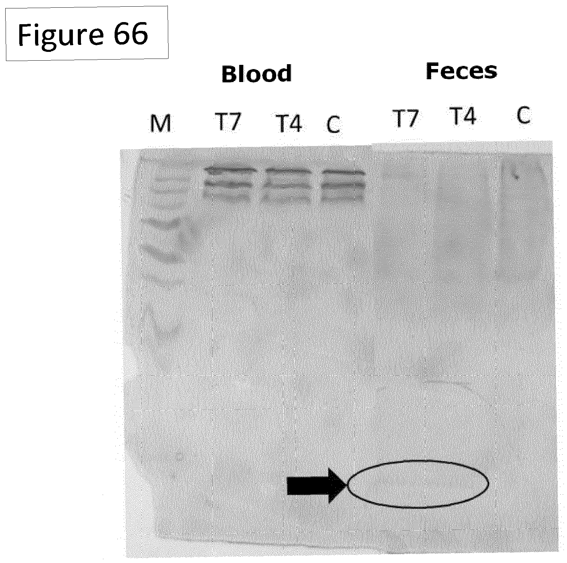

Figure 66 shows reverse zymography of mouse blood and feces (in and out fractions, respectively). Deflamin was administered orally to rats during 0 (control), 4 (T4) and 7 days (T7). M: molecular mass markers.

Reverse zymography (Figure 66) shows the presence of a proteinase inhibitor (i.e. deflamin) in the mouse feces, but not in the mouse blood. This result is corroborated by the data presented in Figure 34. Although not clearly visible in Figure 66 this activity was found to be more intense in the animals which ingested deflamin during a longer period of time (i.e. 7 days).

These results suggest thatdeflamin survives the mouse digestive process because it maintains its biological activity after passing through the colon. No inhibitor activity was detected in the mouse blood, suggesting that deflamin may not enter the blood stream. Alternatively, the methodology followed may have been not sensitive enough to warrant its detection in the blood samples.

Ingestion of deflamin by mice led to its detection in the out fraction (i.e. colon). Weak evidence suggests that deflamin administered orally may not enter the blood stream. This could be interpreted to mean that after exerting its bioactivity, deflamin is excreted in the mouse feces. If the absence of deflamin in the blood stream of animals feed with it is confirmed, this may be highly advantageous in the sense of avoiding the well known secondary lateral effects that result from an unspecific inhibition of body MMPs in general, widely described for synthetic MMPIs. It is important to highlight the perspectives opened by the observation that deflamin seems to maintain its biological anti-gelatinolytic activity even after passing through the mouse whole digestive tract, something which is intimately associated with its potential application in human health and nutrition.

### Effect of Seed Cooking on L. albus Capacity to Inhibit HT29 Cell Migration

Many heat-labile bioactive compounds have been described in seeds. However, most seeds are ingested after cooking for a variety of reasons, including for example the presence of toxic, heat-labile peptides, proteins (e.g. lectins, enzyme inhibitors, and enzymes which release toxic compounds such as the glycosidases present in cyanogenic plants) and other compounds. Under these conditions, bioactive compounds present in functional seeds often need to resist cooking in addition to survive the digestive process.

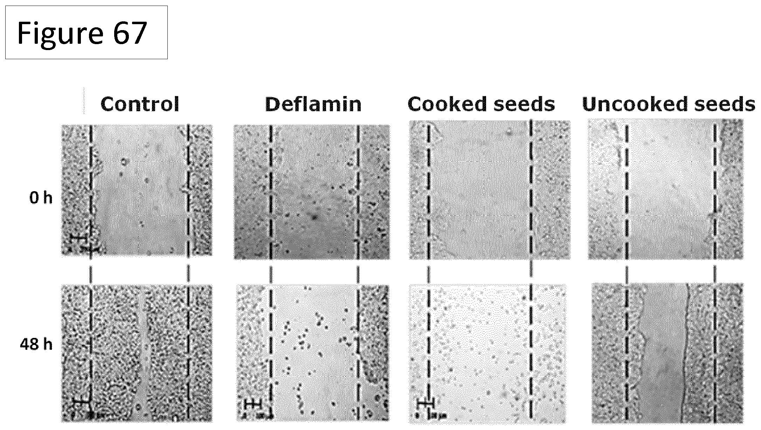

Figure 67 shows HT29 cell anti-migration effect of deflamin. Representative images of wound closure assays in HT29 cells after treatments with deflamin or with soluble protein extracts from cooked seeds and uncooked seeds. Cells were grown until reaching 80% confluence and wounding was made by scratching the cells with a pipette tip (0 h). Cells were then exposed to 100 µg protein extract/mL and wound healing was monitored after 48 h.

Figure 67 analyses the effect of seed cooking on *L. albus* seeds ability to inhibit HT29 cell migration by the wound healing assay. Isolated deflamin totally inhibited the migration of HT29 cells at a protein concentration of 100 µg/mL. Actually, this deflamin concentration not only blocked cell migration, but additionally detached cells from the solid support. The extract of total seed protein also inhibited cell migration, an effect which was found to be particularly intense in the case of cooked seeds. This apparently surprising result may be explained by the concentration effect on deflamin and other heat-resistant proteins exerted by boiling. In other words, the amount of deflamin present in 100 µg of seed total soluble protein is higher in the cooked seeds than in the uncooked simply because many seed proteins were denatured (and therefore made insoluble) by the heat treatment.

It is possible to conclude that deflamin exhibits a potent capacity to inhibit cell invasion and MMP-9 and MMP-2 activities at low concentrations, without affecting in a significant way colon cell growth. Therefore, deflamin shows high potential as an anti-inflammatory and anti-tumoural agent in CRC. Its high resistance to the digestive process, to boiling, and to low pH values make deflamin an excellent candidate to be used as a nutraceutical in human health and nutrition. Its bioactivity is equally potent in the cooked total seed extract, an observation which makes lupins an excellent functional food, to be implemented throughout the world as another great benefice of the well established Mediterranean diet- Included on the November 16, 2010 by the UNESCO's Intergovernmental Committee in the Representative List of Intangible Cultural Heritage of Humanity.

### The Amino Acid Residue Sequence of Lunasin and Those of Deflamin Precursors

The data presented above indicate the presence of both β-conglutin and δ-conglutin-2 large chain fragments in deflamin preparations. Therefore, β-conglutin and δ-conglutin-2 large chain may be considered as deflamin precursors.

The NCBI BLAST (Basic Local Alignment Search Tool) tool available at http://blast.ncbi.nlm.nih.gov/Blastcgi, was used to check possible similarities in amino acid residue sequences between the soybean 43-amino acid residue lunasin and the proteins whose polypeptides were identified as components of deflamin, namely fragments of β-conglutin and the heavy chain of δ-conglutin.

When appropriate, the ExPASy BLAST tool available at http://web.expasy.org/tmp/1week/blastf29720.html, was also used.

Based on the above observation that deflamin apparently comprises a mixture of β-conglutin and δ-conglutin <10 kDa fragments, the query and subject sequences utilized in the BLAST analyses were as shown in lunasin (*Glycine max*) (SEQ ID NO: 191), β-conglutin (*Lupinus albus*) (SEQ ID NO: 192), δ-conglutin-2 large chain (*Lupinus angustifolius*)(SEQ ID NO: 193) and δ-conglutin (*Lupinus albus*)(SEQ ID NO: 194).

When compared to all entries present in protein databases, lunasin (*Glycine max*) amino acid residue sequence exhibits a very strong homology (over 95%) to G. max 2S albumin. This was clearly illustrated by appropriate BLAST analyses.

An amino acid residue sequence comparison of lunasin with those of deflamin precursors was also performed. No amino acid residue sequence homology was found between Lunasin and conglutin beta precursor from *L. albus* and Lunasin vs conglutin delta-2 large chain. Considerable homology was encountered between the amino acid sequence of lunasin and the fragment corresponding to the light chain of δ-conglutin from *L. albus.*

Along the 43 amino acid residue lunasin, there is a stretch of 25 amino acids which exhibits 48% homology (12 residues out of 25) to the region of *Lupinus albus* δ-conglutin comprising residues 21 to 45.

It is possible to conclude that the amino acid sequence of lunasin shows no homology to those of deflamin precursors, namely β-conglutin and δ-conglutin-2 large chain.

Seq ID No: 195 confirms that lunasin contains a 25 long region whose amino acid sequence exhibits a 48% homology to a corresponding region within *Lupinus albus* δ-conglutin small chain, but not to *Lupinus angustifolius* conglutin delta-2 large chain. However, this observation is contradicted by the results reported by Herrera (2009).

Seq ID NO: 195 shows Sequence matches between *Lupinus albus* δ-conglutin (Accession number Q333K7; black, orange and red) and two polypeptides: *Glycine max* lunasin (Accession number AF005030; green) and *Lupinus angustifolius* conglutin delta-2 large chain (Accession number P09931; blue).

In addition to a different amino acid residue sequence, one other major difference between lunasin and deflamin concerns their susceptibility to digestion proteolysis. Whilst deflamin resists the digestive process, lunasin does not (Cruz-Huerta *et al*., 2015). Furthermore, unlike deflamin, the extraction and purification of lunasin are inappropriate to undergo scaling-up processes, rendering this bioactive peptide unsuitable to be mass produced.

### Deflamin from Seeds Other Than L. albus - I

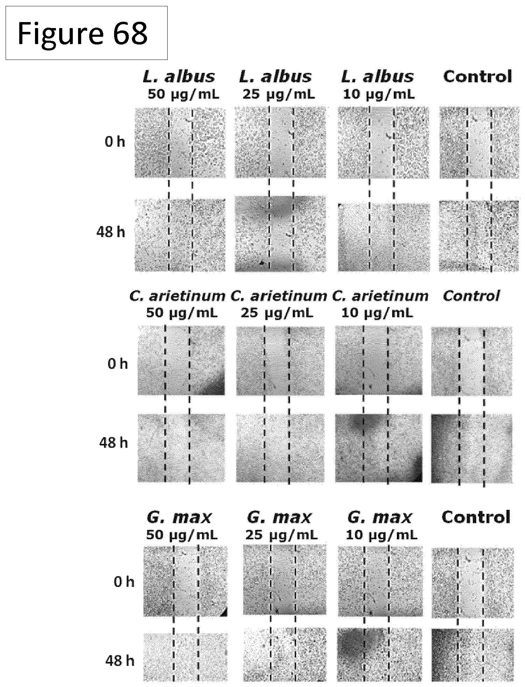

Deflamin was found to be present in seeds other than those of *Lupinus.* Figure 68 shows the inhibitory effect exerted upon HT29 cell migration by several concentrations of total soluble protein extracts from *L. albus,* C. *arietinum* and *G*. *max.* The results indicate the presence of cell migration inhibitory activity in the seeds analysed.

Figure 68 shows representative images of HT29 cell migration as assessed by the wound healing assay. Cells were grown until reaching 80% confluence and the monolayer was scratched with a pipette tip (0 h). Cell migration was determined after a 48 h exposure of HT29 cells to buffer (control), and to several concentrations of the total soluble proteins were extracted from the seeds of *L. albus,* C. *arietinum* and *G*. *max.*

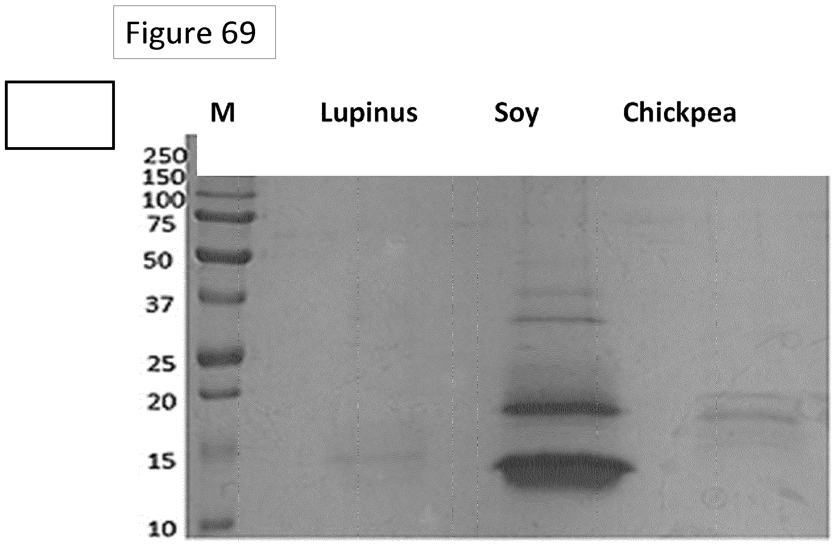

Deflamin was subsequently purified and isolated from *L. albus,* C. *arietinum* and *G*. *max* seeds following the procedure described in Figure 4 for *L. albus* deflamin. The polypeptide profiles of deflamin from *L. albus* (termed deflamin La), *G*. *max* (termed deflamin Gm) and C. *arietinum* (termed deflamin Ca) are shown in Figure 69.

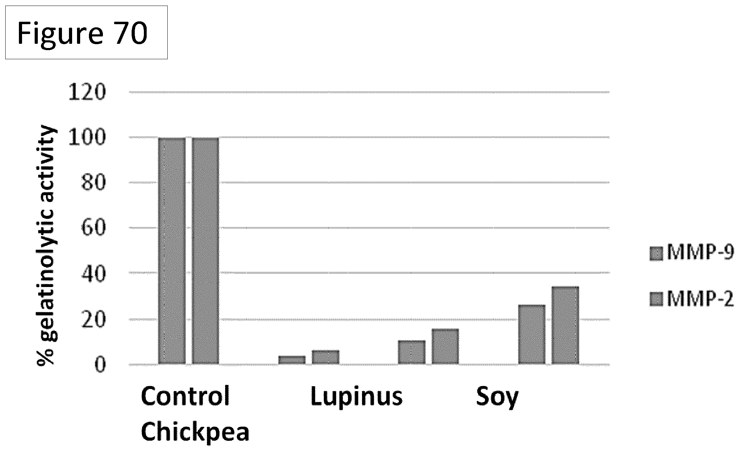

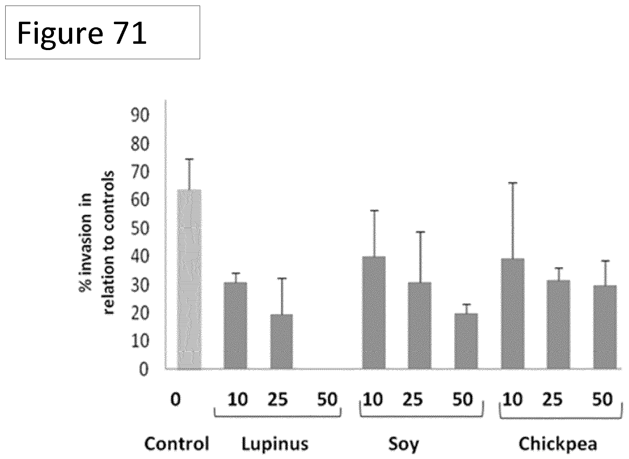

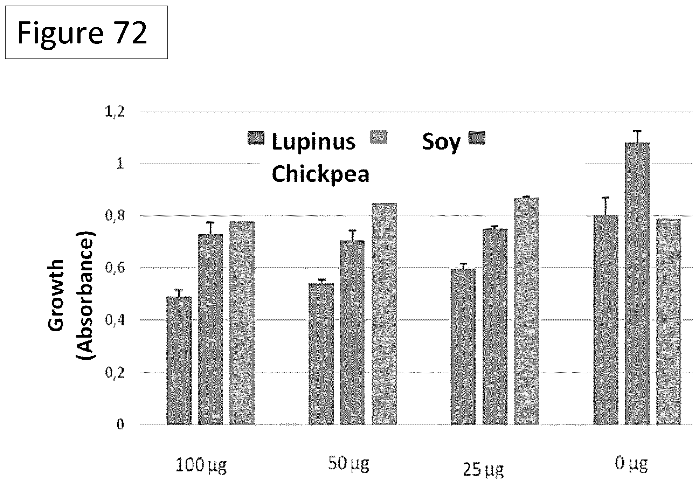

The results presented in Figures 70, 71 and 72 compares the anti-gelatinolytic activity measured *in vitro* by the DQ gelatin assay, the inhibitory activity upon HT29 cell migration as determined by the wound healing assay, and the HT29 cell growth assayed by the MMT method of deflamin isolated from *L. albus* (deflamin La), *G*. *max* (deflamin Gm) and C. *arietinum* (deflamin Ca), respectively.

The data presented in Figures 70 to 72 reveal that deflamin from soybean and chickpea also inhibit *in vitro* MMP-9 and MMP-2 gelatinases as well as HT29 cell migration, but do not affect to any significant extent HT29 cell growth, paralleling the results obtained for deflamin from lupins. However, deflamin from lupins seems to be more potent than that from soybean or chickpea. This last observation may not be relevant in what the above mentioned Mediterranean diet is concerned, since people usually ingest larger amounts of chickpea or soybean per meal than lupins.

### Brief Discussion of Selected Topics of the Results Presented

Deflamin is a novel digestion-resistant gelatinase inhibitor which reduces colitis injury through oral supplementation. MMP-9 inhibitors (MMPIs) are mostly regarded as anti-angiogenic agents for primary tumors and metastasis deterrents, but they have also been demonstrated to effectively inhibit pre-cancer states such as colitis and other inflammatory bowel diseases. For over 30 years now, MMPs have been considered by researchers across the world as attractive therapeutic targets, for cancer as well as inflammation. As a result, a myriad of MMPIs has already been synthesized, some of which have been used as potential therapeutic agents (Bourguet *et al.*, 2012). However, only a few small MMPIs entered the clinical trial stage, most of which terminated prematurely either due to lack of benefits or to strong adverse side effects (Wang *et al.*, 2012). Ideally, for a specific MMP-9 inhibitor to be successfully used in inflammatory bowel diseases (IBD) treatments as a dietary supplement, it should be colon-available, rather than serum-bioavailable, resistant to the digestive process and also non-toxic for colon cells. Results presented here clearly show that deflamin survives the digestion process and is able to attenuate the lesions provoked by TNBS-induced colitis, leading to a reduction in several functional and histological markers of colon inflammation, namely: attenuation of colon length decrease, reduction of the extent of visible injury (ulcer formation), decrease in diarrhea severity, reduced mortality rate, reduction of mucosal hemorrhage and reduction of general histological features of colon inflammation. Moreover, this effect was evident in the p.o. treatments, as well as in the i.p. treatments, corroborating its potential use in a dietary approach. In fact, the overall results obtained in the oral administrations suggest that deflamin is not only resistant to digestion, but it was more efficient than i.p. treatment in reducing the colitis injuries possibly by acting more effectively in situ. Interestingly, a preventive approach, with a more prolonged dietary administration of deflamin was not significantly different from the curative approach, were deflamin was administered only 3 h after TNBS induction. This suggests it can act as an inflammatory deterrent in an effective and fast manner, suggesting a potential use as a nutraceutical in both acute situations, as well as in chronic inflammation.

Oral administration of deflamin reduces the expression of inflammatory markers involved in the inflammatory signaling cascade.

The histological analysis and the expression of some important markers of inflammation also corroborated the anti-inflammatory effects of deflamin. Our immunostaining assays performed in the colons from animals of the experimental colitis study showed that induction of colon inflammation led to an increased expression of COX-2 compared to sham animals, which is in accordance to clinical and epidemiologic studies which demonstrated the important role of COX-2 and prostaglandins in the progression of intestinal inflammation in patients with IBD (Ogasawara *et al.*, 2007; Chen *et al.*, 2014). Administration of deflamin led to a reduced staining for COX-2, indicating that it impaired the expression of COX-2 in the injured intestinal tissue.

Also, specifically in the intestine, the up-regulation of the production of nitric oxide was observed. This compound is reported to be produced and released locally in much greater quantities in the inflamed gut than in the non-inflamed gut, being suggested as a novel clinical biomarker for diagnosis and monitoring of IBD patients (Lee *et al.*, 2013). Similarly to COX-2, immunostaining assays in the experimental colitis study showed that there was in fact an increased expression of iNOS in animals subjected to colitis induction. Again, deflamin administration, particularly via p.o., was able to reduce iNOS expression and therefore contribute to impairment of the inflammatory process in the colon.

### Deflamin Inhibits MMP-9 and MMP-2 in vivo

It has been presently demonstrated that deflamin inhibits MMP-9 and MMP-2 in colon cells in *in vitro* assays, and that it is resistant to heat and acid denaturation, making it a good candidate for the treatment of IBDs and colon cancer. However, *in vivo* tests were required to further determine its effectiveness after digestion. In this respect, the overall physiological and morphological results were able to corroborate that deflamin can indeed inhibit the colitis-induced rise in MMP-9 and MMP-2 activities observed in the TNBS group, levelling them to an activity intensities closer to those observed in controls.

Interestingly, although there were no morphological and functional differences observed between preventive and curative treatments, there were significant differences between the specific inhibitions in both enzymes in the zymographic assays. Whilst in the curative treatments, deflamin seems to act directly upon the two gelatinases, which were strongly induced by TNBS, in the preventive treatments a specific inhibition of the active form of MMP-9 and the pro-MMP-2 was observed, but not of the pro-MMP-9, nor the active form of MMP-2. MMPs are usually synthesized as zymogens (pro-MMPs), with their catalytic activity blocked by a cysteine switch and are only activated by its removal, through limited proteolysis. In the zymography assays, the pro-gelatinases also become active because they are denaturated by the SDS, thus exposing the catalytic site (hence the slightly higher mass of the pro-enzymes in the zymographic gels because they still maintain the short amino acid sequence of the cysteine switch). The fact that only the active form of MMP-9 is inhibited by deflamin suggests that it shows a certain degree of specificity towards this form, perhaps to the catalytic site, only exposed in the active form.

On the other hand, pro-MMP2 seems to be inhibited but not MMP2. Because MMP-2 is one of the proteases that activate MMP-9, it seems plausible that in a more prolonged exposure to deflamin, a high inhibition of MMP-9 would induce, through feedback, a higher activation of MMP-2 to activate MMP-9.

Although a more prolonged exposure to deflamin seems to suggest more profound effects in the synthesis and activation of the gelatinases, results suggest that its administration in a curative approach is just as effective in reducing colitis injuries as the preventive mode of administration. The higher specificity towards MMP-9 is nonetheless important because it insures low side-effects, as opposed to the majority of broad-range MMPIs used in clinical trials.

### Deflamin Also Reduces Inflammation in Other Models

In order to elucidate its range as an anti-inflammatory agent, we further tested if deflamin was able to reduce inflammation in the paw oedema. Although there was a reduction in the % of the paw's volume in both i.p. and p.o. administrations, they were not statistically significant, suggesting that the absorption and distribution of deflamin through the blood flow is limited, in both administrations. However, the significant efficacy observed in the topic administrations of deflamin corroborate that its effect is higher when applied *in situ.* It also suggests that deflamin can be absorbed through the skin. Considering the relation between MMP-9 and skin cancer diseases (Philips *et al.*, 2011), our results open novel possibilities for deflamin clinical applications.

### Conclusion -1

As a potent inhibitor of the matrix metalloproteinases MMP-9 and MMP-2 and exhibiting powerful anti-inflammatory activities, deflamin represents a novel type of MMPI which is edible, proteinaceous in nature, survives the digestion process and which may be used as a functional food in the prevention/treatment of inflammation, as well as of any diseases derived from them. Being effective in oral, intravenously or topic applications, deflamin may prove useful as a functional foods in the prevention or treatment of a very wide array of diseases.

*L. albus* deflamin is a mixture of β-conglutin and δ-conglutin large chain fragments.

The presence of fragments of β-conglutin and δ-conglutin in deflamin is rather interesting. β-Conglutin is a trimeric protein devoid of disulphide bridges in which the monomers consist of a very large number of polypeptides, glycosylated or not, ranging from 16 to over 70 kDa, but a large number of proteolytic processing sites give rise to the abundance of 7S protein subunits observed. Its complete degradation post-germination strongly supports the storage function of β-conglutin. Interestingly, another fragment of this protein is known for its potent bioactivities against fungi: Blad, an abundant transient β-conglutin derived polypeptide chain of 20 kDa displaying lectin like activity. Being highly reactive and with the presence of a bioactive cupine domain, it is possible that there are other fragments of β-conglutin with specific uncharted activities yet to be discovered. Previous works revealed however that deflamin has neither antifungal nor bactericide activity (results not shown), and the sequences of the deflamin fragments do not match that of Blad.

δ-Conglutin belongs to the 2S sulphur-rich albumin family which might also have specific unknown bioactivities in lupine. *Lupinus* seeds 2S albumin, also termed δ-conglutin, is a monomeric protein which comprises two small polypeptide chains linked by two interchain disulfide bonds: a smaller polypeptide chain, which consists of 37 amino acid residues resulting in a molecular mass of 4.4 kDa, and a larger polypeptide chain containing 75 amino acid residues with a molecular mass of 8.8 kDa. This later, larger polypeptide chain is somewhat similar to some of the polypeptide profiles obtained for deflamin, particularly in peak 3 (see Figures 44 to 46). The larger polypeptide chain contains two intrachain disulfide bridges and one free sulfhydryl group. This could tentatively explain the slight difference in apparent molecular mass detected between R- and NR-SDS-PAGE of deflamin (see Figure 43). This protein presents specific inherent unique features among the proteins from *L. albus*: besides its high cystein content, it exhibits a low absorbance at 280 nm.

As far as the physiological role of δ-conglutin is concerned, a storage function has been proposed for this class of proteins. However, structural similarity with the plant cereal inhibitor family, which includes bi-functional trypsin/amylase inhibitors, may suggest a defence function for this protein in addition to its storage role and might corroborate its role as an MMPI. The presence of free sulphydryl groups in δ-conglutin could be related to a high degree of affinity towards the Zn²⁺ active site in MMPs, and could explain its mode of inhibition. Indeed, one way to isolate these conglutins is through Zn precipitation. Furthermore, its presence in *L. albus* seeds was assessed to be around 3 to 4% of the seed weight, which is consistent to the yields obtained above. Also, the *Lupinus* seed 2S albumin is typically present in both the albumin and the globulin fractions, thus explaining the results obtained previously for the MMP inhibitory activity in the two protein fractions (Lima *et al.*, 2016).

The HPLC peaks 1 and 4 of deflamin (Figures 44-46) were only composed by β-conglutin fragments and still presented MMPI activities, albeit at lower levels. Therefore, the highest activity seems may be attributed to a specific mixture comprising fragments of both proteins, β- and δ-conglutins, and not to β-conglutin exclusively. The fact that only the large polypeptide chain of δ-conglutin was found to be present in deflamin might suggest that its three sulphydryl groups could be free to interact with MMPs or with β-conglutin fragments (this could explain the presence of a group of apparently three minor higher molecular mass bands which comprise deflamin) and that this complex holds the highest activity. Alternatively, δ-conglutin smaller polypeptide chain may be present in deflamin.

### Conclusion - 2

In the last decade a substantial amount of research has turned towards the discovery of novel plant foods containing MMPIs, but few, if any present the potential of deflamin, as it is easy to isolate and displays high MMP-9 inhibitory activities. In a preferred embodiment, deflamin has been characterised as a complex mixture of soluble fragments from two specific protein precursors: δ- and β-conglutins. Overall, this polypeptide mixture was shown to be highly soluble in water; its bioactivities resist to boiling, to low pH values and possibly to digestive proteases; it strongly inhibits matrix metalloproteinase (MMP)-9 and/or MMP-2, i.e. it is an MMP inhibitor (MMPI) at low concentrations and in a dose-dependent manner, and it reduces the invasion capacity of the human colon adenocarcinoma cell line HT29 without exerting cytotoxicity. These data was strongly supported by an array of pre-clinical performed with animal models. These features make deflamin a novel type of MMPI that can be used as an ingredient of functional foods in the prevention/treatment of tumourigenesis and cell invasion, as well as of any disease derived from them. As a potent inhibitor of the matrix metalloproteinases MMP-9 and MMP-2, deflamin may prove useful in functional foods in the prevention and treatment of a very wide array of diseases related to MMP-9 activity. Its efficacy when administered orally and its capacity to survive the digestive process suggest that it may act efficiently in the colon, without exerting the deleterious side-effects which characterize the synthetic MMPIs, making deflamin an excellent candidate to be used in the prevention and treatment of colorectal cancer.

### Conclusion - 3

Deflamin exhibits:
- Deflamin exhibits a potent gelatinolytic activity in a dose-dependent manner;
- Deflamin exhibits a potent inhibition of colon cancer cell invasion in a dose-dependent manner;
- High deflamin concentrations detach the colon cancer cells from the solid surface;
- Deflamin does not apparently induce significant cytotoxic effects on colon cancer cells even at a 100 µg.mL⁻¹ concentration;
- Deflamin does not induce a significant reduction in cell growth nor a reduction in the number of living cells.

In one or more further embodiments, deflamin may actually be produced or improved during its own purification and isolation as a result of the *in vitro* harsh treatments imposed on lupin seed storage proteins, which disassemble oligomeric structures, cleave polypeptides by limited proteolysis and remove most unfolded polypeptides which are no longer water soluble. Other polypeptides are released and may become a part of deflamin. In other words, it is possible that deflamin, apparently composed of a mixture of polypeptides which are fragments derived from different protein precursors, is formed *in vitro* after the extraction of the reserve proteins and their partial denaturation / proteolysis.

### The Presence of Deflamin in Seeds from Other Species - II

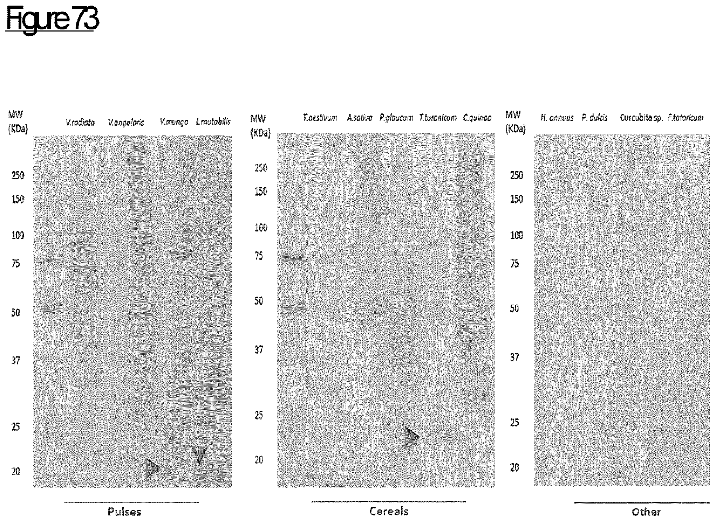

For the identification of the presence of deflamin in the seeds from other species, a reverse zymogaphy of the soluble proteins from different edible seeds was performed (Figure 73). Figure 73 shows representative images of reverse zymography performed on 12.5% (w/v) acrylamide SDS-PAGE gel with gelatin and 1 mL of HT-29 medium containing MMP-9 and MMP-2. Each well was loaded with 50 µg protein of the total extracts from the different seed species. Leguminosae: V.a - *Vigna angularis* (*azuki* bean); V.r - *Vigna radiata* (mung bean); V.m - *Vigna mungo* (*urad* bean); L.m - *Lupinus mutabilis* (Andes' lupine). Cereals: T.a - *Triticum aestivum* (common wheat); A.s - *Avena sativa* (oat); P.g. - *Pennisetum glaucum* (millet); T.t - T. *turgidum var. turanicum* (kamut wheat); Other dicotyledons: C.q - *Chenopodium quinoa* (quinoa), H.a - *Helianthus annus* (sunflower); P.d - *Prunus dulcis* (almond); C - *Curcubita* sp. (pumpkin); F.t - *Fagopyrum tataricum* (buckwheat); S.h - *Salvia hispanica* (chia). Arrows indicate the presence of deflamin.

Among the other species tested and in addition to those initially evaluated for deflamin (i.e. L. *albus,* C. *arietinum* and G. *max*), only the seeds from *Vigna mungo* (urad bean), *Lupinus mutabilis* and *Triticum turanicum* showed bands similar to deflamin. Hence, other species of Leguminosae, particularly of the genus *Lupinus*, and also of the genus *Triticum* were further analyzed.

### Presence of Deflamin in the Seeds of Other Species of the Genus Lupinus

Seeds from the following species of *Lupinus* were analyzed for the presence of deflamin:
- *L. albus*
- *L. mutabilis*
- *L. hispanicus*
- *L. nootkatensis*
- *L. angustifolius*
- *L. luteus*

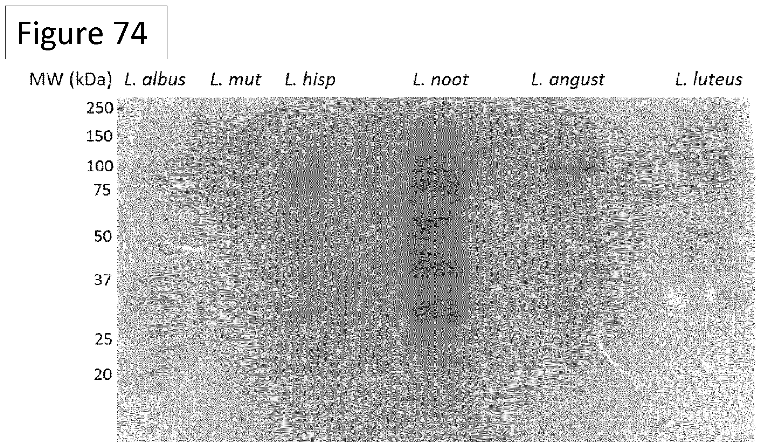

The reverse zymography gel is shown in Figure 74 below. Although difficult to see in Figure H, all *Lupinus* species analysed were shown to contain deflamin.

Figure 74 shows reverse zymography performed on 12.5% polyacrylamide gel with gelatin and 1mL of HT-29 medium containing MMP-9 and MMP-2. Each well was loaded with 50 µg of the total extracts of the different seed species: *L. albus*; *L.mutabilis*; *L. hispanicus*; *L. nootkatensis*; *L. angustifolius*; e *L. luteus.*

### Isolation of Deflamin from L. mutabilis Seeds

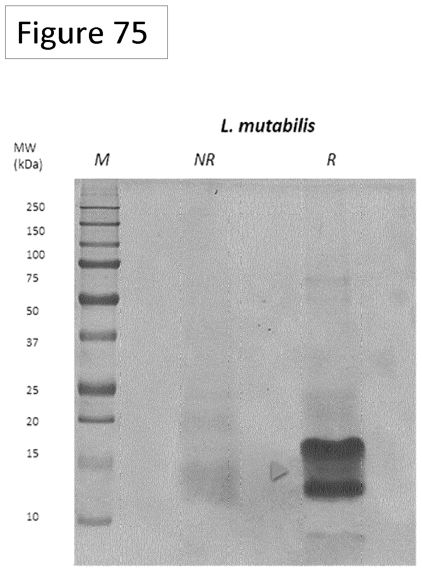

Figure 75 shows representative polypeptide profiles of the potential homologue of deflamin under reducing (R) and non-reducing (NR) conditions by SDS-PAGE in 12.5% (w/v) acrylamide gel, using the method of deflamin isolation. The wells were loaded with 100 µg of protein purified from *L. mutabilis.*

### The Presence of Deflamin in the Seeds from Other Legume Species

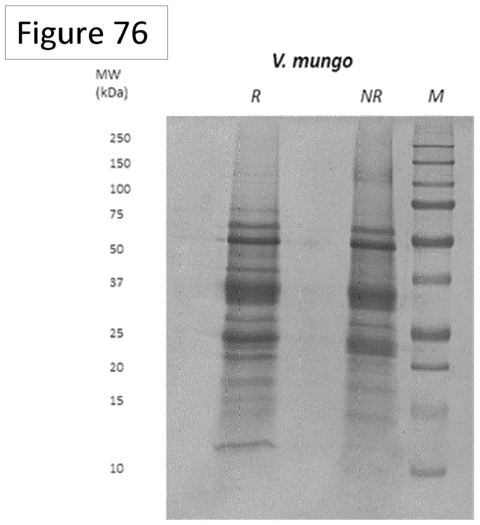

Purification of deflamin from the seeds of *Vigna mungo* (urad bean) originated a pattern of polypeptides rather different than those from *Lupinus* species (Figure 76).

Figure 76 shows representative polypeptide profiles of the potential homologue of deflamin under reducing (R) and non-reducing (NR) conditions by SDS-PAGE in 12.5% (w/v) acrylamide gel, using the method of deflamin isolation. The wells were loaded with 100 µg of protein purified from *Vigna mungo.*

### Isolation of Deflamin from the Seeds of Species from the Genus Triticum

The following Triticum species were analyzed for the presence of deflamin:
- *T. spelta* (spelt)
- *T. turgidum var. durum* (durum wheat)
- *T. aestivum* (common wheat)
- *T. turgidum var. turanicum* (kamut)

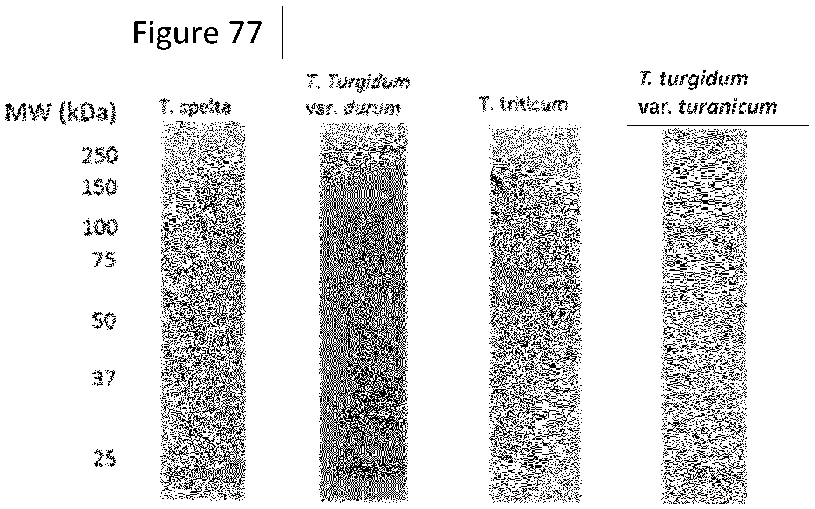

The reverse zymography gels are shown in Figure 77 below. Reverse zymography showed the presence of MMP-9 inhibitory bands in the species:
- *T. spelta*
- *T. turgidum var. durum*
- *Triticum turgidum var. turanicum**,
and most probably other ancient wheat species and varieties, but not, apparently, in *T*. *aestivum.*

* Khorasan wheat or Oriental wheat (*Triticum turgidum* ssp. *turanicum* also called *Triticum turanicum*), commercially known as kamut, is a tetraploid wheat species. Identifications sometimes seen as *T. polonicum* seem to be incorrect. Recent genetic evidence from DNA fingerprinting suggests that the variety may be derived from a natural hybrid between *T*. *durum* and *T. polonicum.*

Figure 77 shows reverse zymography performed on 12.5% (w/v) acrylamide gel with gelatin and 1 mL of HT-29 medium containing MMP-9 and MMP-2. Each well was loaded with 50 µg of protein in the total extracts from different seed species: *T. spelta* (spelt); T. *turgidum var. durum* (durum wheat); *T. aestivum* (common wheat); and *Triticum turgidum var. turanicum* (kamut).

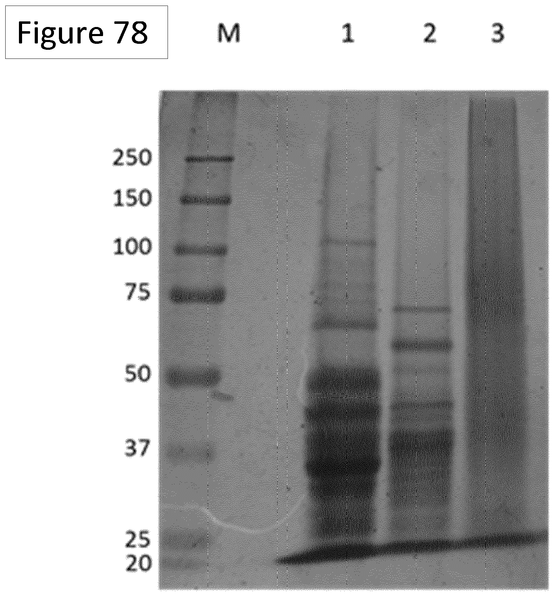

Isolation of deflamin from the seeds of these species (i.e., *T. spelta, T. turgidum var. durum,* and *Triticum turgidum var. turanicum*) was then carried out. The results are expressed in Figure 78 below.

Figure 78 shows representative polypeptide profiles of the potential homologue of deflamin in reducing (R) and non-reducing (NR) buffer by SDS-PAGE in 12.5% (w/v) acrylamide gel, using the

## Claims

1. A method of extraction of deflamin from suitable seeds, comprising:
- at least one step at high temperature of at least 80 degrees Celsius or boiling; and
- at least one step at low pH of pH 4 or lower;
- at least one step of contacting the extract with high ethanol concentrations of at least 30%, 40%, 70% or at least 90% v/v ethanol;
wherein preferably said method comprises the following steps in this order/sequence or a different order/sequence:
(a) boiling the intact seeds in water, followed by extraction in water or buffer, and fat removal, or
reducing the intact seeds to flour, extraction in water or buffer followed by fat removal and boiling, or
fat removal from the flour followed by extraction in water or buffer and boiling,
(b) exposing the soluble fraction to a sufficiently low pH value of pH 4.0 or lower to allow the precipitation of most of the remaining proteins/polypeptides,
(c) resuspending the precipitated fraction in 30% to 50% (v/v) ethanol, preferably about 40%
(v/v) ethanol, with the solution also optionally also containing 0.4 M NaCl, to obtain a supernatant that contains deflamin,
and optionally performing the following steps:
(d) making up the soluble fraction to 90% (v/v) ethanol to precipitate deflamin and optionally storing at - 5_{°}C to - 30_{°}C, preferably at - 20_{°}C, or
precipitating the deflamin by other means, such as freeze-drying,
(e) cleaning the precipitated deflamin from contaminants by repeating steps (c) and (d),
(f) dissolving the precipitated deflamin, for example, in water, and desalting.

2. A method of extraction of deflamin from suitable seeds dependent on claim 1, said method comprising the following steps in the same order/sequence or in a different order/sequence:
• providing a flour from said seeds;
• defatting said flour;
• boiling for a period the remaining sample from said defatting step;
• centrifuging said sample for a period;
• thereafter discarding a resulting pellet and further processing a resultant supernatant to precipitate polypeptides whilst lowering its pH;
• further centrifuging to obtain a further pellet; and discard said supernatant; and
• further processing said further pellet with ethanol and centrifuging to obtain a further pellet which is then discarded; the remaining supernatant comprising deflamin, wherein optionally:
- the method further comprises the step of adding ethanol to said remaining supernatant and storing for a period to allow precipitation of deflamin and further centrifuging to obtain a deflamin pellet, and/or
- the method further comprises the step of one or more further ethanol precipitations.

3. A method according to claim 2, wherein said seed are *Lupinus albus* seeds, *Cicer*aritinum or *Glycine max*, and/or wherein said seeds are cooked.

4. A deflamin polypeptide composition for use in a method of treatment of the human or animal body by therapy, wherein said therapy is preferably preventing or treating inflammation or cancer, wherein said deflamin polypeptide composition comprises deflamin as obtainable by the method of any one of claims 1 to 3.

5. A deflamin polypeptide composition for use according to claim 4 wherein said composition comprises a mixture of polypeptides obtainable from seed storage proteins by the method of any one of claims 1 to 3 which each comprise a sequence that is a portion of a conglutin protein,
- wherein said portion is at least 5, 10, 20, 30 or 50 amino acids long, and/or
- wherein said conglutin protein is optionally a conglutin beta or conglutin delta; and/or
- wherein said conglutin protein is optionally a conglutin beta 1, 2, 3, 4, 5, 6 or 7 or a conglutin delta 2 protein.

6. A deflamin composition for use according to claim 4 or 5 which comprises at least a mixture of up to 100 polypeptides which have a length of 10 to 200 amino acids.

7. A deflamin composition for use according to any one of the claims 4 to 6 which is obtained by extraction from plant material or by recombinant expression.

8. A deflamin composition for use according to any one of claims 4 to 7 wherein any of said polypeptides comprise the sequence of a plant seed protein or have at least 70% identity to a plant seed protein, wherein said plant is optionally of the genus *Lupinus*, *Ciceror Glycine*, and said protein is optionally a conglutin protein or a naturally occurring homologue of any conglutin.

9. A method of making deflamin comprising expressing the deflamin polypeptides from one or more nucleic acids encoding the polypeptides and purifying said polypeptides, wherein said expression is preferably in a cell, and wherein the deflamin polypeptides are obtainable by the method of any one of claims 1 to 3.

10. One or more nucleic acid vectors which together or individually express a deflamin composition for use in a method of treatment of the human or animal body by therapy, wherein said therapy is preferably preventing or treating inflammation or cancer, wherein said deflamin composition is optionally as defined in any one of claims 5 to 8, and wherein the deflamin as obtainable by the method of any one of claims 1 to 3.

11. A product comprising a multiplicity of different polypeptides as defined in any one of claims 5 to 8 which together form a deflamin composition for simultaneous, separate or sequential use in a method of treatment of the human or animal body by therapy, wherein said therapy is preferably preventing or treating inflammation or cancer, and wherein the deflamin is obtainable by the method of any one of claims 1 to 3.

12. A product comprising a multiplicity of nucleic acid vectors which together express a deflamin composition for simultaneous, separate or sequential use in a method of treatment of the human or animal body by therapy, wherein said therapy is preferably preventing or treating inflammation or cancer, and wherein the deflamin is obtainable by the method of any one of claims 1 to 3.

13. A composition which is:
(i) a deflamin composition which is obtainable by the method of any one of claims 1 to 3 and which is optionally as defined in any one of claims 5 to 8; or
(ii) a composition comprising one of more nucleic acids which individually or together encode a deflamin polypeptide composition which is obtainable by the method of any one of claims 1 to 3, wherein optionally
- said nucleic acids are expression vectors which can express a deflamin composition, preferably a viral vector, and/or
- said composition is in the form of a pharmaceutical composition optionally also comprising a pharmaceutically acceptable carrier or diluent.

## Patentansprüche

1. Verfahren zur Extraktion von Deflamin aus geeigneten Samen, umfassend:
- mindestens einen Schritt bei einer hohen Temperatur von mindestens 80 Grad Celsius oder zum Kochen; und
- mindestens einen Schritt bei einem niedrigen pH-Wert von 4 oder weniger;
- mindestens einen Schritt des Kontakts des Extrakts mit hohen Ethanolkonzentrationen von mindestens 30 %, 40 %, 70 % oder mindestens 90 % v/v Ethanol;
wobei das Verfahren vorzugsweise die folgenden Schritte in dieser Reihenfolge/Sequenz oder in einer anderen Reihenfolge/Sequenz umfasst:
(a) Kochen der intakten Samen in Wasser, gefolgt von einer Extraktion in Wasser oder Puffer und Fettentfernung oder Reduktion der intakten Samen zu Mehl, Extraktion in Wasser oder Puffer gefolgt von Fettentfernung und Kochen oder Fettentfernung aus dem Mehl gefolgt von einer Extraktion in Wasser oder Puffer und Kochen,
(b) Aussetzen der löslichen Fraktion einem ausreichend niedrigen pH-Wert von 4,0 oder weniger, um die Ausfällung der meisten der verbleibenden Proteine/Polypeptide zu ermöglichen,
(c) Resuspendieren der ausgefällten Fraktion in 30 % bis 50 % (v/v) Ethanol, vorzugsweise etwa 40 % (v/v) Ethanol, wobei die Lösung gegebenenfalls auch 0,4 M NaCl enthält, um einen Überstand zu erhalten, der Deflamin enthält, und gegebenenfalls Durchführung der folgenden Schritte:
(d) Auffüllen der löslichen Fraktion auf 90 % (v/v) Ethanol zur Ausfällung von Deflamin und gegebenenfalls Lagerung bei -5°C bis -30°C, vorzugsweise bei -20°C, oder
Ausfällen des Deflamins auf andere Weise, z. B. durch Gefriertrocknung,
(e) Reinigung des ausgefällten Deflamins von Verunreinigungen durch Wiederholung der Schritte (c) und (d),
(f) Auflösen des ausgefällten Deflamins, z. B. in Wasser, und Entsalzen.

2. Verfahren zur Extraktion von Deflamin aus geeigneten Samen nach Anspruch 1, wobei das Verfahren, das die folgenden Schritte in der gleichen Reihenfolge/Sequenz oder in einer anderen Reihenfolge/Sequenz umfasst:
• Bereitstellung von Mehl aus den Samen;
• Entfetten das besagte Mehl;
• Kochen der verbleibenden Probe aus dem Entfettungsschritt für einen Zeitraum;
• Zentrifugieren der Probe für einen Zeitraum;
• danach Verwerfen eines entstandenen Pellets und Weiterverarbeitung des verbleibenden Überstand, um Polypeptide auszufällen und gleichzeitig den pH-Wert zu senken;
• weiteres Zentrifugieren, um ein weiteres Pellet zu erhalten; und Verwerfen des Überstandes; und
• Weiterverarbeitung des weiteren Pellets mit Ethanol und Zentrifugieren zur Gewinnung eines weiteres Pellet, das dann verworfen wird; der verbleibende Überstand enthält Deflamin, wobei gegebenenfalls:
- das Verfahren umfasst ferner den Schritt der Zugabe von Ethanol zu dem verbleibenden Überstand und die Lagerung für einen Zeitraum, um die Ausfällung von Deflamin zu ermöglichen, und das weitere Zentrifugieren, um ein Deflamin-Pellet zu erhalten, und/oder
- das Verfahren umfasst ferner den Schritt einer oder mehrerer weiterer Ethanolausfällungen.

3. Verfahren nach Anspruch 2, wobei es sich bei den Samen um Samen von *Lupinus albus*, *Ciceraritinum* oder *Glycine max* handelt und/oder wobei die Samen gekocht werden.

4. Deflamin-Polypeptidzusammensetzung zur Verwendung in einem Verfahren zur Behandlung des menschlichen oder tierischen Körpers durch Therapie, wobei die Therapie vorzugsweise der Vorbeugung oder Behandlung von Entzündungen oder Krebs dient, wobei die Deflamin-Polypeptidzusammensetzung umfasst Deflamin, wie erhältlich nach dem Verfahren gemäß einem der Ansprüche 1 bis 3.

5. Deflamin-Polypeptidzusammensetzung zur Verwendung nach Anspruch 4, wobei die Zusammensetzung ein Gemisch von Polypeptiden umfasst, die aus Samenspeicherproteinen durch das Verfahren nach einem der Ansprüche 1 bis 3 erhältlich sind und jeweils eine Sequenz umfassen, die ein Teil eines Conglutin-Proteins ist,
- wobei dieser Teil mindestens 5, 10, 20, 30 oder 50 Aminosäuren lang ist, und/oder
- wobei das Conglutin-Protein gegebenenfalls ein Conglutin Beta oder Conglutin Delta ist; und/oder
- wobei das Conglutin-Protein gegebenenfalls ein Conglutin beta 1, 2, 3, 4, 5, 6 oder 7 oder ein Conglutin-Delta-2-Protein ist.

6. Deflamin-Zusammensetzung zur Verwendung nach Anspruch 4 oder 5, die mindestens ein Gemisch aus bis zu 100 Polypeptiden mit einer Länge von 10 bis 200 Aminosäuren umfasst.

7. Deflamin-Zusammensetzung zur Verwendung nach einem der Ansprüche 4 bis 6, die durch Extraktion aus Pflanzenmaterial oder durch rekombinante Expression erhalten wird.

8. Deflamin-Zusammensetzung zur Verwendung nach einem der Ansprüche 4 bis 7, wobei jedes der Polypeptide die Sequenz eines Pflanzensamenproteins umfasst oder zu mindestens 70 % mit einem Pflanzensamenprotein identisch ist, wobei die Pflanze gegebenenfalls zur Gattung *Lupinus*, *Cicer* oder *Glycin* gehört und das Protein gegebenenfalls ein Conglutin-Protein oder ein natürlich vorkommendes Homolog eines beliebigen Conglutins ist.

9. Verfahren zur Herstellung von Deflamin, umfassend die Expression der Deflamin-Polypeptide aus einer oder mehreren Nukleinsäuren, die für die Polypeptide kodieren, und die Reinigung der Polypeptide, wobei die Expression vorzugsweise in einer Zelle erfolgt, und wobei die Deflamin-Polypeptide durch das Verfahren nach einem der Ansprüche 1 bis 3 erhältlich sind.

10. Ein oder mehrere Nukleinsäurevektoren, die zusammen oder einzeln eine Deflamin-Zusammensetzung exprimieren, zur Verwendung in einem Verfahren zur Behandlung des menschlichen oder tierischen Körpers durch Therapie, wobei die Therapie vorzugsweise der Vorbeugung oder Behandlung von Entzündungen oder Krebs dient, wobei die Deflamin-Zusammensetzung gegebenenfalls wie in einem der Ansprüche 5 bis 8 definiert ist, und wobei das Deflamin durch das Verfahren nach einem der Ansprüche 1 bis 3 erhältlich ist.

11. Produkt, umfassend eine Vielzahl verschiedener Polypeptide, wie in einem der Ansprüche 5 bis 8 definiert, die zusammen eine Deflamin-Zusammensetzung zur gleichzeitigen, getrennten oder aufeinanderfolgenden Verwendung in einem Verfahren zur Behandlung des menschlichen oder tierischen Körpers durch Therapie bilden, wobei die Therapie vorzugsweise der Vorbeugung oder Behandlung von Entzündungen oder Krebs dient, und wobei das Deflamin durch das Verfahren nach einem der Ansprüche 1 bis 3 erhältlich ist.

12. Produkt, umfassend eine Vielzahl von Nukleinsäurevektoren, die zusammen eine Deflamin-Zusammensetzung exprimieren, zur gleichzeitigen, getrennten oder aufeinanderfolgenden Verwendung in einem Verfahren zur Behandlung des menschlichen oder tierischen Körpers durch Therapie, wobei die Therapie vorzugsweise der Vorbeugung oder Behandlung von Entzündungen oder Krebs dient, und wobei das Deflamin durch das Verfahren nach einem der Ansprüche 1 bis 3 erhältlich ist.

13. Zusammensetzung, die ist:
(i) eine Deflamin-Zusammensetzung, die durch das Verfahren nach einem der Ansprüche 1 bis 3 erhältlich ist und die gegebenenfalls wie in einem der Ansprüche 5 bis 8 definiert ist; oder
(ii) eine Zusammensetzung, umfassend eine oder mehrere Nukleinsäuren, die einzeln oder zusammen für eine Deflamin-Polypeptidzusammensetzung kodieren, die durch das Verfahren nach einem der Ansprüche 1 bis 3 erhältlich ist, wobei gegebenenfalls
- die Nukleinsäuren Expressionsvektoren sind, die eine Deflamin-Zusammensetzung exprimieren können, vorzugsweise ein viraler Vektor, und/oder
- die Zusammensetzung in Form einer pharmazeutischen Zusammensetzung vorliegt, die gegebenenfalls auch einen pharmazeutisch akzeptablen Träger oder ein Verdünnungsmittel umfasst.

## Revendications

1. Méthode d'extraction de la déflamine à partir de graines appropriées, comprenant :
- au moins une étape à une température élevée d'au moins 80 degrés Celsius ou à l'ébullition ; et
- au moins une étape à un pH faible de 4 ou moins ;
- au moins une étape de mise en contact de l'extrait avec des concentrations élevées d'éthanol d'au moins 30 %, 40 %, 70 % ou au moins 90 % d'éthanol v/v ;
ou, de préférence, ladite méthode comprend les étapes suivantes dans cet ordre/cette séquence ou dans un ordre/une séquence différent(e) :
(a) ébullition des graines intactes dans de l'eau, suivie d'une extraction dans de l'eau ou un tampon, et d'un dégraissage ou
réduction des graines intactes en farine, extraction dans l'eau ou dans un tampon suivie d'un dégraissage et d'une ébullition, ou
dégraissage de la farine suivie d'une extraction dans l'eau ou dans un tampon et d'une ébullition,
(b) exposition de la fraction soluble à un pH suffisamment bas de 4,0 ou moins pour permettre la précipitation de la plupart des protéines/polypeptides restants,
(c) remise en suspension de la fraction précipitée dans 30 à 50 % (v/v) d'éthanol, de préférence environ 40 % (v/v) d'éthanol, la solution contenant aussi éventuellement 0,4 M NaCl, pour obtenir un surnageant qui contient de la déflamine, et effectuer éventuellement les étapes suivantes :
(d) porter la fraction soluble à 90 % (v/v) d'éthanol pour précipiter la déflamine, et éventuellement stocker entre -5°C et -30°C, de préférence à -20°C, ou
précipiter la déflamine par d'autres moyens, tels que la lyophilisation,
(e) nettoyer la déflamine précipitée des contaminants en répétant les étapes (c) et (d),
(f) dissolution de la déflamine précipitée, par exemple dans l'eau, et dessalage.

2. Méthode d'extraction de la déflamine à partir de graines appropriées selon la revendication 1, ladite méthode comprenant les étapes suivantes dans le même ordre/la même séquence ou dans un ordre/une séquence différent(e) :
• fournir une farine à partir desdites graines ;
• le dégraissage de ladite farine ;
• l'ébullition pendant un certain temps de l'échantillon restant de ladite étape de dégraissage ;
• la centrifugation dudit échantillon pendant un certain temps ;
• par la suite, le rejet d'une pastille résultante et le traitement supplémentaire d'un produit surnageant résultant pour précipiter les polypeptides tout en abaissant son pH ;
• centrifugation supplémentaire pour obtenir une pastille supplémentaire ; et le rejet dudit surnageant ; et
• traitement supplémentaire de ladite pastille supplémentaire avec de l'éthanol et centrifugation en vue de l'obtention d'une pastille supplémentaire qui est ensuite rejetée ; le surnageant restant comprenant de la déflamine, ou éventuellement :
- la méthode comprend en outre l'étape consistant à ajouter de l'éthanol au surnageant restant et à le stocker pendant un certain temps pour permettre la précipitation de la déflamine et centrifugation supplémentaire pour obtenir une pastille de déflamine, et/ou
- la méthode comprend en outre l'étape d'une ou plusieurs précipitations supplémentaires d'éthanol.

3. Méthode selon la revendication 2, dans lequel lesdites graines sont des graines de *Lupinus albus*, de *Ciceraritinum* ou de *Glycine max*, et/ou dans lequel lesdites graines sont cuites.

4. Composition polypeptidique de déflamine pour utilisation dans une méthode de traitement du corps humain ou animal par thérapie, dans laquelle ladite thérapie consiste de préférence à prévenir ou à traiter l'inflammation ou le cancer, dans laquelle ladite composition polypeptidique de déflamine comprend la déflamine telle qu'elle peut être obtenue par la méthode de l'une quelconque des revendications 1 à 3.

5. Composition polypeptidique de déflamine pour utilisation selon la revendication 4, dans laquelle ladite composition comprend un mélange de polypeptides pouvant être obtenus à partir de protéines de stockage de graines par la méthode de l'une quelconque des revendications 1 à 3, qui comprennent chacune une séquence qui est une partie d'une protéine de conglutine,
- dans laquelle ladite portion a une longueur d'au moins 5, 10, 20, 30 ou 50 acides aminés, et/ou
- dans laquelle ladite protéine de conglutine est éventuellement une conglutine bêta ou une conglutine delta ; et/ou
- dans laquelle ladite protéine de conglutine est éventuellement une conglutine bêta 1, 2, 3, 4, 5, 6 ou 7 ou une protéine conglutine delta 2.

6. Composition de déflamine pour utilisation selon la revendication 4 ou 5, qui comprend au moins un mélange de jusqu'à 100 polypeptides d'une longueur de 10 à 200 acides aminés.

7. Composition de déflamine pour utilisation selon l'une des revendications 4 à 6, qui est obtenue par extraction à partir de matériel végétal ou par expression recombinante.

8. Composition de déflamine pour utilisation selon l'une des revendications 4 à 7, dans laquelle l'un quelconque desdits polypeptides comprend la séquence d'une protéine de graine végétale ou présente une identité d'au moins 70 % avec une protéine de graine végétale, ladite plante étant éventuellement du genre *Lupinus*, *Cicer* ou *Glycine*, et ladite protéine étant éventuellement une protéine de conglutine ou un homologue naturel d'une conglutine quelconque.

9. Méthode de fabrication de la déflamine comprenant l'expression des polypeptides de déflamine à partir d'un ou de plusieurs acides nucléiques codant pour les polypeptides et la purification desdits polypeptides, dans laquelle l'expression se fait de préférence dans une cellule, et dans laquelle les polypeptides de déflamine peuvent être obtenus par la méthode de l'une quelconque des revendications 1 à 3.

10. Un ou plusieurs vecteurs d'acide nucléique qui expriment ensemble ou individuellement une composition déflamine pour utilisation dans une méthode de traitement du corps humain ou animal par thérapie, dans laquelle ladite thérapie est de préférence la prévention ou le traitement de l'inflammation ou du cancer, dans laquelle ladite composition déflamine est éventuellement telle que définie dans l'une quelconque des revendications 5 à 8, et dans laquelle la déflamine peut être obtenue par la méthode de l'une quelconque des revendications 1 à 3.

11. Produit comprenant une multiplicité de polypeptides différents tels que définis dans l'une quelconque des revendications 5 à 8, qui forment ensemble une composition déflamine pour une utilisation simultanée, séparée ou séquentielle dans une méthode de traitement du corps humain ou animal par thérapie, dans laquelle ladite thérapie est de préférence la prévention ou le traitement de l'inflammation ou du cancer, et dans laquelle la déflamine peut être obtenue par la méthode de l'une quelconque des revendications 1 à 3.

12. Produit comprenant une multiplicité de vecteurs d'acide nucléique qui expriment ensemble une composition de déflamine pour une utilisation simultanée, séparée ou séquentielle dans une méthode de traitement du corps humain ou animal par une thérapie, dans laquelle ladite thérapie est de préférence la prévention ou le traitement de l'inflammation ou du cancer, et dans laquelle la déflamine peut être obtenue par la méthode de l'une quelconque des revendications 1 à 3.

13. Une composition qui est :
(i) une composition de déflamine qui peut être obtenue par la méthode de l'une quelconque des revendications 1 à 3 et qui est éventuellement définie dans l'une quelconque des revendications 5 à 8 ; ou
(ii) une composition comprenant un ou plusieurs acides nucléiques qui codent individuellement ou ensemble pour une composition polypeptidique de déflamine qui peut être obtenue par la méthode de l'une quelconque des revendications 1 à 3, dans laquelle éventuellement
- lesdits acides nucléiques sont des vecteurs d'expression qui peuvent exprimer une composition de déflamine, de préférence un vecteur viral, et/ou
- ladite composition se présente sous la forme d'une composition pharmaceutique, comprenant éventuellement aussi un support ou un diluant pharmaceutiquement acceptable.
